# EUROPEAN PATENT SPECIFICATION

(11) **EP 2 411 506 B1**
(45) Date of publication and mention of the grant of the patent: **07.11.2018**
(21) Application number: 10722830.6
(22) Date of filing: 26.03.2010
(51) Int. Cl.: C12N 5/0783, A61K 35/50, A61P 35/00, A61K 35/12

(54) **TUMOR SUPPRESSION USING HUMAN PLACENTA-DERIVED INTERMEDIATE NATURAL KILLER CELLS AND IMMUNOMODULATORY COMPOUNDS**
TUMORSUPPRESSION UNTER VERWENDUNG VON AUS MENSCHLICHER PLAZENTA GEWONNENEN INTERMEDIÄREN NATÜRLICHEN KILLERZELLEN UND IMMUNMODULATORISCHEN VERBINDUNGEN
ELIMINATION DE TUMEURS AU MOYEN DE CELLULES TUEUSES INTERMÉDIAIRES TIRÉES DU PLACENTA HUMAIN ET COMPOSÉS IMMUNOMODULATEURS

(30) Priority: 25.03.2009 US 163449 P
(43) Date of publication of application: 01.02.2012
(73) Proprietor: Celularity, Inc., Warren, NJ 07059 (US)
(72) Inventor: ZHANG, Xiaokui, Warren,NJ 07059 (US); KANG, Lin, Edison, NJ 08820 (US); HEIDARAN, Mohammad, Potomac,MD 20854 (US); JASKO, Steven, Rutherford, NJ 07070 (US); ZEITLIN, Andy, Basking Ridge, NJ 07920 (US); PAL, Ajai, Bridgewater, NJ 08807 (US); HARIRI, Robert, J., Warren,NJ 07059 (US)
(74) Representative: Jones Day
(86) International application number: PCT/US2010/028885
(87) International publication number: WO 2010/111631

(56) References cited:
- WO-A2-2007/011693
- WO-A2-2008/019148
- WO-A2-2009/045360
- TABIASCO ET AL: "Human Decidual NK Cells: Unique Phenotype and Functional Properties - A Review" PLACENTA, W.B. SAUNDERS, GB LNKD- DOI:10.1016/J.PLACENTA.2006.01.009, vol. 27, 1 April 2006 (2006-04-01), pages 34-39, XP005368303 ISSN: 0143-4004
- TAKAHASHI E ET AL: "Induction of CD16(+) CD56(bright) NK cells with antitumour cytotoxicity not only from CD16(-) CD56(bright) NK cells but also from CD16(-) CD56(dim) NK cells" SCANDINAVIAN JOURNAL OF IMMUNOLOGY, BLACKWELL SCIENCE PUBL., OXFORD, GB LNKD- DOI:10.1111/J.1365-3083.2006.01883.X, vol. 65, no. 2, 1 February 2007 (2007-02-01), pages 126-138, XP002528954 ISSN: 0300-9475

### SEQUENCE LISTING

<110> Celgene Cellular Therapeutics Zhang, Xiaokui Kang, Lin Heidaran, Mohammad Jasko, Steven Zeitlin, Andy Pal, Ajai Hariri, Robert B.
<120> TUMOR SUPPRESSION USING HUMAN PLACENTA-DERIVED INTERMEDIATE NATURAL KILLER
   CELLS AND IMMUNOMODULATORY COMPOUNDS
<130> 9516-834-999
<150> US 61/163,449
   <151> 2009-03-26
<160> 20
<170> FastSEQ for Windows Version 4.0
<210> 1
   <211> 22
   <212> RNA
   <213> Homo sapiens
<400> 1
   aacccguaga uccgaacuug ug 22
<210> 2
   <211> 22
   <212> RNA
   <213> Homo sapiens
<400> 2
   ucggauccgu cugagcuugg cu 22
<210> 3
   <211> 22
   <212> RNA
   <213> Homo sapiens
<400> 3
   uucccuuugu cauccuucgc cu 22
<210> 4
   <211> 23
   <212> RNA
   <213> Homo sapiens
<400> 4
   uaagugcuuc cauguuucag ugg 23
<210> 5
   <211> 20
   <212> RNA
   <213> Homo sapiens
<400> 5
   ccucugggcc cuuccuccag 20
<210> 6
   <211> 23
   <212> RNA
   <213> Homo sapiens
<400> 6
   uccagcuccu auaugaugcc uuu 23
<210> 7
   <211> 21
   <212> RNA
   <213> Homo sapiens
<400> 7
   cagcagcaca cugugguuug u 21
<210> 8
   <211> 22
   <212> RNA
   <213> Homo sapiens
<400> 8
   aagugcuguc auagcugagg uc 22
<210> 9
   <211> 24
   <212> RNA
   <213> Homo sapiens
<400> 9
   uucuccaaaa gaaagcacuu ucug 24
<210> 10
   <211> 22
   <212> RNA
   <213> Homo sapiens
<400> 10
   ucgugcaucc cuuuagagug uu 22
<210> 11
   <211> 22
   <212> RNA
   <213> Homo sapiens
<400> 11
   aucgugcauc cuuuuagagu gu 22
<210> 12
   <211> 21
   <212> RNA
   <213> Homo sapiens
<400> 12
   aaagcgcuuc ccuuugcugg a 21
<210> 13
   <211> 21
   <212> RNA
   <213> Homo sapiens
<400> 13
   aaagcgcuuc ccuucagagu g 21
<210> 14
   <211> 22
   <212> RNA
   <213> Homo sapiens
<400> 14
   caaagugccu cccuuuagag ug 22
<210> 15
   <211> 24
   <212> RNA
   <213> Homo sapiens
<400> 15
   acaaagugcu ucccuuuaga gugu 24
<210> 16
   <211> 22
   <212> RNA
   <213> Homo sapiens
<400> 16
   acaaagugcu ucccuuuaga gu 22
<210> 17
   <211> 19
   <212> RNA
   <213> Homo sapiens
<400> 17
   aggcacggug ucagcaggc 19
<210> 18
   <211> 19
   <212> RNA
   <213> Homo sapiens
<400> 18
   gggcgccugu gaucccaac 19
<210> 19
   <211> 23
   <212> RNA
   <213> Homo sapiens
<400> 19
   aaacucuacu uguccuucug agu 23
<210> 20
   <211> 22
   <212> RNA
   <213> Homo sapiens
<400> 20
   aacccguaga uccgaucuug ug 22

## Description

### 1. FIELD

Presented herein are methods of treatment of viral infections and cancer, *e.g.,* blood cancers and solid tumors, and of suppressing the growth or proliferation of cancer, *e.g.,* tumor cells, using isolated natural killer cells from placenta. In certain embodiments, the natural killer cells are used in combination with, or treated with, one or more immunomodulatory compounds, *e.g.,* immunomodulatory compounds referred to as IMiDs™.

### 2. BACKGROUND

Placental perfusate comprises a collection of placental cells obtained by passage of a perfusion solution through the placental vasculature, and collection of the perfusion fluid from the vasculature, from the maternal surface of the placenta, or both. Methods of perfusing mammalian placentas are described, *e.g.,* in U.S. Patent No. 7,045,148 and U.S. Patent No. 7,255,879. The population of placental cells obtained by perfusion is heterogenous, comprising hematopoietic (CD34⁺) cells, nucleated cells such as granulocytes, monocytes and macrophages, a small percentage (less than 1%) tissue culture substrate-adherent placental stem cells, and natural killer cells.

Natural killer (NK) cells are cytotoxic lymphocytes that constitute a major component of the innate immune system. NK cells do not express T-cell antigen receptors (TCR), CD3 or surface immunoglobulins (Ig) B cell receptor, but usually express the surface markers CD16 (FcγRIII) and CD56 in humans. NK cells are cytotoxic; small granules in their cytoplasm contain special proteins such as perforin and proteases known as granzymes. Upon release in close proximity to a cell slated for killing, perforin forms pores in the cell membrane of the target cell through which the granzymes and associated molecules can enter, inducing apoptosis. One granzyme, granzyme B (also known as granzyme 2 and cytotoxic T-lymphocyte-associated serine esterase 1), is a serine protease crucial for rapid induction of target cell apoptosis in the cell-mediated immune response.

The induction of CD16⁺CD56^{bright} natural killer (NK) cells with antitumor cytotoxicity from CD16⁻CD56^{bright} NK cells and CD16⁻CD56^{dim} NK cells from human peripheral blood mononuclear cells, and NK to attack viral-infected cells has been described by Takahashi E. et al (Scandinavian Journal of Immunology 65:126-138 (2007)).

NK cells are activated in response to interferons or macrophage-derived cytokines. Activated NK cells are referred to as lymphokine activated killer (LAK) cells. NK cells possess two types of surface receptors, labeled "activating receptors" and "inhibitory receptors," that control the cells' cytotoxic activity.

Among other activities, NK cells play a role in the host rejection of tumors. Because cancer cells have reduced or no class I MHC expression, they can become targets of NK cells. Accumulating clinical data suggest that haploidentical transplantation of human NK cells isolated from PBMC or bone marrow can help prevent leukemic relapse after bone marrow transplantation without causing detectable graft versus host disease (GVHD). *See* Ruggeri et al., Science 295:2097-2100 (2002)). Natural killer cells can become activated by cells lacking, or displaying reduced levels of, major histocompatibility complex (MHC) proteins. Activated and expanded NK cells and LAK cells have been used in both *ex vivo* therapy and *in vivo* treatment of patients having advanced cancer, with some success against bone marrow related diseases, such as leukemia; breast cancer; and certain types of lymphoma. LAK cell treatment requires that the patient first receive IL-2, followed by leukopheresis and then an ex vivo incubation and culture of the harvested autologous blood cells in the presence of IL-2 for a few days. The LAK cells must be reinfused along with relatively high doses of IL-2 to complete the therapy. This purging treatment is expensive and can cause serious side effects. These include fluid retention, pulmonary edema, drop in blood pressure, and high fever.

In spite of the advantageous properties of NK cells in killing tumor cells and virus-infected cells, they remain difficult to work with and to apply in immunotherapy, primarily due to the difficulty in maintaining their tumor-targeting and tumoricidal capabilities during culture and expansion. Thus, there is a need in the art for a ready supply of natural killer cells.

### 3. SUMMARY

The present invention provides isolated natural killer cells comprising isolated CD56⁺, CD16⁻, CD3⁻ placental intermediate natural killer (PINK) cells for use in a method for treatment of an individual having cancer, wherein the method comprises administering to said individual said isolated natural killer cells comprising isolated CD56⁺, CD16⁻, CD3⁻ PINK cells, wherein said PINK cells are isolated from placental perfusate and express one or more of the microRNAs hsa-miR-100, hsa-miR-127, hsa-miR-211, hsa-miR-302c, hsa-miR-326, hsa-miR-337, hsa-miR-497, hsa-miR-512-3p, hsa-miR-515-5p, hsa-miR-517b, hsa-miR-517c, hsa-miR-518a, hsa-miR-518e, hsa-miR-519d, hsa-miR-520g, hsa-miR-520h, hsa-miR-564, hsa-miR-566, hsa-miR-618, or hsa-miR-99a at a detectably higher level than peripheral blood natural killer cells; and
wherein said cancer is acute promyelocytic leukemia, acute myeloblastic leukemia, acute megakaryoblastic leukemia, precursor B acute lymphoblastic leukemia, precursor T acute lymphoblastic leukemia, Burkitt's leukemia (Burkitt's lymphoma), acute biphenotypic leukemia, chronic monocytic leukemia, chronic lymphocytic leukemia (CLL)/Small lymphocytic lymphoma, B-cell prolymphocytic leukemia; hairy cell lymphoma; T-cell prolymphocytic leukemia, lymphoplasmacytic lymphoma, Waldenström macroglobulinemia, splenic marginal zone lymphoma, plasmacytoma, a monoclonal immunoglobulin deposition disease, or a heavy chain disease, extranodal marginal zone B cell lymphoma (MALT lymphoma), nodal marginal zone B cell lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, T cell large granular lymphocytic leukemia, aggressive NK cell leukemia, extranodal NK/T cell lymphoma, nasal type, enteropathy-type T cell lymphoma, hepatosplenic T cell lymphoma, blastic NK cell lymphoma, mycosis fungoides (Sezary syndrome), a primary cutaneous CD30-positive T cell lymphoproliferative disorder, primary cutaneous anaplastic large cell lymphoma, lymphomatoid papulosis, angioimmunoblastic T cell lymphoma, peripheral T cell lymphoma, unspecified, anaplastic large cell lymphoma, a Hodgkin lymphoma, or a nodular lymphocyte-predominant Hodgkin lymphoma.

Hereinafter, reference to natural killer cells within the context of the invention is to be understood as reference to natural killer cells comprising PINK cells of the invention. Consistent with this, hereinafter reference to PINK cells within the context of the invention is to be understood as reference to PINK cells as defined above, also referred to as PINK cells of the invention.

In one embodiment, the method additionally comprises administering to said individual an effective amount of lenalidomide, pomalidomide, or thalidomide. In one embodiment, said isolated natural killer cells have been contacted with pomalidomide, lenalidomide, or thalidomide prior to said administering.

In another embodiment, the isolated natural killer cells comprise natural killer cells not obtained from placental perfusate.

The present invention also provides isolated natural killer cells comprising isolated CD56⁺, CD16⁻, CD3⁻ PINK cells for use in a method for treatment of an individual having a viral infection, wherein the method comprises administering to said individual said isolated natural killer cells comprising isolated PINK cells of the invention.

In one embodiment, the method additionally comprises administering to said individual an effective amount of lenalidomide, pomalidomide, or thalidomide. In another embodiment, the isolated natural killer cells have been contacted with pomalidomide, lenalidomide, or thalidomide prior to said administering.

In another embodiment, the isolated natural killer cells comprise natural killer cells not obtained from placental perfusate.

Further, in one embodiment, the isolated natural killer cells are combined natural killer cells that comprise natural killer cells isolated from placental perfusate and natural killer cells isolated from umbilical cord blood. In a specific embodiment, said umbilical cord blood is isolated from the placenta from which said placental perfusate is obtained.

In a specific embodiment, the isolated natural killer cells are combined natural killer cells that comprise: a detectably higher number of CD3⁻CD56⁺CD16⁻ natural killer cells than an equivalent number of natural killer cells from peripheral blood;
a detectably lower number of CD3⁻CD56⁺CD16⁺ natural killer cells than an equivalent number of natural killer cells from peripheral blood; a detectably higher number of CD3⁻CD56⁺KIR2DL2/L3⁺ natural killer cells than an equivalent number of natural killer cells from peripheral blood; a detectably lower number of CD3⁻CD56⁺NKp46⁺ natural killer cells than an equivalent number of natural killer cells from peripheral blood; a detectably higher number of CD3⁻CD56⁺NKp30⁺ natural killer cells than an equivalent number of natural killer cells from peripheral blood; a detectably higher number of CD3⁻CD56⁺2B4⁺ natural killer cells than an equivalent number of natural killer cells from peripheral blood; or a detectably higher number of CD3⁻CD56⁺CD94⁺ natural killer cells than an equivalent number of natural killer cells from peripheral blood.

In another embodiment, the isolated natural killer cells are contacted with said pomalidomide, lenalidomide, or thalidomide in an amount and for a time sufficient for said natural killer cells to express detectably more granzyme B, or mRNA encoding granzyme B, than an equivalent number of natural killer cells not contacted with said pomalidomide, lenalidomide, or thalidomide. In yet another embodiment, the isolated natural killer cells for use in the present invention have not been cultured prior to said administering.

In a specific embodiment, the PINK cells express one or more of aminopeptidase N protein, apolipoprotein E protein, atrophin-1 interacting protein 1, innexin inx-3 protein, integrin alpha-2 precursor protein, integrin beta-5 precursor, mast cell surface glycoprotein GP49B precursor protein, or ryanodine receptor 1 protein; and wherein said placental intermediate natural killer cells do not express one or more of fibroblast growth factor receptor 4 precursor protein, immunity-associated nucleotide 4-like protein, integrin alpha-L precursor protein, integrin beta 2 precursor protein, integrin beta 4 precursor protein, membrane-bound lytic murein transglycosylase D precursor protein, oxysterol binding protein-related protein 8, or perforin 1 precursor 1 protein.

Disclosed herein are methods of using placental perfusate, placental perfusate cells, natural killer cells from any source, *e.g.,* natural killer cells from placental perfusate, natural killer cells obtained by digestion of placental tissue, or natural killer cells from another tissue source, to suppress tumor cell proliferation, treat viral infection or treat cancer, *e.g.,* blood cancers and/or solid tumors. In certain aspects of the disclosure, the cells are used in combination with an immunomodulatory compound, *e.g.,* an immunomodulatory compound described in Section 5.9, below, or thalidomide.

In one aspect, provided herein are isolated natural killer cells for use in a method of treating an individual having cancer or a viral infection, comprising administering to said individual an effective amount of said isolated natural killer cells. In a specific embodiment, the isolated natural killer cells are treated with, *e.g.,* contacted with, an immunomodulatory compound, *e.g.* an immunomodulatory compound described in Section 5.9, below, or thalidomide, prior to said administration. In another specific embodiment, the isolated natural killer cells are not treated with, *e.g.,* contacted with, an immunomodulatory compound, *e.g.* an amino-substituted thalidomide and/or a compound described in Section 5.9, below, or thalidomide, prior to said administration. In another specific embodiment, the method further comprises additionally administering to the individual an effective amount of an immunomodulatory compound or thalidomide. An "effective amount" in this context means an amount of natural killer cells, and optionally immunomodulatory compound or thalidomide, that results in a detectable improvement in one or more symptoms of said cancer or said viral infection, compared to an individual having said cancer or said viral infection who has not been administered said natural killer cells and, optionally, said immunomodulatory compound or thalidomide. In specific embodiments, said immunomodulatory compound is lenalidomide or pomalidomide. In another embodiment, said cancer is a solid tumor. In another embodiment, said cancer is a blood cancer. In a specific embodiments, the cancer is primary ductal carcinoma, leukemia, acute T cell leukemia, chronic myeloid lymphoma (CML), acute myelogenous leukemia, chronic myelogenous leukemia (CML), lung carcinoma, colon adenocarcinoma, histiocytic lymphoma, colorectal carcinoma, colorectal adenocarcinoma, or retinoblastoma. According to the invention, said natural killer cells comprise CD56⁺, CD16⁻ CD3- placental intermediate natural killer (PINK) cells of the invention. In a specific embodiment, said natural killer cells are, or consist essentially of, CD56⁺, CD16⁻, CD3- placental intermediate natural killer (PINK) cells. In another more specific embodiment, said natural killer cells comprise natural killer cells not obtained from placental perfusate. In another more specific embodiment, said natural killer cells comprise natural killer cells obtained from umbilical cord blood or peripheral blood. In another more specific embodiment, said natural killer cells are combined natural killer cells that comprise natural killer cells isolated from placental perfusate (e.g., PINK cells) and natural killer cells isolated from umbilical cord blood. In a more specific embodiment, said umbilical cord blood is isolated from the placenta from which said placental perfusate is obtained. In another more specific embodiment, said umbilical cord blood is isolated from a placenta other than the placenta from which said placental perfusate is obtained.

In another specific embodiment, said natural killer cells, and said immunomodulatory compound or thalidomide, are administered separately to said individual. In another specific embodiment, said natural killer cells and said immunomodulatory compound or thalidomide are administered together, *e.g.,* in the same formulation, or in separate formulations but at the same time, to said individual. In another more specific embodiment, said natural killer cells are combined natural killer cells that comprise natural killer cells isolated from placental perfusate and natural killer cells isolated from umbilical cord blood. In a more specific embodiment, said umbilical cord blood is isolated from the placenta from which said placental perfusate is obtained. In another more specific embodiment, said umbilical cord blood is isolated from a placenta other than the placenta from which said placental perfusate is obtained.

In another specific embodiment, said natural killer cells have not been cultured prior to said administration. In another specific embodiment, said natural killer cells have been cultured prior to said administration.

In another aspect, disclosed herein is a method of treating an individual having cancer or a viral infection, comprising administering to said individual an effective amount of isolated placental perfusate cells, *e.g.,* placental perfusate cells comprising natural killer cells. In a specific aspect of the disclosure the isolated placental perfusate cells have been pre-treated with an immunomodulatory compound, *e.g.* an immunomodulatory compound described in Section 5.9, below, or thalidomide. In a specific aspect of the disclosure, the method further comprises administering to said individual an effective amount of an immunomodulatory compound or thalidomide. An "effective amount" in this context means an amount of placental perfusate cells, and optionally immunomodulatory compound or thalidomide, that results in a detectable improvement in one or more symptoms of said cancer or said viral infection, compared to an individual having said cancer or said viral infection who has not been administered said placental perfusate cells, and optionally an immunomodulatory compound or thalidomide. In one aspect of the disclosure, said placental perfusate cells are pretreated with an immunomodulatory compound or thalidomide. In another aspect of the disclosure, said placental perfusate cells are not pretreated with an immunomodulatory compound or thalidomide. In another aspect of the disclosure, said immunomodulatory compound is lenalidomide or pomalidomide. In one aspect, said cancer is a blood cancer. In a specific aspect of the disclosure, said cancer is primary ductal carcinoma, leukemia, acute T cell leukemia, chronic myeloid lymphoma (CML), acute myelogenous leukemia, chronic myelogenous leukemia (CML), lung carcinoma, colon adenocarcinoma, histiocytic lymphoma, colorectal carcinoma, colorectal adenocarcinoma, or retinoblastoma. In one aspect of the disclosure, said placental perfusate cells comprise natural killer cells. In another aspect of the disclosure, said natural killer cells comprise, consist essentially of, or are CD56⁺, CD16⁻ placental intermediate natural killer (PINK) cells. In another aspect of the disclosure, said natural killer cells are obtained from umbilical cord blood or peripheral blood. In another aspect of the disclosure, said placental perfusate cells are contacted with an immunomodulatory compound or thalidomide prior to said administration. In a more specific aspect of the disclosure, said natural killer cells in said placental perfusate cells are contacted with said immunomodulatory compound or thalidomide in an amount and for a time sufficient for said natural killer cells to express detectably more granzyme B, or mRNA encoding granzyme B, than an equivalent number of natural killer cells not contacted with said immunomodulatory compound or thalidomide.

In another aspect, the above method of cancer treatment comprises suppressing the proliferation of tumor cells after contacting the tumor cells with an effective amount of the isolated natural killer cells. In a specific embodiment, the isolated natural killer cells have been pre-treated with an immunomodulatory compound, *e.g.* an immunomodulatory compound described in Section 5.9, below, or thalidomide. In another specific embodiment, the tumor cells are additionally contacted with an effective amount of an immunomodulatory compound or thalidomide. An "effective amount" in this context means an amount of natural killer cells, and optionally an immunomodulatory compound or thalidomide, that results in a detectable suppression of said tumor cells compared to an equivalent number of tumor cells not contacted with said natural killer cells, and optionally an immunomodulatory compound or thalidomide. In a more specific embodiment, said natural killer cells are pretreated with an immunomodulatory compound or thalidomide. In another more specific embodiment, said natural killer cells are not pretreated with an immunomodulatory compound or thalidomide. In a specific embodiment of this method, the tumor cells are blood cancer cells. In another specific embodiment, the tumor cells are solid tumor cells. In another specific embodiment, the tumor cells are solid tumor cells. In another embodiment, the tumor cell is a primary ductal carcinoma cell, a leukemia cell, an acute T cell leukemia cell, a chronic myeloid lymphoma (CML) cell, an acute myelogenous leukemia cell, a chronic myelogenous leukemia (CML) cell, a lung carcinoma cell, a colon adenocarcinoma cell, a histiocytic lymphoma cell, multiple myeloma cell, a retinoblastoma cell, a colorectal carcinoma cell, or a colorectal adenocarcinoma cell. In another specific embodiment, said contacting takes place *in vitro.* In another specific embodiment, said contacting takes place *in vivo.* In a more specific embodiment, said *in vivo* contacting takes place in a human. According to the invention said natural killer cells comprise CD56⁺, CD16⁻, CD3- placental intermediate natural killer (PINK) cells. According to the invention, said natural killer cells are CD56⁺, CD16⁻, CD3- placental intermediate natural killer (PINK) cells. In another more specific embodiment, said natural killer cells comprise natural killer cells not obtained from placental perfusate. In another more specific embodiment, said natural killer cells are obtained from umbilical cord blood or peripheral blood. In another more specific embodiment, said natural killer cells are combined natural killer cells that comprise natural killer cells isolated from placental perfusate and natural killer cells isolated from umbilical cord blood. In a more specific embodiment, said umbilical cord blood is isolated from the placenta from which said placental perfusate is obtained. In another more specific embodiment, said umbilical cord blood is isolated from a placenta other than the placenta from which said placental perfusate is obtained.

In another aspect, disclosed herein is a method of suppressing the proliferation of tumor cells, either *in vivo* or *in vitro,* comprising contacting the tumor cells with an effective amount of isolated placental perfusate cells, *e.g.,* placental perfusate cells comprising placental natural killer cells. In one aspect of the disclosure, the isolated placental perfusate cells have been pre-treated with an immunomodulatory compound, *e.g.* an immunomodulatory compound described in Section 5.9, below, or thalidomide. In another aspect of the disclosure, the method further comprises contacting the tumor cells with an effective amount of an immunomodulatory compound or thalidomide. An "effective amount" in this context means an amount of placental perfusate cells, and optionally an immunomodulatory compound or thalidomide, that results in a detectable suppression of said tumor cells compared to an equivalent number of tumor cells not contacted with said placental perfusate cells, and another aspect of the disclosure optionally an immunomodulatory compound or thalidomide. In another aspect of the disclosure, said natural killer cells are pretreated with an immunomodulatory compound or thalidomide. In another aspect of the disclosure, said natural killer cells are not pretreated with an immunomodulatory compound or thalidomide. In one aspect of the disclosure, said placental perfusate cells are, or comprise, total nucleated cells from placental perfusate. In another aspect of the disclosure, said placental perfusate or placental perfusate cells, *e.g.,* total nucleated cells from placental perfusate, have been treated to remove at least one type of cell. In another aspect of the disclosure, said contacting takes place *in vitro.* In another aspect of the disclosure, said contacting takes place *in vivo.* In another aspect of the disclosure, said *in vivo* contacting, takes place in a mammal, *e.g.,* a human. In another aspect of the disclosure, said placental perfusate cells have been treated to enrich for at least one type of cell, *e.g.,* CD56⁺ cells. In another aspect of the disclosure, said placental perfusate cells are CD56⁺ placental cells. In another aspect of the disclosure, the CD56⁺ cells are CD56⁺CD16⁻ natural killer cells, *e.g.,* placental intermediate natural killer (PINK) cells, *e.g.,* obtained from placental perfusate cells or placental cells obtained by mechanical or enzymatic disruption of placental tissue. In another aspect of the disclosure, said CD56⁺ cells are selected by CD56-conjugated microbeads. In another aspect of the disclosure, said CD56⁺ cells comprise cells that exhibit detectably lower expression of NKG2D, NKp46 or CD94, as determined, *e.g.,* by flow cytometry, than an equivalent number of CD56⁺CD16⁺ natural killer cells. In another aspect of the disclosure, the PINK cells are CD3⁻. In another aspect of the disclosure, at least 50% of the cells in said placental perfusate cells are said CD56⁺ cells. In aspect of the disclosure, wherein the CD56⁺ cells are at least 50% of said placental perfusate cells, the tumor cells are primary ductal carcinoma cells, leukemia cells, acute T cell leukemia cells, chronic myeloid lymphoma (CML) cells, acute myelogenous leukemia cells, chronic myelogenous leukemia (CML) cells, lung carcinoma cells, colon adenocarcinoma cells, histiocytic lymphoma cells, multiple myeloma cells, retinoblastoma cells, colorectal carcinoma cells or colorectal adenocarcinoma cells. In specific aspect of the disclosure, said contacting is contacting *in vitro*. In another aspect of the disclosure, said contacting is contacting *in vivo, e.g.,* in a mammal, *e.g.,* a human.

In a specific embodiment of the above methods using cells from placental perfusate, said placental perfusate is perfusate that has been passed through placental vasculature, *e.g.,* only through placental vasculature. In another specific embodiment, said placental perfusate has been passed through the placental vasculature and collected from the maternal face of the placenta. In another specific embodiment, all, or substantially all (*e.g.,* greater than 90%, 95%, 98% or 99%) of cells in said placental perfusate are fetal cells. In another specific embodiment, the placental perfusate comprises fetal and maternal cells. In a more specific embodiment, the fetal cells in said placental perfusate comprise less than about 90%, 80%, 70%, 60% or 50% of the cells in said perfusate. In another specific embodiment, said perfusate is obtained by passage of a 0.9% NaCl solution through the placental vasculature. In another specific embodiment, said perfusate comprises a culture medium. In another specific embodiment, said perfusate has been treated to remove erythrocytes.

In another specific embodiment of the above methods, the natural killer cells comprise cells that exhibit detectably lower expression of NKG2D, NKp46 or CD94, as determined, *e.g.,* by flow cytometry, than an equivalent number of CD56⁺CD16⁺ natural killer cells. In another specific embodiment, said natural killer cells comprise PINK cells of the invention which express one or more of the microRNAs hsa-miR-100, hsa-miR-127, hsa-miR-211, hsa-miR-302c, hsa-miR-326, hsa-miR-337, hsa-miR-497, hsa-miR-512-3p, hsa-miR-515-5p, hsa-miR-517b, hsa-miR-517c, hsa-miR-518a, hsa-miR-518e, hsa-miR-519d, hsa-miR-520g, hsa-miR-520h, hsa-miR-564, hsa-miR-566, hsa-miR-618, and/or hsa-miR-99a at a detectably higher level than peripheral blood natural killer cells, as determined, *e.g.,* by quantitative real-time PCR (qRT-PCR). In another embodiment, the natural killer cells do not express the microRNA hsa-miR-199b, or express the microRNA hsa-miR-199b at a detectably lower level than peripheral blood natural killer cells.

In another specific embodiment of the present invention, said natural killer cells, *e.g.,* PINK cells, are contacted with an immunomodulatory compound or thalidomide in an amount and for a time sufficient for said natural killer cells to express detectably more granzyme B or perforin than an equivalent number of said natural killer cells not contacted with said immunomodulatory compound or thalidomide. In another specific embodiment, said natural killer cells, *e.g.,* PINK cells, are contacted with an immunomodulatory compound or thalidomide in an amount and for a time sufficient for said natural killer cells to exhibit detectably more cytotoxicity towards said tumor cells than an equivalent number of said natural killer cells not contacted with said immunomodulatory compound, *e.g.,* lenalidomide or pomalidomide, or with thalidomide. In another specific embodiment, said natural killer cells, *e.g.,* PINK cells, express one or more of BAX, CCL5, CCR5, CSF2, FAS, GUSB, IL2RA, or TNFRSF18 at a higher level than an equivalent number of said natural killer cells not contacted with said immunomodulatory compound or thalidomide. In another specific embodiment, said natural killer cells, *e.g.,* PINK cells, express one or more of ACTB, BAX, CCL2, CCL3, CCL5, CCR5, CSF1, CSF2, ECE1, FAS, GNLY, GUSB, GZMB, ILIA, IL2RA, IL8, IL10, LTA, PRF1, PTGS2, SKI, and TBX21 at a higher level than an equivalent number of said natural killer cells not contacted with said immunomodulatory compound or thalidomide.

In certain embodiments, in the methods of treatment or tumor suppression above, natural killer cells from placenta are combined with natural killer cells from umbilical cord blood to form combined natural killer cells. As used herein, the phrase "natural killer cell(s) from placenta" does not include natural killer cells from umbilical cord blood or placental blood. In more specific embodiments, the natural killer cells from placenta are combined with natural killer cells from another source in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In a specific embodiments of the present invention, methods, the combined natural killer cells are not cultured, and comprise: a detectably higher number of CD3⁻CD56⁺CD16⁻ natural killer cells than an equivalent number of natural killer cells from peripheral blood; a detectably lower number of CD3⁻CD56⁺CD16⁺ natural killer cells than an equivalent number of natural killer cells from peripheral blood; a detectably higher number of CD3⁻ CD56⁺KIR2DL2/L3⁺ natural killer cells than an equivalent number of natural killer cells from peripheral blood; a detectably lower number of CD3⁻CD56⁺NKp46⁺ natural killer cells than an equivalent number of natural killer cells from peripheral blood; a detectably higher number of CD3⁻CD56⁺NKp30⁺ natural killer cells than an equivalent number of natural killer cells from peripheral blood; a detectably higher number of CD3⁻CD56⁺2B4⁺ natural killer cells than an equivalent number of natural killer cells from peripheral blood; or a detectably higher number of CD3⁻CD56⁺CD94⁺ natural killer cells than an equivalent number of natural killer cells from peripheral blood. In other specific embodiments, the combined natural killer cells are cultured and comprise: a detectably lower number of CD3⁻CD56⁺KIR2DL2/L3⁺ natural killer cells than an equivalent number of natural killer cells from peripheral blood; a detectably higher number of CD3⁻CD56⁺NKp46⁺ natural killer cells than an equivalent number of natural killer cells from peripheral blood; a detectably higher number of CD3⁻ CD56⁺NKp44⁺ natural killer cells than an equivalent number of natural killer cells from peripheral blood; a detectably higher number of CD3⁻CD56⁺NKp30⁺ natural killer cells than an equivalent number of natural killer cells from peripheral blood.

In a specific embodiment of the present invention, the tumor cell is a solid tumor cell. In another specific embodiment, the tumor cell is a liquid tumor cell, *e.g.,* a blood tumor cell. In more specific embodiments, the tumor cell is a primary ductal carcinoma cell, a leukemia cell, an acute T cell leukemia cell, a chronic myeloid lymphoma (CML) cell, an acute myelogenous leukemia cell, a chronic myelogenous leukemia (CML) cell, a lung carcinoma cell, a colon adenocarcinoma cell, a histiocytic lymphoma cell, multiple myeloma cell, a retinoblastoma cell, a colorectal carcinoma cell or a colorectal adenocarcinoma cell.

In another aspect, provided herein is a composition comprising natual killer cells comprising isolated CD56+, CD16-, CD3- placental intermediate natural killer cells, i.e. PINK cells for use in a method of cancer treatment or treatment of a viral infection. In a specific embodiment, said placental natural killer cells are isolated from placental perfusate. In another specific embodiment, said placental natural killer cells are isolated from placenta by physical disruption and/or enzymatic digestion of placental tissue. In another specific embodiment, said natural killer cells comprise at least 50% of cells in the composition. In a specific embodiment, said natural killer cells comprise at least 80% of cells in the composition. In another specific embodiment, said composition comprises isolated CD56⁺, CD16⁺ natural killer cells. In a more specific embodiment, said CD56⁺, CD16⁺ natural killer cells are from a different individual than said CD56⁺, CD16⁻, CD3- PINK cells. In another specific embodiment, said isolated CD56⁺, CD16⁻, CD3- PINK cells are from a single individual. In a more specific embodiment, said isolated CD56⁺, CD16⁻, CD3- PINK cells comprise PINK cells from at least two different individuals. In another specific embodiment, said placental natural killer cells, *e.g.,* said PINK cells, are expanded. In another specific embodiment, said natural killer cells, *e.g.,* PINK cells, have been contacted with an immunomodulatory compound or thalidomide in an amount and for a time sufficient for said natural killer cells to express detectably more granzyme B or perforin than an equivalent number of said natural killer cells not contacted with said immunomodulatory compound or thalidomide. In another specific embodiment, said composition additionally comprises an immunomodulatory compound or thalidomide.

In a more specific embodiment, the composition comprises placental natural killer cells and natural killer cells from another source. In a specific embodiment, said other source is placental blood and/or umbilical cord blood. In another specific embodiment, said other source is peripheral blood. In more specific embodiments, the natural killer cells from placenta are combined with natural killer cells from another source in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In another specific embodiment, the composition comprises isolated placental perfusate. In a specific embodiment, said placental perfusate is from the same individual as said natural killer cells. In another more specific embodiment, said placental perfusate comprises placental perfusate from a different individual than said natural killer cells. In another specific embodiment, all, or substantially all (*e.g.,* greater than 90%, 95%, 98% or 99%) of cells in said placental perfusate are fetal cells. In another specific embodiment, the placental perfusate comprises fetal and maternal cells. In a more specific embodiment, the fetal cells in said placental perfusate comprise less than about 90%, 80%, 70%, 60% or 50% of the cells in said perfusate. In another specific embodiment, said perfusate is obtained by passage of a 0.9% NaCl solution through the placental vasculature. In another specific embodiment, said perfusate comprises a culture medium. In another specific embodiment, said perfusate has been treated to remove erythrocytes. In another specific embodiment, said composition comprises an immunomodulatory compound.

In another specific embodiment, the composition comprises placental perfusate cells. In a more specific embodiment, said placental perfusate cells are from the same individual as said natural killer cells. In another more specific embodiment, said placental perfusate cells are from a different individual than said natural killer cells. In another specific embodiment, the composition comprises isolated placental perfusate and isolated placental perfusate cells, wherein said isolated perfusate and said isolated placental perfusate cells are from different individuals. In another more specific embodiment of any of the above embodiments comprising placental perfusate, said placental perfusate comprises placental perfusate from at least two individuals. In another more specific embodiment of any of the above embodiments comprising placental perfusate cells, said isolated placental perfusate cells are from at least two individuals. The composition can additionally comprise isolated PINK cells, wherein the PINK cells are from a different individual than said placental perfusate or said perfusate cells. In another specific embodiment, said composition comprises an immunomodulatory compound or thalidomide.

### 3.1. Definitions

As used herein, "combined natural killer cells" are natural killer cells from umbilical cord blood and placental perfusate. In certain embodiments, the natural killer cells are from matched umbilical cord blood and human placental perfusate, wherein placental perfusate is obtained from the same placenta as the cord blood. Natural killer cells from both placental perfusate and umbilical cord blood are isolated separately or at the same time, and combined.

As used herein, the terms "immunomodulatory compound" and "IMiD" do not encompass thalidomide.

As used herein, "lenalidomide" means 3-(4'aminoisoindoline-1'-one)-1-piperidine-2,6-dione (Chemical Abstracts Service name) or 2,6-Piperidinedione,3-(4-amino-1,3-dihydro-1-oxo-2H-isoindol-2-yl)- (International Union of Pure and Applied Chemistry (IUPAC) name)

As used herein, "multipotent," when referring to a cell, means that the cell has the capacity to differentiate into a cell of another cell type. In certain embodiments, "a multipotent cell" is a cell that has the capacity to grow into any subset of the mammalian body's approximately 260 cell types. Unlike a pluripotent cell, a multipotent cell does not have the capacity to form all of the cell types.

As used herein, "natural killer cell," without further modification, includes natural killer cells from any tissue source.

As used herein, "PINK" and "PINK cells" refer to a type of natural killer cell, termed placental intermediate natural killer cells, that are obtained from human placenta, *e.g.,* human placental perfusate or placental tissue that has been mechanically and/or enzymatically disrupted. The cells are CD56⁺ and CD16⁻, *e.g.,* as determined by flow cytometry, *e.g.,* fluorescence-activated cell sorting using antibodies to CD56 and CD16. PINK cells are not obtained from cord blood or peripheral blood. The cells are cytolytic in an assay for cytolytic activity, *e.g.,* using a tumor cell line such as K562 tumor cells as target cells.

As used herein, "placental perfusate" means perfusion solution that has been passed through at least part of a placenta, *e.g.,* a human placenta, *e.g.,* through the placental vasculature, including a plurality of cells collected by the perfusion solution during passage through the placenta.

As used herein, "placental perfusate cells" means nucleated cells, *e.g.,* total nucleated cells, isolated from, or isolatable from, placental perfusate.

As used herein, "pomalidomide" means 4-amino-2-[(3RS)-2,6-dioxopiperidin-3-yl)-1H-isoindole-1,3(2H)-dione.

As used herein, "tumor cell suppression," "suppression of tumor cell proliferation," and the like, includes slowing the growth of a population of tumor cells, *e.g.,* by killing one or more of the tumor cells in said population of tumor cells, for example, by contacting the population of tumor cells with PINK cells, a population of cells comprising PINK cells, combined natural killer cells, a population of cells comprising combined natural killer cells, human placental perfusate, or the like.

### 4. BRIEF DESCRIPTION OF THE FIGURES

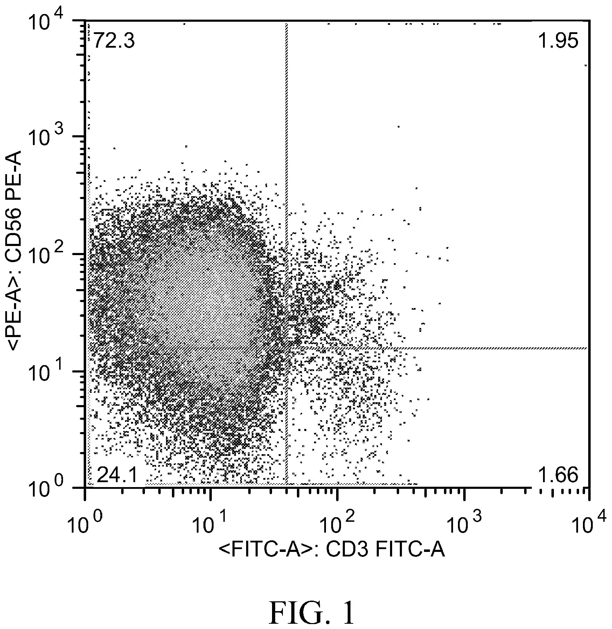

FIG. 1 shows flow cytometry results using anti-CD3 antibodies and anti-CD56 antibodies for cells selected by CD56 microbeads from human placental perfusate (HPP). The majority of the isolated cells are CD56⁺CD3⁻.

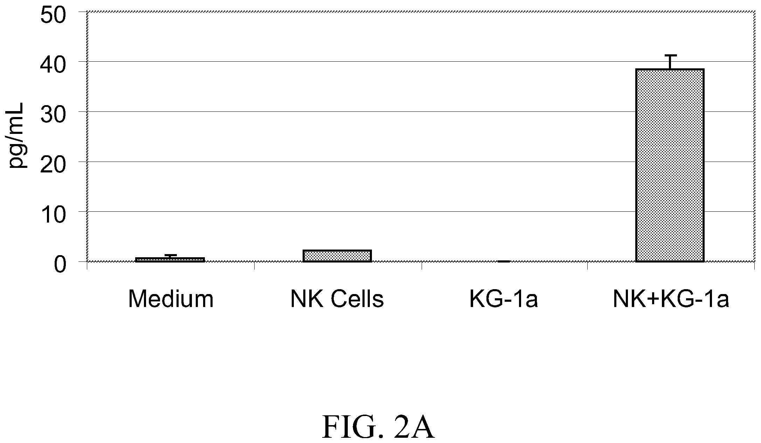

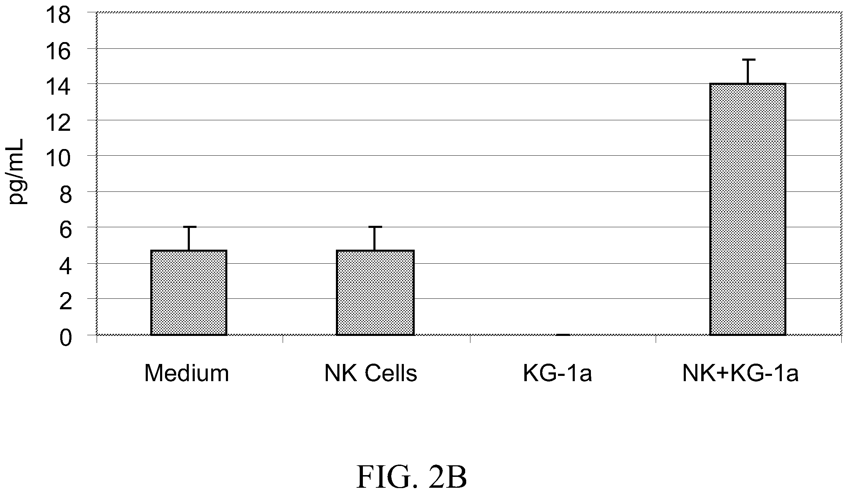

FIGS. 2A and 2B depict production of cytokines by PINK cells and/or tumor cells during 24 hour culture. FIG. 2A depicts secretion of interferon gamma (IFNγ) by placental perfusate-derived intermediate natural killer cells (PINK) cells alone or in the presence of KG-1a tumor cells. PINK cells and KG-1a cells were cultured alone or in combination at a ratio of 1:1. Y axis: picograms of IFNγ produced by the cultures. FIG. 2B depicts secretion of granulocyte-macrophage colony stimulating factor (GM-CSF) by PINK cells alone or in the presence of KG-1a tumor cells. PINK cells and KG-1a cells were cultured alone or in combination at a ratio of 1:1. Y axis: picograms of GM-CSF produced by the cultures.

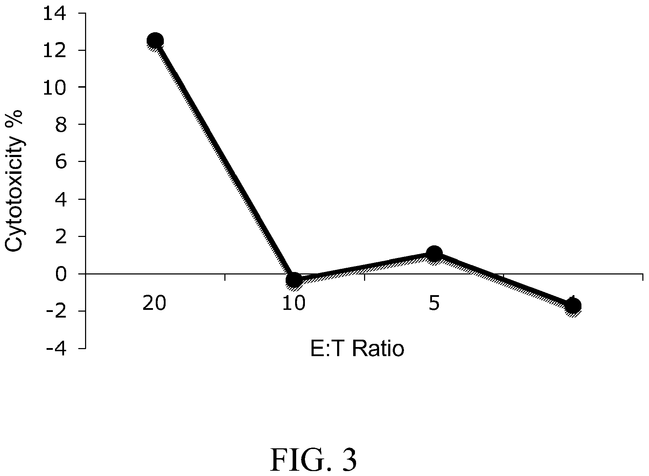

FIG. 3 depicts cytotoxicity of PINK cells to KG-1a tumor cells in 24 hour co-culture at a ratio of 1:1, 5:1, 10:1 or 20:1 PINK cells to tumor cells. X axis: ratio of PINK cells to tumor cells. Y axis: percentage of dead tumor cells compared to tumor cells without PINK cells.

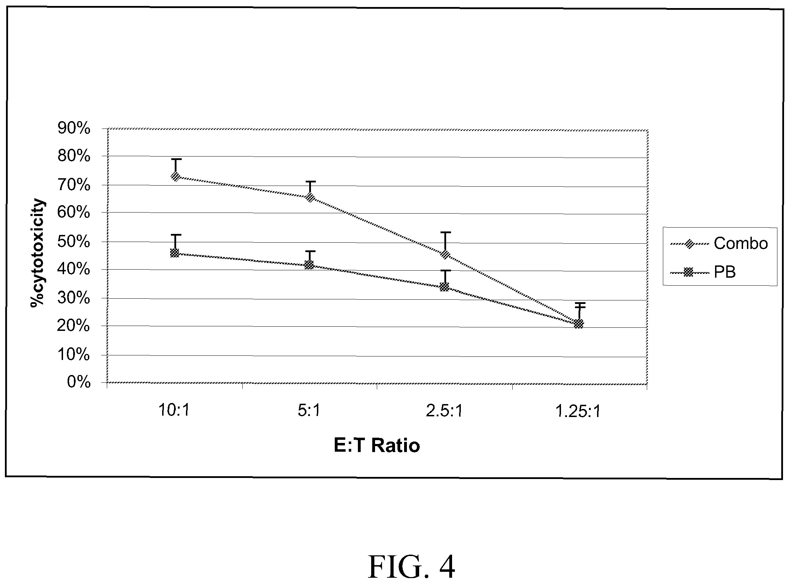

FIG. 4 depicts cytotoxicity of placental NK cells and peripheral blood (PB) NK cells cultured for 21 days towards K562 cells. Error bars stand for standard deviation of 4 units of cultured placental NK cells or 3 units of cultured peripheral blood NK cells.

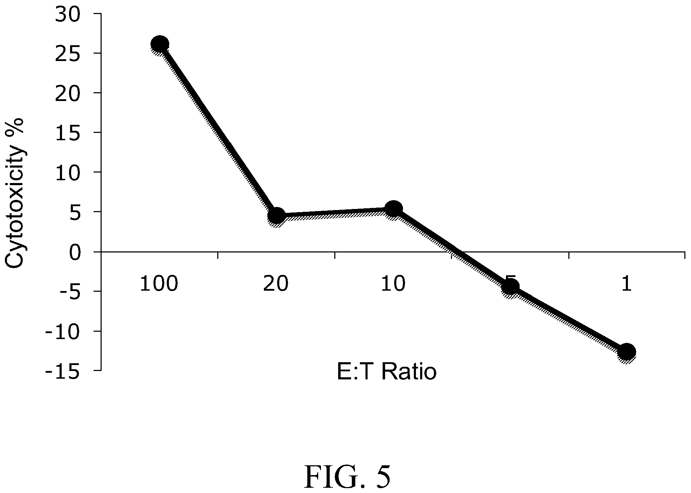

FIG. 5 depicts cytotoxicity of whole human placental perfusate, as obtained from the placenta, to KG-1a tumor cells in 24 hour co-culture at a ratio of 1:1, 5:1, 10:1 or 20:1 or 100:1 HPP cells to tumor cells. X axis: ratio of HPP cells to tumor cells. Y axis: percentage of dead tumor cells compared to tumor cells without HPP cells.

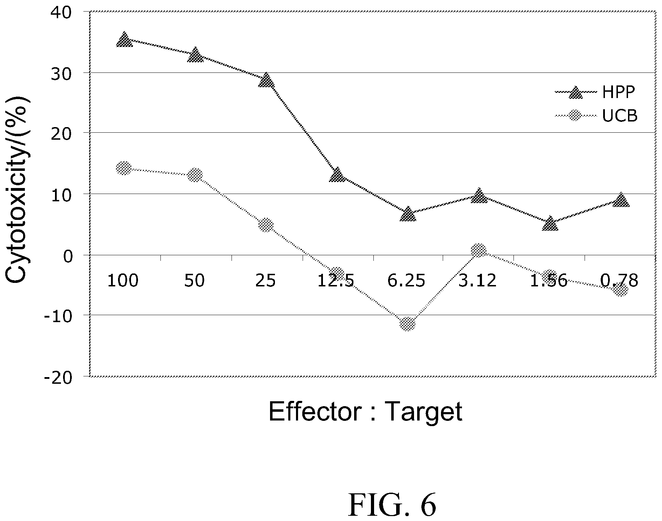

FIG. 6 depicts cytotoxicity of whole human placental perfusate, as obtained from the placenta, and umbilical cord blood, to KG-1a tumor cells in 48 hour co-culture in serial dilutions of 100:1, 50:1, 25:1, 12.5:1, 6.25:1, 3.12:1, 1.56:1 or 0.78:1 HPP cells or UCB cells to tumor cells. X axis: ratio of HPP cells or umbilical cord cells to tumor cells. Y axis: percentage of dead tumor cells after 48 hours culture time compared to tumor cells without HPP cells or umbilical cord cells.

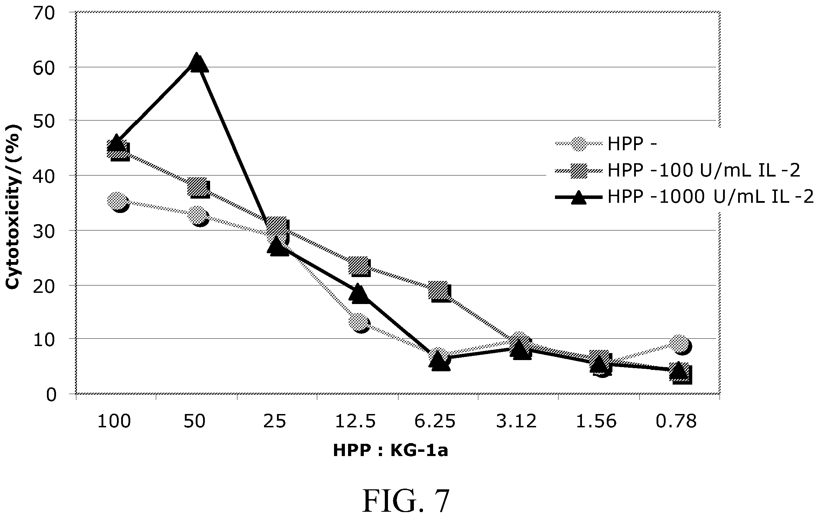

FIG. 7 depicts cytotoxicity of whole human placental perfusate, as obtained from the placenta to KG-1a tumor cells in 48 hour co-culture in serial dilutions of 100:1, 50:1, 25:1, 12.5:1, 6.25:1, 3.12:1, 1.56:1 or 0.78:1 HPP cells to tumor cells. Perfusate was either used as collected, or stimulated for 24 hours with 100 U/mL or 1000 U/mL interleukin-2 (IL-2). X axis: ratio of HPP cells to tumor cells. Y axis: percentage of dead tumor cells after 48 hours culture time compared to tumor cells without HPP cells.

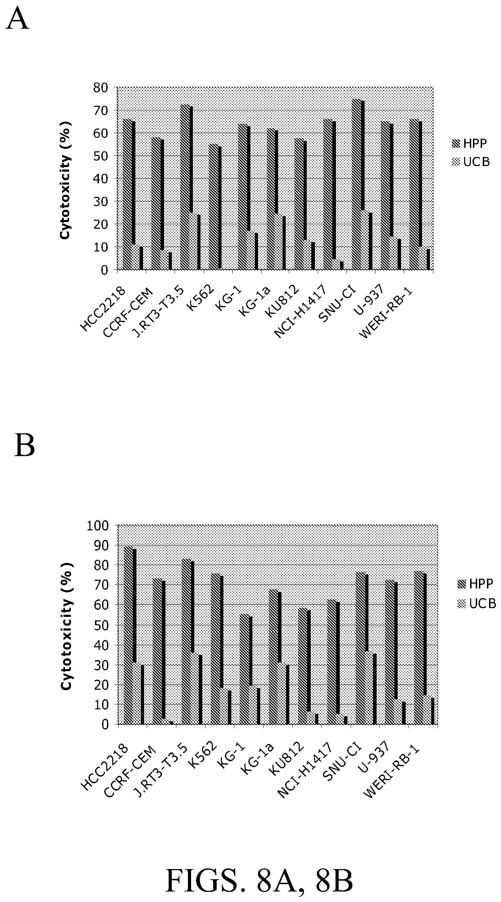

FIGS. 8A and 8B depict the cytotoxic effect of human placental perfusate towards a panel of tumor cell lines after culture with HPP or UCB cells at a 50:1 ratio to the tumor cells. FIG 8A: co-culture for 24 hours. FIG. 8B: co-culture for 48 hours. X axis: tumor cell line tested. Y axis: percentage of dead tumor cells after co-culture, compared to the number of tumor cells in the absence of tumor cells.

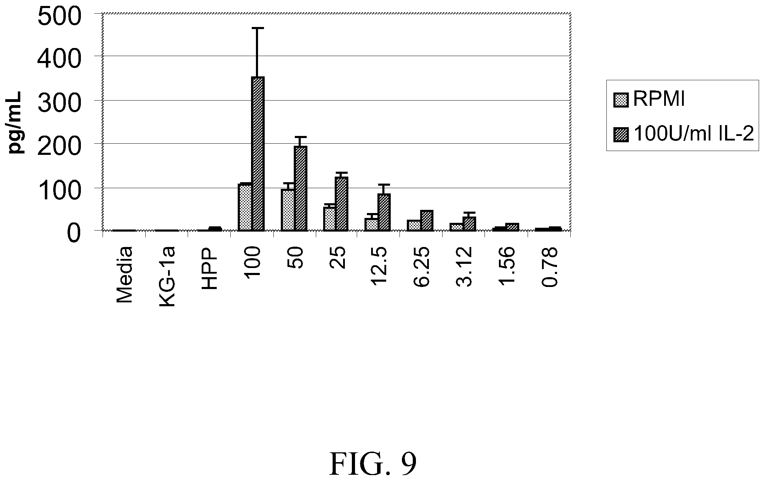

FIG. 9 depicts IFNγ production by HPP cells co-cultured with KG-1a tumor cells at different ratios of HPP cells to tumor cells. X axis: Experimental conditions, including ratio of HPP cells to tumor cells Y axis: IFNγ levels per milliliter after 24 hours co-culture..

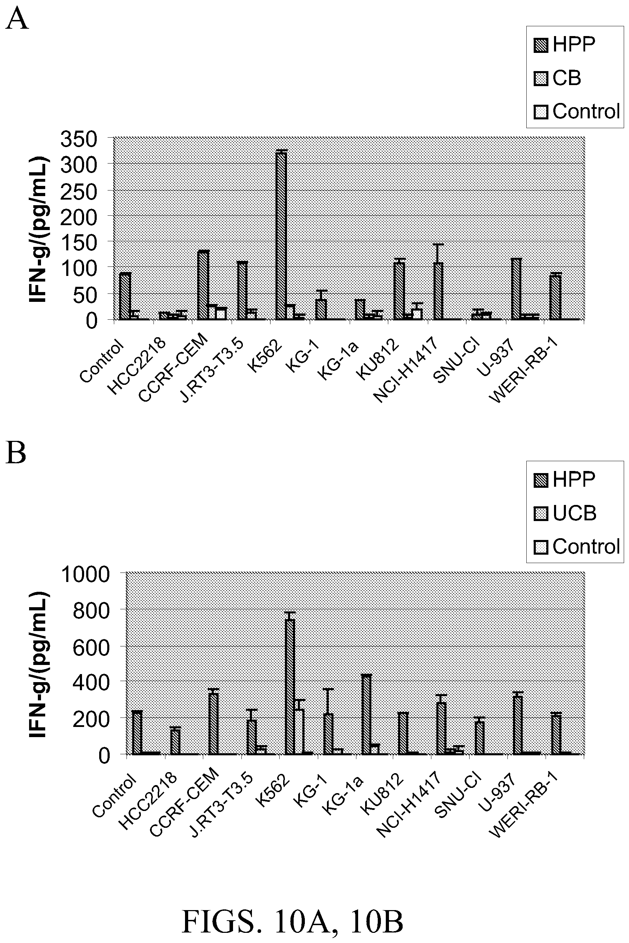

FIGS. 10A and 10B Production of IFNγ by HPP or UCB cells in co-culture with a panel of tumor cells. HPP or UCB cells were co-cultured at a ratio of 50:1 with tumor cell lines for 24 hours (FIG. 10A) or 48 hours (FIG. 10B). IFNγ levels were determined by Luminex assay (HCYTO-60K-03, Millipore). X axis: tumor cell line tested. Y axis: picograms of IFNγ produced by HPP or UCB cells, compared to picograms of IFNγ produced in the absence of tumor cells.

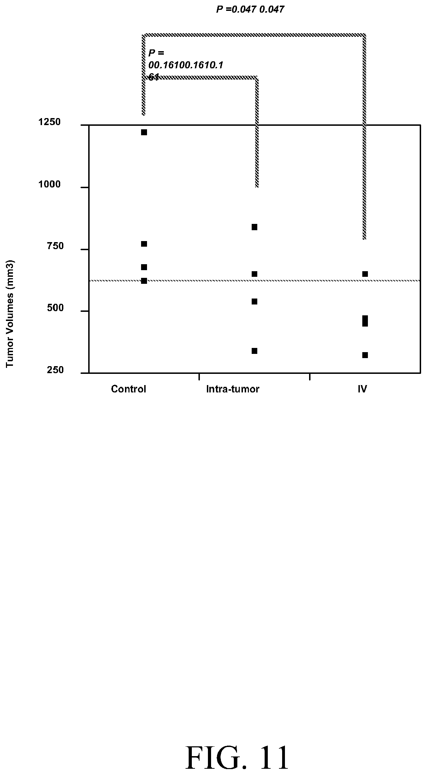

FIG. 11 depicts the reduction in tumor size upon administration of 2 x 10⁷ human placental perfusate (HPP) cells to mice having KG-1 cell tumors approximately 332 mm³ in volume. Intra-tumor - HPP cells were injected directly into the subcutaneous tumor site. IV - HPP cells administered intravenously. Control - vehicle administration only. Tumor volumes in mm³.

### 5. DETAILED DESCRIPTION

The present invention provides isolated natural killer cells comprising isolated CD56⁺, CD16⁻, CD3⁻ placental intermediate natural killer (PINK) cells for use in a method for treatment of an individual having cancer, wherein the method comprises administering to said individual said isolated natural killer cells comprising isolated CD56⁺, CD16⁻, CD3⁻ PINK cells, wherein said PINK cells are isolated from placental perfusate and express one or more of the microRNAs hsa-miR-100, hsa-miR-127, hsa-miR-211, hsa-miR-302c, hsa-miR-326, hsa-miR-337, hsa-miR-497, hsa-miR-512-3p, hsa-miR-515-5p, hsa-miR-517b, hsa-miR-517c, hsa-miR-518a, hsa-miR-518e, hsa-miR-519d, hsa-miR-520g, hsa-miR-520h, hsa-miR-564, hsa-miR-566, hsa-miR-618, or hsa-miR-99a at a detectably higher level than peripheral blood natural killer cells; and
wherein said cancer is acute promyelocytic leukemia, acute myeloblastic leukemia, acute megakaryoblastic leukemia, precursor B acute lymphoblastic leukemia, precursor T acute lymphoblastic leukemia, Burkitt's leukemia (Burkitt's lymphoma), acute biphenotypic leukemia, chronic monocytic leukemia, chronic lymphocytic leukemia (CLL)/Small lymphocytic lymphoma, B-cell prolymphocytic leukemia; hairy cell lymphoma; T-cell prolymphocytic leukemia, lymphoplasmacytic lymphoma, Waldenström macroglobulinemia, splenic marginal zone lymphoma, plasmacytoma, a monoclonal immunoglobulin deposition disease, or a heavy chain disease, extranodal marginal zone B cell lymphoma (MALT lymphoma), nodal marginal zone B cell lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, T cell large granular lymphocytic leukemia, aggressive NK cell leukemia, extranodal NK/T cell lymphoma, nasal type, enteropathy-type T cell lymphoma, hepatosplenic T cell lymphoma, blastic NK cell lymphoma, mycosis fungoides (Sezary syndrome), a primary cutaneous CD30-positive T cell lymphoproliferative disorder, primary cutaneous anaplastic large cell lymphoma, lymphomatoid papulosis, angioimmunoblastic T cell lymphoma, peripheral T cell lymphoma, unspecified, anaplastic large cell lymphoma, a Hodgkin lymphoma, or a nodular lymphocyte-predominant Hodgkin lymphoma.

Hereinafter, reference to natural killer cells within the context of the invention is to be understood as reference to natural killer cells comprising PINK cells of the invention. Consistent with this, hereinafter reference to PINK cells within the context of the invention is to be understood as reference to PINK cells as defined above, also referred to as PINK cells of the invention.

In one embodiment, the method additionally comprises administering to said individual an effective amount of lenalidomide, pomalidomide, or thalidomide. In one embodiment, said isolated natural killer cells have been contacted with pomalidomide, lenalidomide, or thalidomide prior to said administering.

In another embodiment, the isolated natural killer cells comprise natural killer cells not obtained from placental perfusate.

The present invention also provides isolated natural killer cells comprising isolated CD56⁺, CD16⁻, CD3⁻ PINK cells for use in a method for treatment of an individual having a viral infection, wherein the method comprises administering to said individual said isolated natural killer cells comprising isolated PINK cells of the invention.

In one embodiment, the method additionally comprises administering to said individual an effective amount of lenalidomide, pomalidomide, or thalidomide. In another embodiment, the isolated natural killer cells have been contacted with pomalidomide, lenalidomide, or thalidomide prior to said administering.

In another embodiment, the isolated natural killer cells comprise natural killer cells not obtained from placental perfusate.

Further, in one embodiment, the isolated natural killer cells are combined natural killer cells that comprise natural killer cells isolated from placental perfusate and natural killer cells isolated from umbilical cord blood. In a specific embodiment, said umbilical cord blood is isolated from the placenta from which said placental perfusate is obtained.

In a specific embodiment, the isolated natural killer cells are combined natural killer cells that comprise: a detectably higher number of CD3⁻CD56⁺CD16⁻ natural killer cells than an equivalent number of natural killer cells from peripheral blood;
a detectably lower number of CD3⁻CD56⁺CD16⁺ natural killer cells than an equivalent number of natural killer cells from peripheral blood; a detectably higher number of CD3⁻CD56⁺KIR2DL2/L3⁺ natural killer cells than an equivalent number of natural killer cells from peripheral blood; a detectably lower number of CD3⁻CD56⁺NKp46⁺ natural killer cells than an equivalent number of natural killer cells from peripheral blood; a detectably higher number of CD3⁻CD56⁺NKp30⁺ natural killer cells than an equivalent number of natural killer cells from peripheral blood; a detectably higher number of CD3⁻CD56⁺2B4⁺ natural killer cells than an equivalent number of natural killer cells from peripheral blood; or a detectably higher number of CD3⁻CD56⁺CD94⁺ natural killer cells than an equivalent number of natural killer cells from peripheral blood.

In another embodiment, the isolated natural killer cells are contacted with said pomalidomide, lenalidomide, or thalidomide in an amount and for a time sufficient for said natural killer cells to express detectably more granzyme B, or mRNA encoding granzyme B, than an equivalent number of natural killer cells not contacted with said pomalidomide, lenalidomide, or thalidomide. In yet another embodiment, the isolated natural killer cells for use in the present invention have not been cultured prior to said administering.

In a specific embodiment, the PINK cells express one or more of aminopeptidase N protein, apolipoprotein E protein, atrophin-1 interacting protein 1, innexin inx-3 protein, integrin alpha-2 precursor protein, integrin beta-5 precursor, mast cell surface glycoprotein GP49B precursor protein, or ryanodine receptor 1 protein; and wherein said placental intermediate natural killer cells do not express one or more of fibroblast growth factor receptor 4 precursor protein, immunity-associated nucleotide 4-like protein, integrin alpha-L precursor protein, integrin beta 2 precursor protein, integrin beta 4 precursor protein, membrane-bound lytic murein transglycosylase D precursor protein, oxysterol binding protein-related protein 8, or perforin 1 precursor 1 protein.

Provided herein are natural killer cells comprising, placenta-derived natural killer ("PINK") cells obtained from placental perfusate optionally in combination with an immunomodulatory compound, *e.g.,* an immunomodulatory compound disclosed in Section 5.9, below, or thalidomide for use in a method for treating an individual having cancer, *e.g.,* a blood cancer or a solid tumor, or a viral infection, or to suppress the growth or proliferation of a tumor cell or plurality of tumor cells. The natural killer cells may be obtained from any source, for example, without limitation, placenta, umbilical cord blood, placental blood, peripheral blood, spleen, liver. The natural killer cells, *e.g.,* PINK cells, may be either autologous or heterologous to a recipient (*e.g.,* an individual having cancer or a viral infection), or may be from the same individual as or a different individual from, the individual comprising tumor cells to be suppressed. In certain embodiments, the natural killer (NK) cells isolated from placental perfusate, *e.g.,* human placental perfusate, or NK cells that have been isolated from placental tissue that has been disrupted mechanically and/or enzymatically. Methods of using the placental perfusate, placental perfusate-derived cells or placental perfusate-derived natural killer cells, *e.g.,* intermediate natural killer cells, to treat individuals having cancer, *e.g.,* a blood cancer or a solid tumor, or a viral infection, or in the suppression of the proliferation of tumor cells, are described in Section 5.1, below. Methods of obtaining placental perfusate, and obtaining cells from placental perfusate, are described in Section 5.2, below. Methods of obtaining placental natural killer cells by disruption of placental tissue are described in Section 5.3, below. Disruption of placental tissue is described in Section 5.4, below. Characteristics of placental natural killer cells are described in Section 5.5, below. Natural killer cells from matched placenta and umbilical cord are described in Section 5.6, below. Combinations of placental perfusate or placental perfusate cells and other cells, useful in the methods of treatment or tumor suppression described herein, are described in Section 5.7, below. Preservation of placental perfusate and perfusate cells are described in Section 5.8, below. Immunomodulatory compounds for use in the methods disclosed herein are described in Section 5.9, below. Administration of the cells useful in the methods disclosed herein are described in Section 5.10, below.

### 5.1. Use of Placental Perfusate or Natural Killer Cells and

### Immunomodulatory Compounds in Treatment of Cancer or Viral Infection and Suppression of Tumor Cell Growth

In one aspect, the invention generally relates to suppressing the growth, *e.g.,* proliferation, of tumor cells comprising contacting the tumor cells with an effective amount of isolated placental perfusate, isolated placental perfusate cells, isolated natural killer cells, *e.g.,* placental natural killer cells, *e.g.,* placenta-derived intermediate natural killer cells, isolated combined natural killer cells, and/or combinations thereof. The tumor cells can be tumor cells *in vitro,* or can be tumor cells *in vivo, e.g.,* in a mammal, *e.g.,* a human, that has cancer, for example, a blood cancer or solid tumor.

The invention also generally relates to treating an individual having a deficiency of natural killer cells, *e.g.* a deficiency in the absolute number of natural killer cells or a deficiency in the number of functional natural killer cells. In certain embodiments, the deficiency of natural killer cells in the individual is associated with, or leads to, a viral infection, cancer, *e.g.,* a blood cancer or a solid tumor, in the individual. The presence of a viral infection or cancer in the individual, however, may or may not be related to a deficiency of the individual's natural killer cells. In a specific embodiment, said deficiency in natural killer cells arises from a cause related to said cancer or viral infection. In another embodiment, said deficiency in natural killer cells arises from a cause not related to said cancer or viral infection.

### 5.1.1. Treatment of Cancers

In one embodiment, provided herein are isolated natural killer cells comprising isolated CD56+, CD16-, CD3- PINK cells for use in a method of treating an individual having a cancer, for example, a blood cancer or a solid tumor, *e.g.,* an individual having a deficiency of natural killer cells, comprising administering to said individual an effective amount of said isolated natural killer cells, *e.g.,* placental natural killer cells, *e.g.,* placenta-derived intermediate natural killer cells, isolated combined natural killer cells, and/or combinations thereof. In a specific embodiment, the method comprises additionally administering to said individual an effective amount of an immunomodulatory compound, *e.g.,* an immunomodulatory compound described in Section 5.9, below, or thalidomide, wherein said effective amount is an amount that, *e.g.,* results in a detectable improvement of, lessening of the progression of, or elimination of, one or more symptoms of a cancer from which the individual suffers.

According to the present invention, the cancer is acute promyelocytic leukemia, acute myeloblastic leukemia, acute megakaryoblastic leukemia, precursor B acute lymphoblastic leukemia, precursor T acute lymphoblastic leukemia, Burkitt's leukemia (Burkitt's lymphoma), or acute biphenotypic leukemia; chronic monocytic leukemia, chronic lymphocytic leukemia (CLL)/Small lymphocytic lymphoma, or B-cell prolymphocytic leukemia; hairy cell lymphoma; T-cell prolymphocytic leukemia; lymphoplasmacytic lymphoma (*e.g.,* Waldenström macroglobulinemia), splenic marginal zone lymphoma, plasmacytoma, a monoclonal immunoglobulin deposition disease, or a heavy chain disease), extranodal marginal zone B cell lymphoma (MALT lymphoma), nodal marginal zone B cell lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, T cell large granular lymphocytic leukemia, aggressive NK cell leukemia, extranodal NK/T cell lymphoma, nasal type, enteropathy-type T cell lymphoma, hepatosplenic T cell lymphoma, blastic NK cell lymphoma, mycosis fungoides (Sezary syndrome), a primary cutaneous CD30-positive T cell lymphoproliferative disorder (*e.g.,* primary cutaneous anaplastic large cell lymphoma or lymphomatoid papulosis), angioimmunoblastic T cell lymphoma, peripheral T cell lymphoma, unspecified, anaplastic large cell lymphoma, a Hodgkin lymphoma or a nodular lymphocyte-predominant Hodgkin lymphoma. In another aspect of the disclosure, the cancer is multiple myeloma or myelodysplastic syndrome.

In certain embodiments, the individual having a cancer, for example, a blood cancer or a solid tumor, *e.g.,* an individual having a deficiency of natural killer cells, is an individual that has received a bone marrow transplant before said administering. In certain embodiments, the bone marrow transplant was in treatment of said cancer. In certain other embodiments, the bone marrow transplant was in treatment of a condition other than said cancer. In certain embodiments, the individual received an immunosuppressant in addition to said bone marrow transplant. In certain embodiments, the individual who has had a bone marrow transplant exhibits one or more symptoms of graft-versus-host disease (GVHD) at the time of said administration. In certain other embodiments, the individual who has had a bone marrow transplant is administered said cells before a symptom of graft-versus-host disease (GVHD) has manifested.

In certain other embodiments, the individual having a cancer, for example, a blood cancer, has received at least one dose of a TNFα inhibitor, *e.g.,* ETANERCEPT® (Enbrel), prior to said administering. In specific embodiments, said individual received said dose of a TNFα inhibitor within 1, 2, 3, 4, 5, 6, 7, 8, 9, 10, 11 or 12 months of diagnosis of said cancer. In a specific embodiment, the individual who has received a dose of a TNFα inhibitor exhibits acute myeloid leukemia. In a more specific embodiment, the individual who has received a dose of a TNFα inhibitor and exhibits acute myeloid leukemia further exhibits deletion of the long arm of chromosome 5 in blood cells. In another embodiment, the individual having a cancer, for example, a blood cancer, exhibits a Philadelphia chromosome.

In certain other embodiments, the cancer, for example, a blood cancer or a solid tumor, in said individual is refractory to one or more anticancer drugs. In a specific embodiment, the cancer is refractory to GLEEVEC® (imatinib mesylate).

In certain embodiments, the cancer, for example, a blood cancer, in said individual responds to at least one anticancer drug; in this embodiment, placental perfusate, isolated placental perfusate cells, isolated natural killer cells, *e.g.,* placental natural killer cells, *e.g.,* placenta-derived intermediate natural killer cells, isolated combined natural killer cells, and/or combinations thereof, and optionally an immunomodulatory compound, are added as adjunct treatments or as a combination therapy with said anticancer drug. In certain other embodiments, the individual having a cancer, for example, a blood cancer, was treated with at least one anticancer drug, and has relapsed, prior to said administering.

### 5.1.2. Treatment of Viral Infection

In another embodiment, provided herein are isolated natural killer cells comprising isolated CD56+, CD16-, CD3- PINK cells for use in a method of treating an individual having a viral infection, *e.g.,* an individual having a deficiency of natural killer cells, comprising administering to said individual an effective amount of said isolated natural killer cells, *e.g.,* placental natural killer cells, *e.g.,* placenta-derived intermediate natural killer cells, isolated combined natural killer cells, and/or combinations thereof, and optionally an immunomodulatory compound, *e.g.,* an immunomodulatory compound described in Section 5.9, below, or thalidomide, wherein said amount is an amount that, *e.g.,* results in a detectable improvement of, lessening of the progression of, or elimination of, one or more symptoms of said viral infection. In specific embodiments, the viral infection is an infection by a virus of the Adenoviridae, Picornaviridae, Herpesviridae, Hepadnaviridae, Flaviviridae, Retroviridae, Orthomyxoviridae, Paramyxoviridae, Papilommaviridae, Rhabdoviridae, or Togaviridae family. In more specific embodiments, said virus is human immunodeficiency virus (HIV).coxsackievirus, hepatitis A virus (HAV), poliovirus, Epstein-Barr virus (EBV), herpes simplex type 1 (HSV1), herpes simplex type 2 (HSV2), human cytomegalovirus (CMV), human herpesvirus type 8 (HHV8), herpes zoster virus (varicella zoster virus (VZV) or shingles virus), hepatitis B virus (HBV), hepatitis C virus (HCV), hepatitis D virus (HDV), hepatitis E virus (HEV), influenza virus (e.g., influenza A virus, influenza B virus, influenza C virus, or thogotovirus), measles virus, mumps virus, parainfluenza virus, papillomavirus, rabies virus, or rubella virus.

In other more specific embodiments, said virus is adenovirus species A, serotype 12, 18, or 31; adenovirus species B, serotype 3, 7, 11, 14, 16, 34, 35, or 50; adenovirus species C, serotype 1, 2, 5, or 6; species D, serotype 8, 9, 10, 13, 15, 17, 19, 20, 22, 23, 24, 25, 26, 27, 28, 29, 30, 32, 33, 36, 37, 38, 39, 42, 43, 44, 45, 46, 47, 48, 49, or 51; species E, serotype 4; or species F, serotype 40 or 41.

In certain other more specific embodiments, the virus is Apoi virus (APOIV), Aroa virus (AROAV), bagaza virus (BAGV), Banzi virus (BANV), Bouboui virus (BOUV), Cacipacore virus (CPCV), Carey Island virus (CIV), Cowbone Ridge virus (CRV), Dengue virus (DENV), Edge Hill virus (EHV), Gadgets Gully virus (GGYV), Ilheus virus (ILHV), Israel turkey meningoencephalomyelitis virus (ITV), Japanese encephalitis virus (JEV), Jugra virus (JUGV), Jutiapa virus (JUTV), kadam virus (KADV), Kedougou virus (KEDV), Kokobera virus (KOKV), Koutango virus (KOUV), Kyasanur Forest disease virus (KFDV), Langat virus (LGTV), Meaban virus (MEAV), Modoc virus (MODV), Montana myotis leukoencephalitis virus (MMLV), Murray Valley encephalitis virus (MVEV), Ntaya virus (NTAV), Omsk hemorrhagic fever virus (OHFV), Powassan virus (POWV), Rio Bravo virus (RBV), Royal Farm virus (RFV), Saboya virus (SABV), St. Louis encephalitis virus (SLEV), Sal Vieja virus (SVV), San Perlita virus (SPV), Saumarez Reef virus (SREV), Sepik virus (SEPV), Tembusu virus (TMUV), tick-borne encephalitis virus (TBEV), Tyuleniy virus (TYUV), Uganda S virus (UGSV), Usutu virus (USUV), Wesselsbron virus (WESSV), West Nile virus (WNV), Yaounde virus (YAOV), Yellow fever virus (YFV), Yokose virus (YOKV), or Zika virus (ZIKV).

### 5.1.3. Suppression of Tumor Cell Proliferation

Further the above methode of cancer treatment comprises suppressing the proliferation of tumor cells, after contacting the tumor cells with isolated natural killer cells, comprising isolated CD56+, CD16-, CD3- placenta-derived intermediate natural killer, PINK cells, isolated combined natural killer cells, and/or combinations thereof. In a specific embodiment, the tumor cells are additionally contacted with an immunomodulatory compound, *e.g.,* an immunomodulatory compound described in Section 5.9, below, or thalidomide, such that proliferation of the tumor cells is detectably reduced compared to tumor cells of the same type not contacted with the isolated natural killer cells, comprising isolated CD56+, CD16-, CD3-placenta-derived intermediate natural killer cells, isolated combined natural killer cells, and/or combinations thereof. In another aspect, said natural killer cells are not placental natural killer cells, *e.g.,* are not PINK cells.

As used herein, "contacting," with respect to cells, in one embodiment encompasses direct physical, *e.g.,* cell-cell, contact between natural killer cells, comprising said placental intermediate natural killer cells, and/or isolated combined natural killer cells and tumor cells. In another embodiment, "contacting" encompasses presence in the same physical space, *e.g.,* natural killer cells comprising said placental intermediate natural killer cells, and/or isolated combined natural killer cells are placed in the same container *e.g.,* culture dish, multiwell plate) as tumor cells. In another embodiment, "contacting" combined natural killer cells or placental intermediate natural killer cells, and tumor cells is accomplished, *e.g.,* by injecting or infusing the combined natural killer cells or natural killer cells comprising said placental intermediate natural killer cells into an individual, *e.g.,* a human comprising tumor cells, *e.g.,* a cancer patient. "Contacting," in the context of immunomodulatory compounds and/or thalidomide, means, *e.g.,* that the cells and the immunomodulatory compound and/or thalidomide are directly physically contacted with each other, or are placed within the same physical volume (*e.g.,* a cell culture container or an individual).

In certain aspects of the disclosure, placental perfusate is used in any amount that results in a detectable therapeutic benefit to an individual comprising tumor cells, *e.g.,* a cancer patient. In certain other embodiments, natural killer cells comprising said placental intermediate natural killer cells, and/or combined natural killer cells, or combinations thereof, are used in any amount that results in a detectable therapeutic benefit to an individual comprising tumor cells. Thus, in another embodiment, provided herein is a method of suppressing the proliferation of tumor cells comprising contacting the tumor cells with natural killer cells comprising said placenta-derived intermediate natural killer cells, within an individual such that said contacting is detectably or demonstrably therapeutically beneficial to said individual. In a specific embodiment, the method additionally comprises contacting the tumor cells with an immunomodulatory compound, *e.g.,* an immunomodulatory compound described in Section 5.9, below, or thalidomide, such that said contacting is detectably or demonstrably therapeutically beneficial to said individual.

In a specific aspect of the disclosure, the tumor cells are blood cancer cells. In various specific embodiments, the tumor cells are primary ductal carcinoma cells, leukemia cells, acute T cell leukemia cells, chronic myeloid lymphoma (CML) cells, acute myelogenous leukemia cells, chronic myelogenous leukemia (CML) cells, lung carcinoma cells, colon adenocarcinoma cells, histiocytic lymphoma cells, multiple myeloma cell, retinoblastoma cells, colorectal carcinoma cells or colorectal adenocarcinoma cells. In other specific embodiments, the tumor cells can be cells of any of the cancer or tumor types described in Section 5.1.1, above.

As used herein, "therapeutically beneficial" and "therapeutic benefits" include, but are not limited to, *e.g.,* reduction in the size of a tumor; lessening or cessation of expansion of a tumor; reduction in the number of cancer cells in a tissue sample, *e.g.,* a blood sample, per unit volume; the clinical improvement in any symptom of the particular cancer or tumor said individual has, the lessening or cessation of worsening of any symptom of the particular cancer the individual has, *etc.* Contacting of placental perfusate, placental perfusate cells and/or natural killer cells, *e.g.,* PINK cells with tumor cells, optionally with an immunomodulatory compound, *e.g.,* an immunomodulatory compound described in Section 5.9, above, or thalidomide, that accomplishes any one or more of such therapeutic benefits, is said to be therapeutically beneficial.

### 5.1.4. Administration

Determination of the number of cells, *e.g.,* placental perfusate cells, *e.g.,* nucleated cells from placental perfusate, combined natural killer cells, and/or isolated natural killer cells, *e.g.,* placental intermediate natural killer cells, and determination of the amount of an immunomodulatory compound, *e.g.,* an immunomodulatory compound in Section 5.9, below, or thalidomide, can be determined independently of each other.

### 5.1.4.1. Administration of Cells

In certain embodiments, combined natural killer cells, and/or isolated natural killer cells comprising isolated CD56+ CD16- CD3-intermediate natural kille (PINK) cells, are used, *e.g.,* administered to an individual, in any amount or number that results in a detectable therapeutic benefit to the individual, *e.g.,* an effective amount, wherein the individual has a viral infection, cancer, or tumor cells, for example, an individual having tumor cells, a solid tumor or a blood cancer, *e.g.,* a cancer patient. Such cells can be administered to such an individual by absolute numbers of cells, *e.g.,* said individual can be administered at about, at least about, or at most about, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, or 1 x 10¹¹ combined natural killer cells and/or natural killer cells comprising said PINK cells. In other embodiments, combined natural killer cells, and/or isolated natural killer cells, comprising isolated CD56+ CD16- CD3- placenta-derived intermediate natural killer cells can be administered to such an individual by relative numbers of cells, *e.g.,* said individual can be administered at about, at least about, or at most about, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, or 1 x 10¹¹ combined natural killer cells, and/or natural killer cells per kilogram of the individual. Placental perfusate cells and/or natural killer cells, *e.g.,* placenta-derived intermediate natural killer cells can be administered to such an individual according to an approximate ratio between a number of placental perfusate cells and/or natural killer cells, *e.g.,* placental intermediate natural killer cells, and a number of tumor cells in said individual. For example, placental perfusate cells and/or natural killer cells, *e.g.,* placental intermediate natural killer cells, can be administered to said individual in a ratio of about, at least about or at most about 1:1, 1:1, 3:1, 4:1, 5:1, 6:1, 7:1, 8:1, 9:1, 10:1, 15:1, 20:1, 25:1, 30:1, 35:1, 40:1, 45:1, 50:1, 55:1, 60:1, 65:1, 70:1, 75:1, 80:1, 85:1, 90:1, 95:1 or 100:1 to the number of tumor cells in the individual. The number of tumor cells in such an individual can be estimated, *e.g.,* by counting the number of tumor cells in a sample of tissue from the individual, *e.g.,* blood sample, biopsy, or the like. In specific embodiments, *e.g.,* for solid tumors, said counting is performed in combination with imaging of the tumor or tumors to obtain an approximate tumor volume. In a specific embodiment, an immunomodulatory compound or thalidomide, *e.g.,* an effective amount of an immunomodulatory compound or thalidomide, are administered to the individual.

In certain embodiments, the above method of cancer treatment or viral infection treatment comprises suppressing the proliferation of tumor cells, *e.g.,* in an individual; treatment of an individual having a deficiency in the individual's natural killer cells; or treatment of an individual having a viral infection; or treatment of an individual having cancer, *e.g.,* an individual having tumor cells, a blood cancer or a solid tumor, comprises contacting the tumor cells, or administering to said individual, combined natural killer cells, and/or isolated natural killer cells comprising isolated CD56+, CD16-, CD3- placented-derived intermediate natural killer cells. In specific embodiments, the method additionally comprises contacting the tumor cells, or administering to the individual, an immunomodulatory compound or thalidomide. For example, in various embodiments, the above described method comprises suppressing the proliferation of tumor cells, or treating an individual having cancer or a viral infection, or an individual having a deficiency in the individual's NK cells, comprising contacting said tumor cells with, or administering to said individual, an effective amount of isolated NK cells comprising said PINK cells; isolated NK cells comprising said PINK cells, and combined natural killer cells. In a specific embodiment, the above methods additionally comprise contacting the tumor cells with, or administering to said individual, an immunomodulatory compound or thalidomide.

In one specific embodiment, for example, treatment of an individual having a deficiency in the individual's natural killer cells; or treatment of an individual having a cancer or a viral infection, or suppression of tumor cell proliferation comprises contacting said tumor cells, or administering to said individual, combined natural killer cells and/or a plurality of NK cells, comprising CD56+, CD16-, CD3- placental intermediate natural killer cells. In some cases, for example, each milliliter of placental perfusate is supplemented with about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰ or more placental perfusate cells or isolated natural killer cells, *e.g.,* placental intermediate natural killer cells. In other examples, placental perfusate, *e.g.,* one unit (*i.e.,* the collection from a single placenta), or about 100, 150, 200, 250, 300, 350, 400, 450, 500, 550, 600, 650, 700, 750, 800, 850, 900, 950 or 1000 mL of perfusate, is supplemented with about 1 x 10⁴, 5 x 10⁴, 1 x 10⁵, 5 x 10⁵, 1 x 10⁶,5 x 10⁶, 1 x 10⁷, 5 x 10⁷, 1 x 10⁸, 5 x 10⁸ or more isolated NK cells, combined natural killer cells, and/or isolated placental perfusate cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more isolated NK cells, combined natural killer cells, and/or isolated placental perfusate cells. In another specific embodiment, the method additionally comprises contacting the tumor cells with, or administering to the individual, an immunomodulatory compound, or thalidomide.

In another specific embodiment, treatment of an individual having a deficiency in the individual's natural killer cells; or treatment of an individual having a cancer or a viral infection, or suppression of tumor cell proliferation comprises contacting the tumor cells, or administering to the individual, combined natural killer cells, and/or isolated NK cells comprising CD56+, CD16-, CD3- placental intermediate natural killer cells. For example, about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more isolated placental perfusate cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more isolated placental perfusate cells, are supplemented with about, or at least about, 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more isolated NK cells, *e.g.,* PINK cells, and/or combined natural killer cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more isolated NK cells, *e.g.,* PINK cells. In other more specific embodiments, about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more isolated NK cells comprising said PINK cells, combined natural killer cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more isolated NK cells comprising said PINK cells, and/or combined natural killer cells are supplemented with about, or at least about, 1, 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950 or 1000 mL of perfusate, or about 1 unit of perfusate. In another specific embodiment, the method additionally comprises contacting the tumor cells with, or administering to the individual, an immunomodulatory compound, or thalidomide.

In another specific embodiment, treatment of an individual having a deficiency in the individual's natural killer cells; treatment of an individual having cancer; treatment of an individual having a viral infection; or suppression of tumor cell proliferation, comprises contacting the tumor cells, or administering to the individual, isolated natural killer cells, comprising CD56+, CD16-, CD3- placental intermediate natural killer cells, supplemented by placental perfusate, isolated placental perfusate cells, and/or combined natural killer cells. In more specific embodiments, about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more isolated natural killer cells comprising said PINK cells, are supplemented with about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more natural killer cells, *e.g.,* PINK cells, and/or combined natural killer cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more placental perfusate cells and/or combined natural killer cells. In other more specific embodiments, 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more isolated NK cells comprising said placental intermediate natural killer cells, and/or combined natural killer cells are supplemented with about, or at least about, 1, 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950 or 1000 mL of perfusate, or about 1 unit of perfusate. In another specific embodiment, the method additionally comprises contacting the tumor cells, or administering to the individual an immunomodulatory compound or thalidomide.

In another specific embodiment, treatment of an individual having a deficiency in the individual's natural killer cells; treatment of an individual having cancer; treatment of an individual having a viral infection; or suppression of tumor cell proliferation, comprises contacting the tumor cells, or administering to the individual, isolated natural killer cells comprising CD56+, CD16-, CD3 placental intermediate natural killer cells, supplemented by placental perfusate, isolated placental perfusate cells, and/or combined natural killer cells, wherein said cells are supplemented with adherent placental cells, *e.g*., adherent placental stem cells or multipotent cells. In specific embodiments, the placental perfusate, perfusate cells, and/or PINK cells are supplemented with about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more adherent placental stem cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more adherent placental cells, *e.g*., adherent placental stem cells or multipotent cells. In another specific embodiment, treatment of an individual having a deficiency in the individual's natural killer cells; treatment of an individual having cancer; treatment of an individual having a viral infection; or suppression of tumor cell proliferation, is performed using an immunomodulatory compound or thalidomide in combination with isolated natural killer cells comprising said placental intermediate natural killer cells, supplemented by placental perfusate, isolated placental perfusate cells, and/or combined natural killer cells, wherein said cells are supplemented with adherent placental cell-conditioned medium, *e.g*., 0.1, 0.2, 0.3, 0.4, 0.5, 0.6, 0.1, 0.8, 0.9, 1, 2, 3, 4, 5, 6, 7, 8, 9, 10 mL of stem cell-conditioned culture medium per unit of perfusate, perfusate cells, combined natural killer cells, and/or PINK cells, or per 10⁴, 10⁵, 10⁶, 10⁷, 10⁸, 10⁹, 10¹⁰, or 10¹¹ cells. In certain embodiments, the adherent placental cells are the multipotent adherent placental cells described in U.S. Patent No. 7,468,276 and U.S. Patent Application Publication No. 2007/0275362 (U.S. Patent No. 8,057,788). In another specific embodiment, the method additionally comprises contacting the tumor cells, or administering to the individual, an immunomodulatory compound or thalidomide.

In other embodiments, the natural killer cells comprising CD56+, CD16+, CD16-, CD3- PINK cells, combined natural killer cells, and combinations and pools comprising the same, are used as initially obtained, that is, combined natural killer cells from such perfusate and matched umbilical cord blood, or PINK cells isolated from such perfusate or such placental perfusate cells. In other embodiments, the natural killer cells comprising said PINK cells, and combinations and pools of the same are processed prior to use. For example, placental perfusate can be used in its unprocessed form as collected from the placenta. Placental perfusate can also be processed prior to use, *e.g.,* by negative selection of one or more types of cells, reduction in volume by dehydration; lyophilization and rehydration, *etc.* Similarly, populations of perfusate cells can be used as initially isolated from placental perfusate, *e.g*., as total nucleated cells from placental perfusate, or can be processed, *e.g.,* to remove one or more cell types (*e.g.,* erythrocytes). Natural killer cells can be used as initially isolated from a tissue source; *e.g*., PINK cells can be used as initially isolated from placental perfusate, for example, using CD56 microbeads, or can be processed, *e.g.,* to remove one or more non-killer cell types.

In another aspect of the disclosure, treatment of an individual having a deficiency in the individual's natural killer cells; treatment of an individual having cancer; treatment of an individual having a viral infection; or suppression of tumor cell proliferation, using isolated natural killer cells comprising CD56+, CD16-, CD3- placental intermediate natural killer cells, supplemented by placental perfusate, isolated placental perfusate cells, and/or combined natural killer cells, or pools or combinations comprising the same, further comprises contacting said cells or perfusate with interleukin-2 (IL-2) for a period of time prior to said contacting. In certain embodiments, said period of time is about, at least, or at most 1, 2, 3, 4, 5, 6, 7, 8, 9, 10, 12, 14, 16, 18, 20, 22, 24, 26, 28, 30, 32, 34, 36, 38, 40, 42, 44, 46 or 48 hours prior to said contacting.

The natural killer cells comprising said PINK cells, combined natural killer cells, or pools and/or combinations of the same, and optionally immunomodulatory compound or thalidomide, can be administered once to an individual having a viral infection, an individual having cancer, or an individual having tumor cells during a course of anticancer therapy; or can be administered multiple times, *e.g*., once every 1, 2, 3, 4, 5, 6, 7, 8, 9, 10, 11, 12, 13, 14, 15, 16, 17, 18, 19, 20, 21, 22 or 23 hours, or once every 1, 2, 3, 4, 5, 6 or 7 days, or once every 1, 2, 3, 4, 5, 6, 7, 8, 9, 10 or more weeks during therapy. In embodiments in which cells and an immunomodulatory compound or thalidomide are used, the immunomodulatory compound or thalidomide, and cells, can be administered to the individual together, *e.g.,* in the same formulation; separately, *e.g.,* in separate formulations, at approximately the same time; or can be administered separately, *e.g*., on different dosing schedules or at different times of the day. The natural killer cells, comprising said PINK cells can be administered without regard to whether perfusate, perfusate cells, natural killer cells, *e.g.,* PINK cells, pools and/or combinations of the same have been administered to the individual in the past. Thus, the methods provided herein encompasses the administration to a person having a viral infection, having cancer, or having tumor cells any combination of natural killer cells comprising said PINK cells.

The placental perfusate, perfusate cells, natural killer cells, *e.g.,* PINK cells, combined natural killer cells, pools, and/or combinations comprising the same, and optionally immunomodulatory compound or thalidomide, can be part of an anticancer therapy regimen that includes one or more other anticancer agents. Such anticancer agents are well-known in the art. Specific anticancer agents that may be administered to an individual having cancer, *e.g.,* an individual having tumor cells, in addition to the perfusate, perfusate cells, natural killer cells, *e.g.,* PINK cells, pools and/or combinations of the same, and optionally immunomodulatory compound or thalidomide, include, but are not limited to: acivicin; aclarubicin; acodazole hydrochloride; acronine; adozelesin; aldesleukin; altretamine; ambomycin; ametantrone acetate; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; celecoxib (COX-2 inhibitor); chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflomithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; iproplatin; irinotecan; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; taxotere; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; and zorubicin hydrochloride.

Other anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidenmin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; doxorubicin; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imatinib (*e.g.*, GLEEVEC®), imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide + estrogen + progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mitoguazone; mitolactol; mitomycin analogues; mitonafide; mitotoxin fibroblast growth factor-saporin; mitoxantrone; mofarotene; molgramostim; Erbitux, human chorionic gonadotrophin; monophosphoryl lipid A+myobacterium cell wall sk; mopidamol; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; nilutamide; nisamycin; nitric oxide modulators; nitroxide antioxidant; nitrullyn; oblimersen (GENASENSE®); O⁶-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stipiamide; stromelysin inhibitors; sulfinosine; superactive vasoactive intestinal peptide antagonist; suradista; suramin; swainsonine; tallimustine; tamoxifen methiodide; tauromustine; tazarotene; tecogalan sodium; tegafur; tellurapyrylium; telomerase inhibitors; temoporfin; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; velaresol; veramine; verdins; verteporfin; vinorelbine; vinxaltine; vitaxin; vorozole; zanoterone; zeniplatin; zilascorb; and zinostatin stimalamer.

In another embodiment, natural killer cells, comprising CD56, CD16-, CD3- PINK cells, combined natural killer cells, and optionally immunomodulatory compound or thalidomide, can be administered to an individual having a viral infection as part of an antiviral therapy regimen that includes one or more other antiviral agents. Specific antiviral agents that may be administered to an individual having cancer, in addition to the natural killer cells, comprising said PINK cells, or combinations of natural killer cells, include, but are not limited to: imiquimod, podofilox, podophyllin, interferon alpha (IFNα), reticolos, nonoxynol-9, acyclovir, famciclovir, valaciclovir, ganciclovir, cidofovir; amantadine, rimantadine; ribavirin; zanamavir and oseltaumavir; protease inhibitors such as indinavir, nelfinavir, ritonavir, or saquinavir; nucleoside reverse transcriptase inhibitors such as didanosine, lamivudine, stavudine, zalcitabine, or zidovudine; and non-nucleoside reverse transcriptase inhibitors such as nevirapine, or efavirenz.

### 5.2. Isolation of Natural Killer Cells

Methods of isolating natural killer cells are known in the art. Natural killer cells can be isolated or enriched by staining cells from a tissue source, *e.g*., peripheral blood, with antibodies to CD56 and CD5, and selecting for CD56⁺CD5⁻ cells. Natural killer cells can be isolated using a commercially available kit, for example, the NK Cell Isolation Kit (Miltenyi Biotec). Natural killer cells can also be isolated or enriched by removal of non-natural killer cells in a population of cells that comprise the natural killer cells. For example, natural killer cells from peripheral blood may be isolated or enriched by depletion of T cells, B cells, monocytes, dendritic cells, platelets, granulocytes and erythrocytes from the peripheral blood using, *e.g*., antibodies to each of the cell populations to be excluded from the isolated or enriched natural killer cells. In certain embodiments, the antibodies comprise antibodies to CD3, CD14, CD36, CDw123, HLA Class II DR,DP and CD235a (glycophorin A). Negative isolation can be carried out using a commercially available kit, *e.g.,* the NK Cell Negative Isolation Kit (Dynal Biotech). Cells isolated by these methods may be additionally sorted, *e.g.,* to separate CD16⁺ and CD16⁻ cells.

Specific methods of isolating natural killer cells from placenta are described below.

### 5.3. Placental Perfusate

### 5.3.1. Cell Collection Composition

The placental perfusate-derived natural killer cells, useful in tumor suppression or the treatment of an individual having tumor cells, cancer or a viral infection, as provided herein, can be collected by perfusion of a mammalian, *e.g*., human post-partum placenta using a placental cell collection composition. Perfusate can be collected from the placenta by perfusion of the placenta with any physiologically-acceptable solution, *e.g*., a saline solution, culture medium, or a more complex cell collection composition. A cell collection composition suitable for perfusing a placenta, and for the collection and preservation of perfusate cells, *e.g.,* total nucleated placental perfusate cells or PINK cells, is described in detail in related U.S. Application Publication No. 2007/0190042 (U.S. Patent No. 2007/0190042).

The cell collection composition can comprise any physiologically-acceptable solution suitable for the collection and/or culture of stem cells, for example, a saline solution (*e.g*., phosphate-buffered saline, Kreb's solution, modified Kreb's solution, Eagle's solution, 0.9% NaCl. *etc.*), a culture medium (*e.g.,* DMEM, H.DMEM, *etc.*), and the like.

The cell collection composition can comprise one or more components that tend to preserve placental cells, that is, prevent the placental cells from dying, or delay the death of the placental cells, reduce the number of placental cells in a population of cells that die, or the like, from the time of collection to the time of culturing. Such components can be, *e.g.,* an apoptosis inhibitor (*e.g.,* a caspase inhibitor or JNK inhibitor); a vasodilator (*e.g.,* magnesium sulfate, an antihypertensive drug, atrial natriuretic peptide (ANP), adrenocorticotropin, corticotropin-releasing hormone, sodium nitroprusside, hydralazine, adenosine triphosphate, adenosine, indomethacin or magnesium sulfate, a phosphodiesterase inhibitor, *etc.*); a necrosis inhibitor (*e.g*., 2-(1H-Indol-3-yl)-3-pentylamino-maleimide, pyrrolidine dithiocarbamate, or clonazepam); a TNF-α inhibitor; and/or an oxygen-carrying perfluorocarbon (*e.g.,* perfluorooctyl bromide, perfluorodecyl bromide, *etc.*).

The cell collection composition can comprise one or more tissue-degrading enzymes, *e.g.,* a metalloprotease, a serine protease, a neutral protease, a hyaluronidase, an RNase, or a DNase, or the like. Such enzymes include, but are not limited to, collagenases (*e.g*., collagenase I, II, III or IV, a collagenase from *Clostridium histolyticum, etc*.); dispase, thermolysin, elastase, trypsin, LIBERASE, hyaluronidase, and the like.

The cell collection composition can comprise a bacteriocidally or bacteriostatically effective amount of an antibiotic. In certain non-limiting embodiments, the antibiotic is a macrolide (*e.g.,* tobramycin), a cephalosporin (*e.g.,* cephalexin, cephradine, cefuroxime, cefprozil, cefaclor, cefixime or cefadroxil), a clarithromycin, an erythromycin, a penicillin (*e.g.,* penicillin V) or a quinolone (*e.g.,* ofloxacin, ciprofloxacin or norfloxacin), a tetracycline, a streptomycin, *etc.* In a particular embodiment, the antibiotic is active against Gram(+) and/or Gram(-) bacteria, *e.g., Pseudomonas aeruginosa, Staphylococcus aureus,* and the like.

The cell collection composition can also comprise one or more of the following compounds: adenosine (about 1 mM to about 50 mM); D-glucose (about 20 mM to about 100 mM); magnesium ions (about 1 mM to about 50 mM); a macromolecule of molecular weight greater than 20,000 daltons, in one embodiment, present in an amount sufficient to maintain endothelial integrity and cellular viability (*e.g.,* a synthetic or naturally occurring colloid, a polysaccharide such as dextran or a polyethylene glycol present at about 25 g/l to about 100 g/l, or about 40 g/l to about 60 g/l); an antioxidant (*e.g*., butylated hydroxyanisole, butylated hydroxytoluene, glutathione, vitamin C or vitamin E present at about 25 µM to about 100 µM); a reducing agent (*e.g*., N-acetylcysteine present at about 0.1 mM to about 5 mM); an agent that prevents calcium entry into cells (*e.g*., verapamil present at about 2 µM to about 25 µM); nitroglycerin (*e.g*., about 0.05 g/L to about 0.2 g/L); an anticoagulant, in one embodiment, present in an amount sufficient to help prevent clotting of residual blood (*e.g.,* heparin or hirudin present at a concentration of about 1000 units/l to about 100,000 units/l); or an amiloride containing compound (*e.g*., amiloride, ethyl isopropyl amiloride, hexamethylene amiloride, dimethyl amiloride or isobutyl amiloride present at about 1.0 µM to about 5 µM).

### 5.3.2. Collection and Handling of Placenta

Generally, a human placenta is recovered shortly after its expulsion after birth. In a preferred embodiment, the placenta is recovered from a patient after informed consent and after a complete medical history of the patient is taken and is associated with the placenta. Preferably, the medical history continues after delivery. Such a medical history can be used to coordinate subsequent use of the placenta or the cells harvested therefrom. For example, human placental cells can be used, in light of the medical history, for personalized medicine for the infant associated with the placenta, or for parents, siblings or other relatives of the infant.

Prior to recovery of perfusate, the umbilical cord blood and placental blood are removed. In certain embodiments, after delivery, the cord blood in the placenta is recovered. The placenta can be subjected to a conventional cord blood recovery process. Typically a needle or cannula is used, with the aid of gravity, to exsanguinate the placenta (*see, e.g.,* Anderson, U.S. Patent No. 5,372,581; Hessel et al., U.S. Patent No. 5,415,665). The needle or cannula is usually placed in the umbilical vein and the placenta can be gently massaged to aid in draining cord blood from the placenta. Such cord blood recovery may be performed commercially, *e.g*., LifeBank Inc., Cedar Knolls, N.J., ViaCord, Cord Blood Registry and CryoCell. Preferably, the placenta is gravity drained without further manipulation so as to minimize tissue disruption during cord blood recovery.

Typically, a placenta is transported from the delivery or birthing room to another location, *e.g.,* a laboratory, for recovery of cord blood and collection of perfusate. The placenta is preferably transported in a sterile, thermally insulated transport device (maintaining the temperature of the placenta between 20-28°C), for example, by placing the placenta, with clamped proximal umbilical cord, in a sterile zip-lock plastic bag, which is then placed in an insulated container. In another embodiment, the placenta is transported in a cord blood collection kit substantially as described in U.S. Patent No. 7,147,626. Preferably, the placenta is delivered to the laboratory four to twenty-four hours following delivery. In certain embodiments, the proximal umbilical cord is clamped, preferably within 4-5 cm (centimeter) of the insertion into the placental disc prior to cord blood recovery. In other embodiments, the proximal umbilical cord is clamped after cord blood recovery but prior to further processing of the placenta.

The placenta, prior to collection of the perfusate, can be stored under sterile conditions and at either room temperature or at a temperature of 5 to 25°C (centigrade). The placenta may be stored for a period of longer than forty eight hours, and preferably for a period of four to twenty-four hours prior to perfusing the placenta to remove any residual cord blood. The placenta is preferably stored in an anticoagulant solution at a temperature of 5°C to 25°C (centigrade). Suitable anticoagulant solutions are well known in the art. For example, a solution of heparin or warfarin sodium can be used. Preferably, the anticoagulant solution comprises a solution of heparin (*e.g.,* 1% w/w in 1:1000 solution). The exsanguinated placenta is preferably stored for no more than 36 hours before placental perfusate is collected.

### 5.3.3. Placental Perfusion

Methods of perfusing mammalian placentae are disclosed, *e.g*., in Hariri, U.S. Patent Nos. 7,045,148 and 7,255,879, and in U.S. Application Publication Nos. 2007/0190042 (U.S. Patent No. 9.598.669) and 20070275362 (U.S Patent No. 8,057,788).

Perfusate can be obtained by passage of perfusion solution, *e.g*., saline solution, culture medium or cell collection compositions described above, through the placental vasculature. In one embodiment, a mammalian placenta is perfused by passage of perfusion solution through either or both of the umbilical artery and umbilical vein. The flow of perfusion solution through the placenta may be accomplished using, *e.g*., gravity flow into the placenta. Preferably, the perfusion solution is forced through the placenta using a pump, *e.g.,* a peristaltic pump. The umbilical vein can be, *e.g.,* cannulated with a cannula, *e.g.,* a TEFLON® or plastic cannula, that is connected to a sterile connection apparatus, such as sterile tubing. The sterile connection apparatus is connected to a perfusion manifold.

In preparation for perfusion, the placenta is preferably oriented in such a manner that the umbilical artery and umbilical vein are located at the highest point of the placenta. The placenta can be perfused by passage of a perfusion solution through the placental vasculature, or through the placental vasculature and surrounding tissue. In one embodiment, the umbilical artery and the umbilical vein are connected simultaneously to a pipette that is connected via a flexible connector to a reservoir of the perfusion solution. The perfusion solution is passed into the umbilical vein and artery. The perfusion solution exudes from and/or passes through the walls of the blood vessels into the surrounding tissues of the placenta, and is collected in a suitable open vessel from the surface of the placenta that was attached to the uterus of the mother during gestation. The perfusion solution may also be introduced through the umbilical cord opening and allowed to flow or percolate out of openings in the wall of the placenta which interfaced with the maternal uterine wall. In another embodiment, the perfusion solution is passed through the umbilical veins and collected from the umbilical artery, or is passed through the umbilical artery and collected from the umbilical veins, that is, is passed through only the placental vasculature (fetal tissue).

In one embodiment, for example, the umbilical artery and the umbilical vein are connected simultaneously, *e.g*., to a pipette that is connected via a flexible connector to a reservoir of the perfusion solution. The perfusion solution is passed into the umbilical vein and artery. The perfusion solution exudes from and/or passes through the walls of the blood vessels into the surrounding tissues of the placenta, and is collected in a suitable open vessel from the surface of the placenta that was attached to the uterus of the mother during gestation. The perfusion solution may also be introduced through the umbilical cord opening and allowed to flow or percolate out of openings in the wall of the placenta which interfaced with the maternal uterine wall. Placental cells that are collected by this method, which can be referred to as a "pan" method, are typically a mixture of fetal and maternal cells.

In another embodiment, the perfusion solution is passed through the umbilical veins and collected from the umbilical artery, or is passed through the umbilical artery and collected from the umbilical veins. Placental cells collected by this method, which can be referred to as a "closed circuit" method, are typically almost exclusively fetal.

The closed circuit perfusion method can, in one embodiment, be performed as follows. A post-partum placenta is obtained within about 48 hours after birth. The umbilical cord is clamped and cut above the clamp. The umbilical cord can be discarded, or can processed to recover, *e.g*., umbilical cord stem cells, and/or to process the umbilical cord membrane for the production of a biomaterial. The amniotic membrane can be retained during perfusion, or can be separated from the chorion, *e.g*., using blunt dissection with the fingers. If the amniotic membrane is separated from the chorion prior to perfusion, it can be, *e.g*., discarded, or processed, *e.g.,* to obtain stem cells by enzymatic digestion, or to produce, *e.g.,* an amniotic membrane biomaterial, *e.g*., the biomaterial described in U.S. Application Publication No. 2004/0048796. After cleaning the placenta of all visible blood clots and residual blood, *e.g.,* using sterile gauze, the umbilical cord vessels are exposed, *e.g.,* by partially cutting the umbilical cord membrane to expose a cross-section of the cord. The vessels are identified, and opened, *e.g.,* by advancing a closed alligator clamp through the cut end of each vessel. The apparatus, *e.g*., plastic tubing connected to a perfusion device or peristaltic pump, is then inserted into each of the placental arteries. The pump can be any pump suitable for the purpose, *e.g.,* a peristaltic pump. Plastic tubing, connected to a sterile collection reservoir, *e.g*., a blood bag such as a 250 mL collection bag, is then inserted into the placental vein. Alternatively, the tubing connected to the pump is inserted into the placental vein, and tubes to a collection reservoir(s) are inserted into one or both of the placental arteries. The placenta is then perfused with a volume of perfusion solution, *e.g*., about 750 ml of perfusion solution. Cells in the perfusate are then collected, *e.g.,* by centrifugation.

In one embodiment, the proximal umbilical cord is clamped during perfusion, and more preferably, is clamped within 4-5 cm (centimeter) of the cord's insertion into the placental disc.

The first collection of perfusion fluid from a mammalian placenta during the exsanguination process is generally colored with residual red blood cells of the cord blood and/or placental blood. The perfusion fluid becomes more colorless as perfusion proceeds and the residual cord blood cells are washed out of the placenta. Generally from 30 to 100 mL of perfusion fluid is adequate to initially flush blood from the placenta, but more or less perfusion fluid may be used depending on the observed results.

The volume of perfusion liquid used to perfuse the placenta may vary depending upon the number of placental cells to be collected, the size of the placenta, the number of collections to be made from a single placenta, *etc.* In various embodiments, the volume of perfusion liquid may be from 50 mL to 5000 mL, 50 mL to 4000 mL, 50 mL to 3000 mL, 100 mL to 2000 mL, 250 mL to 2000 mL, 500 mL to 2000 mL, or 750 mL to 2000 mL. Typically, the placenta is perfused with 700-800 mL of perfusion liquid following exsanguination.

The placenta can be perfused a plurality of times over the course of several hours or several days. Where the placenta is to be perfused a plurality of times, it may be maintained or cultured under aseptic conditions in a container or other suitable vessel, and perfused with a cell collection composition, or a standard perfusion solution (*e.g*., a normal saline solution such as phosphate buffered saline ("PBS") with or without an anticoagulant (*e.g*., heparin, warfarin sodium, coumarin, bishydroxycoumarin), and/or with or without an antimicrobial agent (*e.g.,* β-mercaptoethanol (0.1 mM); antibiotics such as streptomycin *(e.g.,* at 40-100 µg/ml), penicillin *(e.g.,* at 40U/ml), amphotericin B *(e.g.,* at 0.5 µg/ml). In one embodiment, an isolated placenta is maintained or cultured for a period of time without collecting the perfusate, such that the placenta is maintained or cultured for 1, 2, 3, 4, 5, 6, 7, 8, 9, 10, 11, 12, 13, 14, 15, 16, 17, 18, 19, 20, 21, 22, 23, or 24 hours, or 2 or 3 or more days before perfusion and collection of perfusate. The perfused placenta can be maintained for one or more additional time(s), *e.g*., 1, 2, 3, 4, 5, 6, 7, 8, 9, 10, 11, 12, 13, 14, 15, 16, 17, 18, 19, 20, 21, 22, 23, 24 or more hours, and perfused a second time with, *e.g.,* 700-800 mL perfusion fluid. The placenta can be perfused 1, 2, 3, 4, 5 or more times, for example, once every 1, 2, 3, 4, 5 or 6 hours. In a preferred embodiment, perfusion of the placenta and collection of perfusion solution, *e.g*., placental cell collection composition, is repeated until the number of recovered nucleated cells falls below 100 cells/ml. The perfusates at different time points can be further processed individually to recover time-dependent populations of cells, *e.g.,* total nucleated cells. Perfusates from different time points can also be pooled.

### 5.3.4. Placental Perfusate and Placental Perfusate Cells

In certain embodiments, perfusate or perfusate cells are cryopreserved. In certain other embodiments, the placental perfusate comprises, or the perfusate cells comprise, only fetal cells, or a combination of fetal cells and maternal cells.

Typically, placental perfusate from a single placental perfusion comprises about 100 million to about 500 million nucleated cells. In certain embodiments, the placental perfusate or perfusate cells comprise CD34⁺ cells, *e.g*., hematopoietic stem or progenitor cells. Such cells can, in a more specific embodiment, comprise CD34⁺CD45⁻ stem or progenitor cells, CD34⁺CD45⁺ stem or progenitor cells, myeloid progenitors, lymphoid progenitors, and/or erythroid progenitors. In other embodiments, placental perfusate and placental perfusate cells comprise adherent placental stem cells, *e.g.,* CD34⁻ stem cells. In other embodiments, the placental perfusate and placental perfusate cells comprise, *e.g*., endothelial progenitor cells, osteoprogenitor cells, and natural killer cells.

### 5.4. Disruption and Digestion of Placental Tissue

Placental natural killer cells, *e.g.,* PINK cells, can be, for example, obtained from placental tissue that has been mechanically and/or enzymatically disrupted.

Placental tissue can be disrupted using one or more tissue-degrading enzymes, *e.g.,* a metalloprotease, a serine protease, a neutral protease, an RNase, or a DNase, or the like. Such enzymes include, but are not limited to, collagenases (*e.g*., collagenase I, II, III or IV, a collagenase from *Clostridium histolyticum,* etc.); dispase, thermolysin, elastase, trypsin, LIBERASE, hyaluronidase, and the like. Typically after digestion, the digested tissue is passed through a strainer or filter to remove partially-digested cell clumps, leaving a substantially single-celled suspension.

### 5.5. Placental Natural Killer Cells

In one aspect, provided herein is the isolation, characterization, and use of natural killer cells obtainable from placenta, *e.g*., from placental perfusate and/or from mechanically and/or enzymatically-disrupted placental tissue, and of compositions comprising such natural killer cells. In a specific embodiment, the placental natural killer cells are "placental intermediate natural killer cells," or "PINK" cells, are characterized as being CD56⁺CD16⁻CD3-, *i.e.,* displaying the CD56 cellular marker and lacking the CD16 and CD3 cellular markers, *e.g*., as determined by flow cytometry, *e.g.*, fluorescence-activated cell sorting using antibodies against CD16, CD3 and CD56, as described above. As such, provided herein are isolated PINK cells and isolated pluralities of PINK cells. Also provided herein are isolated pluralities of cells comprising CD56⁺CD16⁻CD3- PINK cells in combination with CD56⁺CD16⁺ natural killer cells. In more specific embodiments, the CD56⁺CD16⁺ natural killer cells can be isolated from placenta, or from another source, *e.g*., peripheral blood, umbilical cord blood, bone marrow, or the like. Thus, in various other embodiments, said PINK cells can be combined with CD56⁺CD16⁺ natural killer cells, *e.g.,* in ratios of, for example, about 1:10, 2:9, 3:8, 4:7:, 5:6, 6:5, 7:4, 8:3, 9:2, 1:10, 1:9, 1:8, 1:7, 1:6, 1:5, 1:4, 1:3, 1:2, 1:1, 2:1, 3:1, 4:1, 5:1, 6:1, 7:1, 8:1 or about 9:1. As used in this context, "isolated" means that the cells have been removed from their normal environment, *e.g*., the placenta.

In certain aspects, the PINK cells are CD3⁻. In certain specific aspects, the PINK cells are CD3⁻ and CD56⁺. In certain aspects, populations of natural killer cells, *e.g*., populations of PINK cells, comprise at least 50%, 55%, 60%, 65%, 70%, 75%, 80%, 85%, 90%, 95%, 98% or 99% CD3⁻, CD56⁺ natural killer cells, *e.g,* PINK cells. In specific embodiments, the natural killer cells are additionally CD16⁻ i.e.,, the PINK cells are CD56+, CD3-, CD16-.

In other embodiments, the PINK cells do not exhibit one or more cellular markers exhibited by fully mature natural killer cells *(e.g.,* CD16), or exhibit such one or more markers at a detectably reduced level compared to fully mature natural killer cells, or exhibit one or more cellular markers associated with natural killer cell precursors but not fully mature natural killer cells. In a specific embodiment, a PINK cell provided herein expresses NKG2D, CD94 and/or NKp46, as determined, *e.g*., by flow cytometry, at a detectably lower level than a fully mature NK cell. In another specific embodiment, a plurality of PINK cells provided herein expresses, in total, NKG2D, CD94 and/or NKp46 at a detectably lower level than an equivalent number of fully mature NK cells.

In certain embodiments, PINK cells express one or more of the microRNAs hsa-miR-100, hsa-miR-127, hsa-miR-211, hsa-miR-302c, hsa-miR-326, hsa-miR-337, hsa-miR-497, hsa-miR-512-3p, hsa-miR-515-5p, hsa-miR-517b, hsa-miR-517c, hsa-miR-518a, hsa-miR-518e, hsa-miR-519d, hsa-miR-520g, hsa-miR-520h, hsa-miR-564, hsa-miR-566, hsa-miR-618, and/or hsa-miR-99a at a detectably higher level than peripheral blood natural killer cells, as determined by, *e.g.,* qRT-PCR. In another embodiment, the natural killer cells do not express the microRNA hsa-miR-199b, or express the microRNA hsa-miR-199b at a detectably lower level than peripheral blood natural killer cells, as determined by, *e.g.,* qRT-PCR.

In certain embodiments, the placental natural killer cells, *e.g.,* PINK cells, have been expanded in culture. In certain other embodiments, the placental perfusate cells have been expanded in culture. In a specific embodiment, said placental perfusate cells have been expanded in the presence of a feeder layer and/or in the presence of at least one cytokine. In a more specific embodiment, said feeder layer comprises K562 cells or peripheral blood mononuclear cells. In another more specific embodiment, said at least one cytokine is interleukin-2.

In another embodiment, provided herein is an isolated plurality (*e.g*., population) of PINK cells. In another specific embodiment, the isolated population of cells is produced by CD56-microbead isolation of cells from placental perfusate. In various specific embodiments, the population comprises at least about 50%, 55%, 60%, 65%, 70%, 75%, 80%, 85%, 90%, 95%, 98% or at least about 99% PINK cells. In another embodiment, the plurality of PINK cells comprises, or consists of, PINK cells that have not been expanded; *e.g*., are as collected from placental perfusate. In another embodiment, the plurality of PINK cells comprise, or consist of, PINK cells that have been expanded. Methods of expanding natural killer cells have been described, *e.g.,* in Ohno et al., U.S. Patent Application Publication No. 2003/0157713; *see also* Yssel et al., J. Immunol. Methods 72(1):219-227 (1984) and Litwin et al., J. Exp. Med. 178(4):1321-1326 (1993) and the description of natural killer cell expansion in Example 1, below.

In other embodiments, the isolated plurality of PINK cells does not exhibit one or more cellular markers exhibited by fully mature natural killer cells *(e.g.,* CD16), or exhibits such one or more markers at a detectably reduced level compared to fully mature natural killer cells, or exhibits one or more cellular markers associated with natural killer cell precursors but not associated with fully mature natural killer cells. In a specific embodiment, a PINK cell provided herein expresses NKG2D, CD94 and/or NKp46, as detected, *e.g*., by flow cytometry, at a detectably lower level than a fully mature NK cell. In another specific embodiment, a plurality of PINK cells provided herein expresses, in total, NKG2D, CD94 and/or NKp46 at a detectably lower level than an equivalent number of fully mature NK cells.

In certain specific embodiments, the population of PINK cells expresses one or more of the microRNAs hsa-miR-100, hsa-miR-127, hsa-miR-211, hsa-miR-302c, hsa-miR-326, hsa-miR-337, hsa-miR-497, hsa-miR-512-3p, hsa-miR-515-5p, hsa-miR-517b, hsa-miR-517c, hsa-miR-518a, hsa-miR-518e, hsa-miR-519d, hsa-miR-520g, hsa-miR-520h, hsa-miR-564, hsa-miR-566, hsa-miR-618, and/or hsa-miR-99a, at a detectably higher level than peripheral blood natural killer cells, as determined, *e.g*., by quantitative real-time PCR (qRT-PCR). In another specific embodiment, the population of PINK cells expresses a detectably higher amount of granzyme B than an equivalent number of peripheral blood natural killer cells.

In other embodiments, the PINK cells provided herein have been expanded in culture. In specific embodiments, the PINK cells have been cultured, *e.g*., expanded in culture, for at least, about, or at most 1, 2, 3, 4, 5, 6, 7, 8, 9, 10, 11, 12, 13, 14, 15, 16, 17, 18, 19, 20, 21, 22, 23, 24, 25, 26, 27 or 28 days. In a specific embodiment, the PINK cells are cultured for about 21 days.

In another aspect, provided herein is an isolated population of cells, *e.g.,* placental cells, comprising PINK cells. In a specific aspect, the isolated population of cells is total nucleated cells from placental perfusate, *e.g*., placental perfusate cells, comprising autologous, isolated PINK cells. In another specific aspect, the population of cells is an isolated population of cells produced by CD56-microbead isolation of cells from placental perfusate. In various specific aspects, the population comprises at least about 50%, 55%, 60%, 65%, 70%, 75%, 80%, 85%, 90%, 95%, 98% or at least about 99% PINK cells.

Because the post-partum placenta comprises tissue and cells from the fetus and from the mother placental perfusate, depending upon the method of collection, can comprise fetal cells only, or a substantial majority of fetal cells (*e.g.,* greater than about 90%, 95%, 98% or 99%), or can comprise a mixture of fetal and maternal cells (*e.g.,* the fetal cells comprise less than about 90%, 80%, 70%, 60%, or 50% of the total nucleated cells of the perfusate). In one embodiment, the PINK cells are derived only from fetal placental cells, *e.g.,* cells obtained from closed-circuit perfusion of the placenta (*see* above) wherein the perfusion produces perfusate comprising a substantial majority, or only, fetal placental cells. In another embodiment, the PINK cells are derived from fetal and maternal cells, *e.g.,* cells obtained by perfusion by the pan method (*see* above), wherein the perfusion produced perfusate comprising a mix of fetal and maternal placental cells. Thus, in one embodiment, provided herein is a population of placenta-derived intermediate natural killer cells, the substantial majority of which have the fetal genotype. In another embodiment, provided herein is a population of placenta-derived intermediate natural killer cells that comprise natural killer cells having the fetal genotype and natural killer cells having the maternal phenotype.

Also disclosed herein are populations of placenta-derived intermediate natural killer cells that comprise natural killer cells from a non-placental source. For example, in one embodiment, disclosed herein is population of PINK cells that also comprises natural killer cells from umbilical cord blood, peripheral blood, bone marrow, or a combination of two or more of the foregoing. The populations of natural killer cells comprising PINK cells and natural killer cells from a non-placental source can comprise the cells in, *e.g.,* a ratio of about 1:10, 2:9, 3:8, 4:7:, 5:6, 6:5, 7:4, 8:3, 9:2, 10:1, 1:9, 1:8, 1:7, 1:6, 1:5, 1:4, 1:3, 1:2, 1:1, 2:1, 3:1, 4:1, 5:1, 6:1, 7:1, 8:1, 9:1, 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, or about 1:100, or the like.

Further aspects disclosed herein are combinations of umbilical cord blood and isolated PINK cells. In various aspects, cord blood is combined with PINK cells at about 1 x 10⁴ , 5 x 10⁴, 1 x 10⁵, 5 x 10⁵, 1 x 10⁶, 5 x 10⁶, 1 x 10⁷, 5 x 10⁷, 1 x 10⁸, or 5 x 10⁸, or more, PINK cells per milliliter of cord blood.

Also provided herein are methods of isolating PINK cells. In one embodiment, PINK cells are collected by obtaining placental perfusate, then contacting the placental perfusate with a composition that specifically binds to CD56⁺ cells, *e.g.,* an antibody against CD56, followed by isolating of CD56⁺ cells on the basis of said binding to form a population of CD56⁺ cells. The population of CD56⁺ cells comprises an isolated population of natural killer cells. In a specific embodiment, CD56⁺ cells are contacted with a composition that specifically binds to CD16⁺ cells, *e.g.,* an antibody against CD16, and the CD16⁺ cells from the population of CD56⁺ cells. In another specific embodiment, CD3⁺ cells are also excluded from the population of CD56⁺ cells.

In one embodiment, PINK cells can be obtained from placental perfusate as follows. A post-partum human placenta is exsanguinated and perfused, *e.g*., with about 200-800 mL of perfusion solution, through the placental vasculature only. In a specific embodiment, the placenta is drained of cord blood and flushed, *e.g*., with perfusion solution, through the placental vasculature to remove residual blood prior to said perfusing. The perfusate is collected and processed to remove any residual erythrocytes. Natural killer cells in the total nucleated cells in the perfusate can be isolated on the basis of expression of CD56 and CD16. In certain embodiments, the isolation of PINK cells comprises isolation using an antibody to CD56, wherein the isolated cells are CD56⁺. In another embodiment, the isolation of PINK cells comprises isolation using an antibody to CD16, wherein the isolated cells are CD16⁻. In another embodiment, the isolation of PINK cells comprises isolation using an antibody to CD56, and exclusion of a plurality of non-PINK cells using an antibody to CD16, wherein the isolated cells comprise CD56⁺, CD16⁻ cells.

After a suspension of placental cells is obtained, *e.g*., from placental perfusate or from enzymatically digested placental tissue natural killer cells can be isolated or enriched using, *e.g*., antibodies to CD3 and CD56. In a specific embodiment, placental natural killer cells are isolated by selecting for cells that are CD56⁺ to produce a first cell population; contacting said first cell population with antibodies specific for CD3 and/or CD16; and removing cells from said first cell population that are CD3⁺ or CD16⁺, thereby producing a second population of cells that is substantially CD56⁺ and CD3⁻, CD56⁺ and CD16⁻, or CD56⁺, CD3⁻ and CD16⁻.

In another aspect, provided herein is a method of isolating placental natural killer cells, comprising CD56+, CD16-, CD3- PINK cells, comprising obtaining a plurality of placental cells, and isolating natural killer cells from said plurality of placental cells. In a specific embodiment, the placental cells are, or comprise, placental perfusate cells, *e.g.,* total nucleated cells from placental perfusate. In another specific embodiment, said plurality of placental cells are, or comprise, placental cells obtained by mechanical and/or enzymatic digestion of placental tissue. In another embodiment, said isolating is performed using one or more antibodies. In a more specific embodiment, said one or more antibodies comprises one or more of antibodies to CD3, CD16 or CD56. In a more specific embodiment, said isolating comprises isolating CD56⁺ cells from CD56⁻ cells in said plurality of placental cells. In a more specific embodiment, said isolating comprises isolating CD56⁺, CD16⁻ placental cells, *e.g.,* placental natural killer cells, *e.g.,* PINK cells, from placental cells that are CD56⁻ or CD16⁺. In a more specific embodiment, said isolating comprises isolating CD56⁺, CD16⁻, CD3⁻ placental cells from placental cells that are CD56⁻, CD16⁺, or CD3⁺. In another aspect, NK cells from placenta, *e.g.*, PINK cells, are isolated or enriched by contacting a plurality of cells comprising the NK cells with an antibody to CD5 and an antibody to CD56, and isolating cells that are CD56⁺ and CD5⁻, wherein said CD56⁺ and CD5⁻ are enriched for placental natural killer cells, *e.g.,* PINK cells. In another, said method of isolating placental natural killer cells results in a population of placental cells that is at least 50%, 55%, 60%, 65%, 70%, 75%, 80%, 85%, 90%, 95%, 98% or at least 99% CD56⁺, CD16⁻ natural killer cells.

Cell separation can be accomplished by any method known in the art, *e.g.,* flow cytometry, fluorescence-activated cell sorting (FACS), or magnetic cell sorting using microbeads conjugated with specific antibodies. The cells may be isolated, *e.g*., using a magnetic activated cell sorting (MACS) technique, a method for separating particles based on their ability to bind magnetic beads (*e.g*., about 0.5-100 µm diameter) that comprise one or more specific antibodies, *e.g*., anti-CD56 antibodies. Magnetic cell separation can be performed and automated using, *e.g,* an AUTOMACS™ Separator (Miltenyi). A variety of useful modifications can be performed on the magnetic microspheres, including covalent addition of antibody that specifically recognizes a particular cell surface molecule or hapten. The beads are then mixed with the cells to allow binding. Cells are then passed through a magnetic field to separate out cells having the specific cell surface marker. In one embodiment, these cells can then be isolated and re-mixed with magnetic beads coupled to an antibody against additional cell surface markers. In this embodiment, the cells are again passed through a magnetic field, isolating cells that bind both the antibodies. Such cells can then be diluted into separate dishes, such as microtiter dishes for clonal isolation.

Optionally, the isolated or enriched natural killer cells can be confirmed in a cytotoxicity assay using tumor cells, *e.g*., cultured K562 tumor cells, or the like as target cells.

### 5.6. Placental Natural Killer Cells from Matched Perfusate and Cord Blood

Further provided herein are natural killer cells obtained, and obtainable from, combinations of matched units of placental perfusate and umbilical cord blood, referred to herein as combined natural killer cells. "Matched units," as used herein, indicates that the NK cells are obtained from placental perfusate cells, and umbilical cord blood cells, wherein the umbilical cord blood cells are obtained from umbilical cord blood from the placenta from which the placental perfusate is obtained, *i.e.,* the placental perfusate cells and umbilical cord blood cells, and thus the natural killer cells from each, are from the same individual.

In certain aspects, the combined placental killer cells comprise only, or substantially only, natural killer cells that are CD56⁺ and CD16⁻. In certain other aspects, the combined placental killer cells comprise NK cells that are CD56⁺ and CD16⁻, and NK cells that are CD56⁺ and CD16⁺. In certain specific embodiments, the combined placental killer cells comprise at least 50%, 60%, 70%, 80%, 90%, 95%, 96%, 97%, 98%, 99% or 99.5% CD56⁺CD16⁻CD3- natural killer cells (PINK cells).

In one embodiment, the combined natural killer cells have not been cultured. In a specific embodiment, the combined natural killer cells comprise a detectably higher number of CD3⁻CD56⁺CD16⁻ natural killer cells than an equivalent number of natural killer cells from peripheral blood. In another specific embodiment, the combined natural killer cells comprise a detectably lower number of CD3⁻CD56⁺CD16⁺ natural killer cells than an equivalent number of natural killer cells from peripheral blood. In another specific embodiment, the combined natural killer cells comprise a detectably higher number of CD3⁻ CD56⁺KIR2DL2/L3⁺ natural killer cells than an equivalent number of natural killer cells from peripheral blood. In another specific embodiment, the combined natural killer cells comprise a detectably lower number of CD3⁻CD56⁺NKp46⁺ natural killer cells than an equivalent number of natural killer cells from peripheral blood. In another specific embodiment, the combined natural killer cells comprise a detectably lower number of CD3⁻ CD56⁺ NKp30⁺ natural killer cells than an equivalent number of natural killer cells from peripheral blood. In another specific embodiment, the combined natural killer cells comprise a detectably lower number of CD3⁻CD56⁺2B4⁺ natural killer cells than an equivalent number of natural killer cells from peripheral blood. In another specific embodiment, the combined natural killer cells comprise a detectably lower number of CD3⁻CD56⁺CD94⁺ natural killer cells than an equivalent number of natural killer cells from peripheral blood.

In another embodiment, the combined natural killer cells have been cultured, *e.g.*, for 21 days. In a specific embodiment, the combined natural killer cells comprise a detectably lower number of CD3⁻CD56⁺KIR2DL2/L3⁺ natural killer cells than an equivalent number of natural killer cells from peripheral blood. In another specific embodiment, the combined natural killer cells have not been cultured. In another specific embodiment, the combined natural killer cells comprise a detectably higher number of CD3⁻CD56⁺NKp44⁺ natural killer cells than an equivalent number of natural killer cells from peripheral blood. In a specific embodiment, the combined natural killer cells comprise a detectably higher number of CD3⁻ CD56⁺NKp30⁺ natural killer cells than an equivalent number of natural killer cells from peripheral blood.

In another embodiment, the combined natural killer cells express a detectably higher amount of granzyme B than an equivalent number of peripheral blood natural killer cells.

Further provided herein are combinations of umbilical cord blood and combined natural killer cells. In various embodiments, cord blood is combined with combined natural killer cells at about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ combined natural killer cells per milliliter of cord blood.

### 5.7. Perfusate/Cell Combinations

In addition to placental perfusate, placental perfusate cells, combined natural killer cells, and placental natural killer cells, *e.g.,* placental intermediate natural killer cells, provided herein are compositions comprising the perfusate or cells, for use in suppressing the proliferation of a tumor cell or plurality of tumor cells.

### 5.7.1. Combinations of Placental Perfusate, Perfusate Cells And Placenta-Derived Intermediate Natural Killer Cells

Further provided herein are compositions comprising combinations of the placental perfusate, placental perfusate cells, natural killer cells comprising CD56+, CD16-, CD3- placental intermediate natural killer cells, and/or combined natural killer cells described in the Sections above. In one aspect of the disclosure, for example, disclosed herein is a volume of placental perfusate supplemented with placental perfusate cells and/or natural killer cells, *e.g.,* placental intermediate natural killer cells, for example, obtained from placental perfusate cells or placental tissue that has been mechanically or enzymatically disrupted. In specific aspects of the disclosure, for example, each milliliter of placental perfusate is supplemented with about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more placental perfusate cells, natural killer cells, *e.g.,* placental intermediate natural killer cells, and/or combined natural killer cells. In another aspect of the disclosure, placental perfusate cells are supplemented with placental perfusate, placental intermediate natural killer cells, and/or combined natural killer cells. In another aspect of the disclosure, natural killer cells, *e.g*., placental intermediate natural killer cells, are supplemented with placental perfusate, placental perfusate cells, and/or combined natural killer cells.

In certain aspects of the disclosure, when perfusate is used for supplementation, the volume of perfusate is about, greater than about, or less than about, 50%, 45%, 40%, 35%, 30%, 25%, 20%, 15%, 10%, 8%, 6%, 4%, 2% or 1% of the total volume of cells (in solution) plus perfusate. In certain other aspects of the disclosure, when placental perfusate cells are combined with a plurality of natural killer cells, *e.g.,* PINK cells and/or combined natural killer cells, the placental perfusate cells generally comprise about, greater than about, or fewer than about, 50%, 45%, 40%, 35%, 30%, 25%, 20%, 15%, 10%, 8%, 6%, 4%, 2% or 1% of the total number of cells. In certain other aspects of the disclosure, when natural killer cells, *e.g.,* PINK cells are combined with a plurality of placental perfusate cells and/or combined natural killer cells, the NK cells generally comprise about, greater than about, or fewer than about, 50%, 45%, 40%, 35%, 30%, 25%, 20%, 15%, 10%, 8%, 6%, 4%, 2% or 1% of the total number of cells. In certain other embodiments, when combined natural killer cells are combined with natural killer cells comprising CD56+, CD16-, CD3- PINK cells, the combined natural killer cells generally comprise about, greater than about, or fewer than about, 50%, 45%, 40%, 35%, 30%, 25%, 20%, 15%, 10%, 8%, 6%, 4%, 2% or 1% of the total number of cells. In certain other aspects of the disclosure, when natural killer cells, *e.g.,* PINK cells, combined natural killer cells or placental perfusate cells are used to supplement placental perfusate, the volume of solution *(e.g.,* saline solution, culture medium or the like) in which the cells are suspended comprises about, greater than about, or less than about, 50%, 45%, 40%, 35%, 30%, 25%, 20%, 15%, 10%, 8%, 6%, 4%, 2% or 1% of the total volume of perfusate plus cells, where the NK cells are suspended to about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more cells per milliliter prior to supplementation.

In other embodiments, any of the above combinations of cells is, in turn, combined with umbilical cord blood or nucleated cells from umbilical cord blood.

Further disclosed herein is pooled placental perfusate that is obtained from two or more sources, *e.g.,* two or more placentas, and combined, *e.g.,* pooled. Such pooled perfusate can comprise approximately equal volumes of perfusate from each source, or can comprise different volumes from each source. The relative volumes from each source can be randomly selected, or can be based upon, *e.g.,* a concentration or amount of one or more cellular factors, *e.g*., cytokines, growth factors, hormones, or the like; the number of placental cells in perfusate from each source; or other characteristics of the perfusate from each source. Perfusate from multiple perfusions of the same placenta can similarly be pooled.

Similarly, provided herein are CD56+, CD16-, CD3- placenta-derived intermediate natural killer cells, that are obtained from two or more sources, *e.g*., two or more placentas, and pooled. Such pooled cells can comprise approximately equal numbers of cells from the two or more sources, or different numbers of cells from one or more of the pooled sources. The relative numbers of cells from each source can be selected based on, *e.g.,* the number of one or more specific cell types in the cells to be pooled, *e.g.,* the number of CD34⁺ cells, the number of CD56⁺ cells, *etc.*

Pools can comprise, *e.g*., placental perfusate supplemented with placental perfusate cells; placental perfusate supplemented with placenta-derived intermediate natural killer (PINK) cells; placental perfusate supplemented with both placental perfusate cells and PINK cells; placental perfusate cells supplemented with placental perfusate; placental perfusate cells supplemented with PINK cells; placental perfusate cells supplemented with both placental perfusate and PINK cells; PINK cells supplemented with placental perfusate; PINK cells supplemented with placental perfusate cells; or PINK cells supplemented with both placental perfusate cells and placental perfusate.

Further provided herein are placental intermediate natural killer cells, and pools of the same or combinations of the same, that have been assayed to determine the degree or amount of tumor suppression (that is, the potency) to be expected from, *e.g.,* a given number of PINK cells. For example, an aliquot or sample number of cells is contacted with a known number of tumor cells under conditions in which the tumor cells would otherwise proliferate, and the rate of proliferation of the tumor cells in the presence of placental perfusate, perfusate cells, placental natural killer cells, or combinations thereof, over time *(e.g.,* 1, 2, 3, 4, 5, 6, 7, 8, 9, or 10 weeks, or longer) is compared to the proliferation of an equivalent number of the tumor cells in the absence of perfusate, perfusate cells, placental natural killer cells, or combinations thereof. The potency of the placental perfusate, placental perfusate cells and/or PINK cells, or combinations or pools of the same, can be expressed, *e.g*., as the number of cells or volume of solution required to suppress tumor cell growth, *e.g*., by about 10%, 15%, 20%, 25%, 30%, 35%, 40%, 45%, 50%, or the like.

In certain embodiments, natural killer cells comprising CD56+ CD16- CD3- PINK cells are provided as pharmaceutical grade administrable units. Such units can be provided in discrete volumes, *e.g*., 100 mL, 150 mL, 200 mL, 250 mL, 300 mL, 350 mL, 400 mL, 450 mL, 500 mL, or the like. Such units can be provided so as to contain a specified number of, *e.g.,* placental perfusate cells, placental intermediate natural killer cells, or both, *e.g.,* 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more cells per unit. Such units can be provided to contain specified numbers of any two, or all three, of placental perfusate, placental perfusate cells, and/or PINK cells.

In the above combinations of placental perfusate, placental perfusate cells and/or PINK cells, any one, any two, or all three of the placental perfusate, placental perfusate cells and/or PINK cells can be autologous to a recipient (that is, obtained from the recipient), or homologous to a recipient (that is, obtained from at last one other individual from said recipient).

Any of the above combinations or pools of PINK cells, placental perfusate cells and/or placental perfusate can comprise CD56⁺CD16⁺ natural killer cells from, *e.g*., placental perfusate, peripheral blood, umbilical cord blood, bone marrow, or the like. In specific embodiments, the combinations comprise about, at least about, or at most about 1 x 10⁴, 5 x 10⁴, 1 x 10⁵, 5 x 10⁵, 1 x 10⁶, 5 x 10⁶, 5 x 10⁷, 1 x 10⁸, 5 x 10⁸ or more such natural killer cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more cells per unit. The CD56⁺CD16⁺ natural killer cells can be used as isolated from a natural source, or can be expanded prior to inclusion in one of the above combinations or pools. The CD56⁺CD16⁺ NK cells can be autologous (that is, obtained from the same individual as the placental perfusate, placental perfusate cells and/or PINK cells; or obtained from a recipient) or homologous (that is, derived from an individual different from the placental perfusate, placental perfusate cells and/or PINK cells; or from an individual that is not a recipient).

Preferably, each unit is labeled to specify volume, number of cells, type of cells, whether the unit has been enriched for a particular type of cell, potency of a given number of cells in the unit, or a given number of milliliters of the unit, and/or whether the cells cause a measurable suppression of proliferation of a particular type or types of tumor cell.

Also provided herein are compositions comprising natural killer cells comprising CD56+ CD16- CD3- placental intermediate natural killer (PINK) cells, alone or in combination with placental perfusate cells and/or placental perfusate. Thus, in another aspect, provided herein is a composition comprising natural killer cells comprising isolated CD56⁺, CD16⁻ CD3-PINK cells, wherein said natural killer cells are isolated from placental perfusate, and wherein said natural killer cells comprise at least 50% of cells in the composition. In a specific embodiment, said natural killer cells comprise at least 80% of cells in the composition. In another specific embodiment, said composition comprises isolated CD56⁺, CD16⁺ natural killer cells. In a more specific embodiment, said CD56⁺, CD16⁺ natural killer cells are from a different individual than said CD56⁺, CD16⁻ natural killer cells. In another specific embodiment, said natural killer cells are from a single individual. In a more specific embodiment, said isolated natural killer cells comprise natural killer cells from at least two different individuals. In another specific embodiment, the composition comprises isolated placental perfusate. In a more specific embodiment, said placental perfusate is from the same individual as said natural killer cells. In another more specific embodiment, said placental perfusate comprises placental perfusate from a different individual than said natural killer cells. In another specific embodiment, the composition comprises placental perfusate cells. In a more specific embodiment, said placental perfusate cells are from the same individual as said natural killer cells. In another more specific-embodiment, said placental perfusate cells are from a different individual than said natural killer cells. In another specific embodiment, the composition additionally comprises isolated placental perfusate and isolated placental perfusate cells, wherein said isolated perfusate and said isolated placental perfusate cells are from different individuals. In another more specific embodiment of any of the above embodiments comprising placental perfusate, said placental perfusate comprises placental perfusate from at least two individuals. In another more specific embodiment of any of the above embodiments comprising placental perfusate cells, said isolated placental perfusate cells are from at least two individuals.

### 5.7.2. Compositions Comprising Adherent Placental Stem Cells

In other embodiments, the natural killer cells comprising CD56+ CD16- CD3-PINK cells, or a combination or pool of natural killer cells, is supplemented with isolated adherent placental cells, *e.g.,* placental stem cells as described, *e.g,* in U.S. Patent Nos. 7,045,148 and 7,255,879, and in U.S. Patent Application Publication Nos. 2007/0275362 (U.S. Patent No. 8,057,788) and 2008/0032401. "Adherent placental cells" means that the cells are adherent to tissue culture plastic. The term "placental cell," as used herein, does not include natural killer cells unless specifically stated otherwise. The adherent placental cells useful in the compositions and methods disclosed herein are not trophoblasts, embryonic germ cells or embryonic stem cells.

The placental perfusate, plurality of placental perfusate cells, and/or plurality of natural killer cells, *e.g.,* PINK cells, or a combination or pool of any of the foregoing can be supplemented with, *e.g*., 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more adherent placental cells. The adherent placental cells in the combinations can be, *e.g.*, adherent placental cells that have been cultured for, *e.g.,* 1, 2, 3, 4, 5, 6, 7, 8, 9, 10, 12, 14, 16, 18, 20, 22, 24, 26, 28, 30, 32, 34, 36, 38, or 40 population doublings, or more.

Isolated adherent placental cells, when cultured in primary cultures or in cell culture, adhere to the tissue culture substrate, *e.g.,* tissue culture container surface *(e.g.,* tissue culture plastic). Adherent placental cells in culture assume a generally fibroblastoid, stellate appearance, with a number of cytoplasmic processes extending from the central cell body. Adherent placental cells are, however, morphologically distinguishable from fibroblasts cultured under the same conditions, as the adherent placental cells exhibit a greater number of such processes than do fibroblasts. Morphologically, adherent placental cells are also distinguishable from hematopoietic stem cells, which generally assume a more rounded, or cobblestone, morphology in culture.

The isolated adherent placental cells, and populations of adherent placental cells, useful in the present invention, express a plurality of markers that can be used to identify and/or isolate the cells, or populations of cells that comprise the adherent placental cells. The adherent placental cells, and adherent placental cell populations useful in the present invention include adherent placental cells and adherent placental cell-containing cell populations obtained directly from the placenta, or any part thereof (*e.g*., amnion, chorion, amnion-chorion plate, placental cotyledons, umbilical cord, and the like). The adherent placental stem cell population, in one embodiment, is a population (that is, two or more) of adherent placental stem cells in culture, *e.g.,* a population in a container, *e.g.,* a bag.

In certain embodiments, the isolated adherent placental cells are isolated placental stem cells. In certain other embodiments, the isolated placental cells are isolated placental multipotent cells. In one embodiment, the isolated placental cells are CD34⁻, CD10⁺ and CD105⁺ as detected by flow cytometry. In another specific embodiment, the isolated CD34⁻, CD10⁺, CD105⁺ placental cells have the potential to differentiate into cells having a characteristic of a neural cell, cells having a characteristic of an osteogenic cell, and/or cells having a characteristic of a chondrogenic cell, *e.g.,* either *in vitro* or *in vivo,* or both. In another specific embodiment, the isolated CD34⁻, CD10⁺, CD105⁺ placental cells are additionally CD200⁺. In another specific embodiment, the isolated CD34⁻, CD10⁺, CD105⁺ placental cells are additionally CD45⁻ or CD90⁺. In another specific embodiment, the isolated CD34⁻, CD10⁺, CD105⁺ placental cells are additionally CD45⁻ and CD90⁺, as detected by flow cytometry. In another specific embodiment, the isolated CD34⁻, CD10⁺, CD105⁺, CD200⁺ placental cells are additionally CD90⁺ or CD45⁻, as detected by flow cytometry. In another specific embodiment, the isolated CD34⁻, CD10⁺, CD105⁺, CD200⁺ placental cells are additionally CD90⁺ and CD45⁻, as detected by flow cytometry, *i.e*., the cells are CD34⁻, CD10⁺, CD45⁻, CD90⁺, CD105⁺ and CD200⁺. In another specific embodiment, said CD34⁻, CD10⁺, CD45⁻, CD90⁺, CD105⁺, CD200⁺ cells are additionally CD44⁺, CD80⁻ and/or CD86⁻. In another specific embodiment, said CD34⁻, CD10⁺, CD44⁺, CD45⁻, CD90⁺, CD105⁺, CD200⁺ cells are additionally one or more of CD80⁻, CD86⁻, CD117⁻, CD133⁻, cytokeratin⁺, KDR⁺, HLA-A,B,C⁺, HLA-DR,DP,DQ⁻, and HLA-G⁻. In another specific embodiment, the CD34⁻, CD10⁺, CD105⁺ cells are additionally one or more of SSEA1⁻, SSEA3⁻ and/or SSEA4⁻. In another specific embodiment, the CD34⁻, CD10⁺, CD105⁺ cells are additionally SSEA1⁻, SSEA3⁻ and SSEA4⁻.

In certain embodiments, said placental cells are CD34⁻, CD10⁺, CD105⁺ and CD200⁺, and one or more of CD38⁻, CD45⁻, CD80⁻, CD86⁻, CD133⁻, HLA-DR,DP,DQ⁻, SSEA3⁻, SSEA4⁻, CD29⁺, CD44⁺, CD73⁺, CD90⁺, CD105⁺, HLA-A,B,C⁺, PDL1⁺, ABC-p⁺, and/or OCT-4⁺, as detected by flow cytometry. In other embodiments, any of the CD34⁻, CD10⁺, CD105⁺ cells described above are additionally one or more of CD29⁺, CD38⁻, CD44⁺, CD54⁺, SH3⁺ or SH4⁺. In another specific embodiment, the cells are additionally CD44⁺. In another specific embodiment of any of the isolated CD34⁻, CD10⁺, CD105⁺ placental cells above, the cells are additionally one or more of CD117⁻, CD133⁻, KDR⁻(VEGFR2⁻), HLA-A,B,C⁺, HLA-DP,DQ,DR⁻, or Programmed Death-1 Ligand (PDL1)⁺, or any combination thereof.

In another embodiment, the CD34⁻, CD10⁺, CD105⁺ placental cells are additionally one or more of CD13⁺, CD29⁺, CD33⁺, CD38⁻, CD44⁺, CD45⁻, CD54⁺, CD62E⁻, CD62L⁻, CD62P⁻, SH3⁺ (CD73⁺), SH4⁺ (CD73⁺), CD80⁻, CD86⁻, CD90⁺, SH2⁺ (CD105⁺), CD106/VCAM⁺, CD117⁻, CD144/VE-cadherin^{low}, CD184/CXCR4⁻, CD200⁺, CD133⁻, OCT-4⁺, SSEA3⁻, SSEA4⁻, ABC-p⁺, KDR⁻ (VEGFR2⁻), HLA-A,B,C⁺, HLA-DP,DQ,DR⁻, HLA-G⁻, or Programmed Death-1 Ligand (PDL1)⁺, or any combination thereof. In a other embodiment, the CD34⁻, CD10⁺, CD105⁺ placental cells are additionally CD13⁺, CD29⁺, CD33⁺, CD38⁻, CD44⁺, CD45⁻, CD54/ICAM⁺, CD62E⁻, CD62L⁻, CD62P⁻, SH3⁺ (CD73⁺), SH4⁺ (CD73⁺), CD80⁻, CD86⁻, CD90⁺, SH2⁺ (CD105⁺), CD106/VCAM⁺, CD117⁻, CD144/VE-cadherin^{low}, CD184/CXCR4⁻, CD200⁺, CD133⁻, OCT-4⁺, SSEA3⁻, SSEA4⁻, ABC-p⁺, KDR⁻ (VEGFR2⁻), HLA-A,B,C⁺, HLA-DP,DQ,DR⁻, HLA-G⁻, and Programmed Death-1 Ligand (PDL1)⁺.

In another specific embodiment, any of the placental cells described herein are ABC-p⁺, as detected by flow cytometry, or OCT-4⁺ (POU5F1⁺), as determined by RT-PCR, wherein ABC-p is a placenta-specific ABC transporter protein (also known as breast cancer resistance protein (BCRP) and as mitoxantrone resistance protein (MXR)), and OCT-4 is the Octamer-4 protein (POU5F1). In another specific embodiment, any of the placental cells described herein are additionally SSEA3⁻ or SSEA4⁻, as determined by flow cytometry, wherein SSEA3 is Stage Specific Embryonic Antigen 3, and SSEA4 is Stage Specific Embryonic Antigen 4. In another specific embodiment, any of the placental cells described herein are additionally SSEA3⁻ and SSEA4⁻.

In another specific embodiment, any of the placental cells described herein are one or more of MHC-I⁺ (*e.g*., HLA-A,B,C⁺), MHC-II⁻ (*e.g.,* HLA-DP,DQ,DR⁻) or HLA-G⁻. In another specific embodiment, any of the placental cells described herein are one or more of MHC-I⁺ (*e.g*., HLA-A,B,C⁺), MHC-II⁻ (*e.g.,* HLA-DP,DQ,DR⁻) and HLA-G⁻.

Also provided herein are populations of the isolated placental cells, or populations of cells, *e.g.,* populations of placental cells, comprising, *e.g.,* that are enriched for, the isolated placental cells, that are useful in the methods and compositions disclosed herein. Preferred populations of cells comprising the isolated placental cells, wherein the populations of cells comprise, e.g., at least 10%, 15%, 20%, 25%, 30%, 35%, 40%, 45%, 50%, 55%, 60%, 65%, 70%, 75%, 80%, 85%, 90%, 95% or 98% isolated CD10⁺, CD105⁺ and CD34⁻ placental cells; that is, at least 10%, 15%, 20%, 25%, 30%, 35%, 40%, 45%, 50%, 55%, 60%, 65%, 70%, 75%, 80%, 85%, 90%, 95% or 98% of cells in said population are isolated CD10⁺, CD105⁺ and CD34⁻ placental cells. In a specific aspect embodiment, the isolated CD34⁻, CD10⁺, CD105⁺ placental cells are additionally CD200⁺. In another specific embodiment, the isolated CD34⁻, CD10⁺, CD105⁺, CD200⁺ placental cells are additionally CD90⁺ or CD45⁻, as detected by flow cytometry. In another specific embodiment, the isolated CD34⁻, CD10⁺, CD105⁺, CD200⁺ placental cells are additionally CD90⁺ and CD45⁻, as detected by flow cytometry. In another specific embodiment, any of the isolated CD34⁻, CD10⁺, CD105⁺ placental cells described above are additionally one or more of CD29⁺, CD38⁻, CD44⁺, CD54⁺, SH3⁺ or SH4⁺. In another specific embodiment, the isolated CD34⁻, CD10⁺, CD105⁺ placental cells, or isolated CD34⁻, CD10⁺, CD105⁺, CD200⁺ placental cells, are additionally CD44⁺. In a specific embodiment of any of the populations of cells comprising isolated CD34⁻, CD10⁺, CD105⁺ placental cells above, the isolated placental cells are additionally one or more of CD13⁺, CD29⁺, CD33⁺, CD38⁻, CD44⁺, CD45⁻, CD54⁺, CD62E⁻, CD62L⁻, CD62P⁻, SH3⁺ (CD73⁺), SH4⁺ (CD73⁺), CD80⁻, CD86⁻, CD90⁺, SH2⁺ (CD105⁺), CD106/VCAM⁺, CD117⁻, CD144/VE-cadherin^{low}, CD184/CXCR4⁻, CD200⁺, CD133⁻, OCT-4⁺, SSEA3⁻, SSEA4⁻, ABC-p⁺, KDR⁻ (VEGFR2⁻), HLA-A,B,C⁺, HLA-DP,DQ,DR⁻, HLA-G⁻, or Programmed Death-1 Ligand (PDL1)⁺, or any combination thereof. In another specific embodiment, the CD34⁻, CD10⁺, CD105⁺ cells are additionally CD13⁺, CD29⁺, CD33⁺, CD38⁻, CD44⁺, CD45⁻, CD54/ICAM⁺, CD62E⁻, CD62L⁻, CD62P⁻, SH3⁺ (CD73⁺), SH4⁺ (CD73⁺), CD80⁻, CD86⁻, CD90⁺, SH2⁺ (CD105⁺), CD106/VCAM⁺, CD117⁻, CD144/VE-cadherin^{low}, CD184/CXCR4⁻, CD200⁺, CD133⁻, OCT-4⁺, SSEA3⁻, SSEA4⁻, ABC-p⁺, KDR⁻ (VEGFR2⁻), HLA-A,B,C⁺, HLA-DP,DQ,DR⁻, HLA-G⁻, and Programmed Death-1 Ligand (PDL1)⁺.

In certain embodiments, the isolated placental cells useful in the methods and compositions described herein are one or more, or all, of CD10⁺, CD29⁺, CD34⁻, CD38⁻, CD44⁺, CD45⁻, CD54⁺, CD90⁺, SH2⁺, SH3⁺, SH4⁺, SSEA3⁻, SSEA4⁻, OCT-4⁺, and ABC-p⁺, wherein said isolated placental cells are obtained by physical and/or enzymatic disruption of placental tissue. In a specific embodiment, the isolated placental cells are OCT-4⁺ and ABC-p⁺. In another specific embodiment, the isolated placental cells are OCT-4⁺ and CD34⁻, wherein said isolated placental cells have at least one of the following characteristics: CD10⁺, CD29⁺, CD44⁺, CD45⁻, CD54⁺, CD90⁺, SH3⁺, SH4⁺, SSEA3⁻, and SSEA4⁻. In another specific embodiment, the isolated placental cells are OCT-4⁺, CD34⁻, CD10⁺, CD29⁺, CD44⁺, CD45⁻, CD54⁺, CD90⁺, SH3⁺, SH4⁺, SSEA3⁻, and SSEA4⁻. In another embodiment, the isolated placental cells are OCT-4⁺, CD34⁻, SSEA3⁻, and SSEA4⁻. In another specific embodiment, the isolated placental cells are OCT-4⁺ and CD34⁻, and is either SH2⁺ or SH3⁺. In another specific embodiment, the isolated placental cells are OCT-4⁺, CD34⁻, SH2⁺, and SH3⁺. In another specific embodiment, the isolated placental cells are OCT-4⁺, CD34⁻, SSEA3⁻, and SSEA4⁻, and are either SH2⁺ or SH3⁺. In another specific embodiment, the isolated placental cells are OCT-4⁺ and CD34⁻, and either SH2⁺ or SH3⁺, and is at least one of CD10⁺, CD29⁺, CD44⁺, CD45⁻, CD54⁺, CD90⁺, SSEA3⁻, or SSEA4⁻. In another specific embodiment, the isolated placental cells are OCT-4⁺, CD34⁻, CD10⁺, CD29⁺, CD44⁺, CD45⁻, CD54⁺, CD90⁺, SSEA3⁻, and SSEA4⁻, and either SH2⁺ or SH3⁺.

In another embodiment, the isolated placental cells useful in the methods and compositions disclosed herein are SH2⁺, SH3⁺, SH4⁺ and OCT-4⁺. In another specific embodiment, the isolated placental cells are CD10⁺, CD29⁺, CD44⁺, CD54⁺, CD90⁺, CD34⁻, CD45⁻, SSEA3⁻, or SSEA4⁻. In another embodiment, the isolated placental cells are SH2⁺, SH3⁺, SH4⁺, SSEA3⁻ and SSEA4⁻. In another specific embodiment, the isolated placental cells are SH2⁺, SH3⁺, SH4⁺, SSEA3⁻ and SSEA4⁻, CD10⁺, CD29⁺, CD44⁺, CD54⁺, CD90⁺, OCT-4⁺, CD34⁻ or CD45⁻.

In another embodiment, the isolated placental cells useful in the methods and compositions disclosed herein are CD10⁺, CD29^{+,} CD34⁻, CD44^{+,} CD45⁻, CD54⁺, CD90⁺, SH2⁺, SH3⁺, and SH4⁺; wherein said isolated placental cells are additionally one or more of OCT-4⁺, SSEA3⁻ or SSEA4⁻.

In certain embodiments, isolated placental cells useful in the methods and compositions disclosed herein are CD200⁺ or HLA-G⁻. In a specific embodiment, the isolated placental cells are CD200⁺ and HLA-G⁻. In another specific, the isolated placental cells are additionally CD73⁺ and CD105⁺. In another specific, the isolated placental cells are additionally CD34⁻, CD38⁻ or CD45⁻. In another specific embodiment, the isolated placental cells are additionally CD34⁻, CD38⁻ and CD45⁻. In another specific embodiment, said stem cells are CD34⁻, CD38⁻, CD45⁻, CD73⁺ and CD105⁺. In another specific embodiment, said isolated CD200⁺ or HLA-G⁻ placental cells facilitate the formation of embryoid-like bodies in a population of placental cells comprising the isolated placental cells, under conditions that allow the formation of embryoid-like bodies. In another specific embodiment, the isolated placental cells are isolated away from placental cells that are not stem or multipotent cells. In another specific embodiment, said isolated placental cells are isolated away from placental cells that do not display these markers.

In another embodiment, a cell population useful in the methods and compositions described herein is a population of cells comprising, e.g., that is enriched for, CD200⁺, HLA-G⁻ stem cells. In a specific embodiment, said population is a population of placental cells. In various embodiments, at least about 10%, at least about 20%, at least about 30%, at least about 40%, at least about 50%, or at least about 60% of cells in said cell population are isolated CD200⁺, HLA-G⁻ placental cells. In certain embodiments, at least about 70% of cells in said cell population are isolated CD200⁺, HLA-G⁻ placental cells. In certain other embodiments, at least about 90%, 95%, or 99% of said cells are isolated CD200⁺, HLA-G⁻ placental cells. In a specific embodiment of the cell populations, said isolated CD200⁺, HLA-G⁻ placental cells are also CD73⁺ and CD105⁺. In another specific embodiment, said isolated CD200⁺, HLA-G⁻ placental cells are also CD34⁻, CD38⁻ or CD45⁻. In another specific embodiment, said isolated CD200⁺, HLA-G⁻ placental cells are also CD34⁻, CD38⁻, CD45⁻, CD73⁺ and CD105⁺. In another embodiment, said cell population produces one or more embryoid-like bodies when cultured under conditions that allow the formation of embryoid-like bodies. In another specific embodiment, said cell population is isolated away from placental cells that are not stem cells. In another specific embodiment, said isolated CD200⁺, HLA-G⁻ placental cells are isolated away from placental cells that do not display these markers.

In another embodiment, the isolated placental cells useful in the methods and compositions described herein are CD73⁺, CD105⁺, and CD200⁺. In another specific embodiment, the isolated placental cells are HLA-G⁻. In another specific embodiment, the isolated placental cells are CD34⁻, CD38⁻ or CD45⁻. In another specific embodiment, the isolated placental cells are CD34⁻, CD38⁻ and CD45⁻. In another specific embodiment, the isolated placental cells are CD34⁻, CD38⁻, CD45⁻, and HLA-G⁻. In another specific embodiment, the isolated CD73⁺, CD105⁺, and CD200⁺ placental cells facilitate the formation of one or more embryoid-like bodies in a population of placental cells comprising the isolated placental cells, when the population is cultured under conditions that allow the formation of embryoid-like bodies. In another specific embodiment, the isolated placental cells are isolated away from placental cells that are not the isolated placental cells. In another specific embodiment, the isolated placental cells are isolated away from placental cells that do not display these markers.

In another embodiment, a cell population useful in the methods and compositions described herein is a population of cells comprising, e.g., that is enriched for, isolated CD73⁺, CD105⁺, CD200⁺ placental cells. In various embodiments, at least about 10%, at least about 20%, at least about 30%, at least about 40%, at least about 50%, or at least about 60% of cells in said cell population are isolated CD73⁺, CD105⁺, CD200⁺ placental cells. In another embodiment, at least about 70% of said cells in said population of cells are isolated CD73⁺, CD105⁺, CD200⁺ placental cells. In another embodiment, at least about 90%, 95% or 99% of cells in said population of cells are isolated CD73⁺, CD105⁺, CD200⁺ placental cells. In a specific embodiment of said populations, the isolated placental cells are HLA-G⁻. In another specific embodiment, the isolated placental cells are additionally CD34⁻, CD38⁻ or CD45⁻. In another specific embodiment, the isolated placental cells are additionally CD34⁻, CD38⁻ and CD45⁻. In another specific embodiment, the isolated placental cells are additionally CD34⁻, CD38⁻, CD45⁻, and HLA-G⁻. In another specific embodiment, said population of cells produces one or more embryoid-like bodies when cultured under conditions that allow the formation of embryoid-like bodies. In another specific embodiment, said population of placental cells is isolated away from placental cells that are not stem cells. In another specific embodiment, said population of placental cells is isolated away from placental cells that do not display these characteristics.

In certain other embodiments, the isolated placental cells are one or more of CD10⁺, CD29⁺, CD34⁻, CD38⁻, CD44⁺, CD45⁻, CD54⁺, CD90⁺, SH2⁺, SH3⁺, SH4⁺, SSEA3-, SSEA4⁻, OCT-4⁺, HLA-G⁻ or ABC-p⁺. In a specific embodiment, the isolated placental cells are CD10⁺, CD29⁺, CD34⁻, CD38⁻, CD44⁺, CD45⁻, CD54⁺, CD90⁺, SH2⁺, SH3⁺, SH4⁺, SSEA3-, SSEA4⁻, and OCT-4⁺. In another specific embodiment, the isolated placental cells are CD10⁺, CD29⁺, CD34⁻, CD38⁻, CD45⁻, CD54⁺, SH2⁺, SH3⁺, and SH4⁺. In another specific embodiment, the isolated placental cells are CD10⁺, CD29⁺, CD34⁻, CD38⁻, CD45⁻, CD54⁺, SH2⁺, SH3⁺, SH4⁺ and OCT-4⁺. In another specific embodiment, the isolated placental cells are CD10⁺, CD29⁺, CD34⁻, CD38⁻, CD44⁺, CD45⁻, CD54⁺, CD90⁺, HLA-G⁻, SH2⁺, SH3⁺, SH4⁺. In another specific embodiment, the isolated placental cells are OCT-4⁺ and ABC-p⁺. In another specific embodiment, the isolated placental cells are SH2⁺, SH3⁺, SH4⁺ and OCT-4⁺. In another embodiment, the isolated placental cells are OCT-4⁺, CD34⁻, SSEA3⁻, and SSEA4⁻. In a specific embodiment, said isolated OCT-4⁺, CD34⁻, SSEA3⁻, and SSEA4⁻ placental cells are additionally CD10⁺, CD29⁺, CD34⁻, CD44⁺, CD45⁻, CD54⁺, CD90⁺, SH2⁺, SH3⁺, and SH4⁺. In another embodiment, the isolated placental cells are OCT-4⁺ and CD34⁻, and either SH3⁺ or SH4⁺. In another embodiment, the isolated placental cells are CD34⁻ and either CD10⁺, CD29⁺, CD44⁺, CD54⁺, CD90⁺, or OCT-4⁺.

In another embodiment, the isolated placental cells useful in the methods and compositions described herein are CD200⁺ and OCT-4⁺. In a specific embodiment, the isolated placental cells are CD73⁺ and CD105⁺. In another specific embodiment, said isolated placental cells are HLA-G⁻. In another specific embodiment, said isolated CD200⁺, OCT-4⁺ placental cells are CD34⁻, CD38⁻ or CD45⁻. In another specific embodiment, said isolated CD200⁺, OCT-4⁺ placental cells are CD34⁻, CD38⁻ and CD45⁻. In another specific embodiment, said isolated CD200⁺, OCT-4⁺ placental cells are CD34⁻, CD38⁻, CD45⁻, CD73⁺, CD105⁺ and HLA-G⁻. In another specific embodiment, the isolated CD200⁺, OCT-4⁺ placental cells facilitate the production of one or more embryoid-like bodies by a population of placental cells that comprises the isolated cells, when the population is cultured under conditions that allow the formation of embryoid-like bodies. In another specific embodiment, said isolated CD200⁺, OCT-4⁺ placental cells are isolated away from placental cells that are not stem cells. In another specific embodiment, said isolated CD200⁺, OCT-4⁺ placental cells are isolated away from placental cells that do not display these characteristics.

In another embodiment, a cell population useful in the methods and compositions described herein is a population of cells comprising, e.g., that is enriched for, CD200⁺, OCT-4⁺ placental cells. In various embodiments, at least about 10%, at least about 20%, at least about 30%, at least about 40%, at least about 50%, or at least about 60% of cells in said cell population are isolated CD200⁺, OCT-4⁺ placental cells. In another embodiment, at least about 70% of said cells are said isolated CD200⁺, OCT-4⁺ placental cells. In another embodiment, at least about 80%, 90%, 95%, or 99% of cells in said cell population are said isolated CD200⁺, OCT-4⁺ placental cells. In a specific embodiment of the isolated populations, said isolated CD200⁺, OCT-4⁺ placental cells are additionally CD73⁺ and CD105⁺. In another specific embodiment, said isolated CD200⁺, OCT-4⁺ placental cells are additionally HLA-G⁻. In another specific embodiment, said isolated CD200⁺, OCT-4⁺ placental cells are additionally CD34⁻, CD38⁻ and CD45⁻. In another specific embodiment, said isolated CD200⁺, OCT-4⁺ placental cells are additionally CD34⁻, CD38⁻, CD45⁻, CD73⁺, CD105⁺ and HLA-G⁻. In another specific embodiment, the cell population produces one or more embryoid-like bodies when cultured under conditions that allow the formation of embryoid-like bodies. In another specific embodiment, said cell population is isolated away from placental cells that are not isolated CD200⁺, OCT-4⁺ placental cells. In another specific embodiment, said cell population is isolated away from placental cells that do not display these markers.

In another embodiment, the isolated placental cells useful in the methods and compositions described herein are CD73⁺, CD105⁺ and HLA-G⁻. In another specific embodiment, the isolated CD73⁺, CD105⁺ and HLA-G⁻ placental cells are additionally CD34⁻, CD38⁻ or CD45⁻. In another specific embodiment, the isolated CD73⁺, CD105⁺, HLA-G⁻ placental cells are additionally CD34⁻, CD38⁻ and CD45⁻. In another specific embodiment, the isolated CD73⁺, CD105⁺, HLA-G⁻ placental cells are additionally OCT-4⁺. In another specific embodiment, the isolated CD73⁺, CD105⁺, HLA-G⁻ placental cells are additionally CD200⁺. In another specific embodiment, the isolated CD73⁺, CD105⁺, HLA-G⁻ placental cells are additionally CD34⁻, CD38⁻, CD45⁻, OCT-4⁺ and CD200⁺. In another specific embodiment, the isolated CD73⁺, CD105⁺, HLA-G⁻ placental cells facilitate the formation of embryoid-like bodies in a population of placental cells comprising said cells, when the population is cultured under conditions that allow the formation of embryoid-like bodies. In another specific embodiment, said the isolated CD73⁺, CD105⁺, HLA-G⁻ placental cells are isolated away from placental cells that are not the isolated CD73⁺, CD105⁺, HLA-G⁻ placental cells. In another specific embodiment, said the isolated CD73⁺, CD105⁺, HLA-G⁻ placental cells are isolated away from placental cells that do not display these markers.

In another embodiment, a cell population useful in the methods and compositions described herein is a population of cells comprising, e.g., that is enriched for, isolated CD73⁺, CD105⁺ and HLA-G⁻ placental cells. In various embodiments, at least about 10%, at least about 20%, at least about 30%, at least about 40%, at least about 50%, or at least about 60% of cells in said population of cells are isolated CD73⁺, CD105⁺, HLA-G⁻ placental cells. In another embodiment, at least about 70% of cells in said population of cells are isolated CD73⁺, CD105⁺, HLA-G⁻ placental cells. In another embodiment, at least about 90%, 95% or 99% of cells in said population of cells are isolated CD73⁺, CD105⁺, HLA-G⁻ placental cells. In a specific embodiment of the above populations, said isolated CD73⁺, CD105⁺, HLA-G⁻ placental cells are additionally CD34⁻, CD38⁻ or CD45⁻. In another specific embodiment, said isolated CD73⁺, CD105⁺, HLA-G⁻ placental cells are additionally CD34⁻, CD38⁻ and CD45⁻. In another specific embodiment, said isolated CD73⁺, CD105⁺, HLA-G⁻ placental cells are additionally OCT-4⁺. In another specific embodiment, said isolated CD73⁺, CD105⁺, HLA-G⁻ placental cells are additionally CD200⁺. In another specific embodiment, said isolated CD73⁺, CD105⁺, HLA-G⁻ placental cells are additionally CD34⁻, CD38⁻, CD45⁻, OCT-4⁺ and CD200⁺. In another specific embodiment, said cell population is isolated away from placental cells that are not CD73⁺, CD105⁺, HLA-G⁻ placental cells. In another specific embodiment, said cell population is isolated away from placental cells that do not display these markers.

In another embodiment, the isolated placental cells useful in the methods and compositions described herein are CD73⁺ and CD105⁺ and facilitate the formation of one or more embryoid-like bodies in a population of isolated placental cells comprising said CD73⁺, CD105⁺ cells when said population is cultured under conditions that allow formation of embryoid-like bodies. In another specific embodiment, said isolated CD73⁺, CD105⁺ placental cells are additionally CD34⁻, CD38⁻ or CD45⁻. In another specific embodiment, said isolated CD73⁺, CD105⁺ placental cells are additionally CD34⁻, CD38⁻ and CD45⁻. In another specific embodiment, said isolated CD73⁺, CD105⁺ placental cells are additionally OCT-4⁺. In another specific embodiment, said isolated CD73⁺, CD105⁺ placental cells are additionally OCT-4⁺, CD34⁻, CD38⁻ and CD45⁻. In another specific embodiment, said isolated CD73⁺, CD105⁺ placental cells are isolated away from placental cells that are not said cells. In another specific embodiment, said isolated CD73⁺, CD105⁺ placental cells are isolated away from placental cells that do not display these characteristics.

In another embodiment, a cell population useful in the methods and compositions described herein is a population of cells comprising, *e.g.,* that is enriched for, isolated placental cells that are CD73⁺, CD105⁺ and facilitate the formation of one or more embryoid-like bodies in a population of isolated placental cells comprising said cells when said population is cultured under conditions that allow formation of embryoid-like bodies. In various embodiments, at least about 10%, at least about 20%, at least about 30%, at least about 40%, at least about 50%, or at least about 60% of cells in said population of cells are said isolated CD73⁺, CD105⁺ placental cells. In another embodiment, at least about 70% of cells in said population of cells are said isolated CD73⁺, CD105⁺ placental cells. In another embodiment, at least about 90%, 95% or 99% of cells in said population of cells are said isolated CD73⁺, CD105⁺ placental cells. In a specific embodiment of the above populations, said isolated CD73⁺, CD105⁺ placental cells are additionally CD34⁻, CD38⁻ or CD45⁻. In another specific embodiment, said isolated CD73⁺, CD105⁺ placental cells are additionally CD34⁻, CD38⁻ and CD45⁻. In another specific embodiment, said isolated CD73⁺, CD105⁺ placental cells are additionally OCT-4⁺. In another specific embodiment, said isolated CD73⁺, CD105⁺ placental cells are additionally CD200⁺. In another specific embodiment, said isolated CD73⁺, CD105⁺ placental cells are additionally CD34⁻, CD38⁻, CD45⁻, OCT-4⁺ and CD200⁺. In another specific embodiment, said cell population is isolated away from placental cells that are not said isolated CD73⁺, CD105⁺ placental cells. In another specific embodiment, said cell population is isolated away from placental cells that do not display these markers.

In another embodiment, the isolated placental cells useful in the methods and compositions described herein are OCT-4⁺ and facilitate formation of one or more embryoid-like bodies in a population of isolated placental cells comprising said cells when cultured under conditions that allow formation of embryoid-like bodies. In a specific embodiment, said isolated OCT-4⁺ placental cells are additionally CD73⁺ and CD105⁺. In another specific embodiment, said isolated OCT-4⁺ placental cells are additionally CD34⁻, CD38⁻, or CD45⁻. In another specific embodiment, said isolated OCT-4⁺ placental cells are additionally CD200⁺. In another specific embodiment, said isolated OCT-4⁺ placental cells are additionally CD73⁺, CD105⁺, CD200⁺, CD34⁻, CD38⁻, and CD45⁻. In another specific embodiment, said isolated OCT-4⁺ placental cells are isolated away from placental cells that are not OCT-4⁺ placental cells. In another specific embodiment, said isolated OCT-4⁺ placental cells are isolated away from placental cells that do not display these characteristics.

In another embodiment, a cell population useful in the methods and compositions described herein is a population of cells comprising, *e.g.,* that is enriched for, isolated placental cells that are OCT-4⁺ and facilitate the formation of one or more embryoid-like bodies in a population of isolated placental cells comprising said cells when said population is cultured under conditions that allow formation of embryoid-like bodies. In various embodiments, at least about 10%, at least about 20%, at least about 30%, at least about 40%, at least about 50%, or at least about 60% of cells in said population of cells are said isolated OCT-4⁺ placental cells. In another embodiment, at least about 70% of cells in said population of cells are said isolated OCT-4⁺ placental cells. In another embodiment, at least about 80%, 90%, 95% or 99% of cells in said population of cells are said isolated OCT-4⁺ placental cells. In a specific embodiment of the above populations, said isolated OCT-4⁺ placental cells are additionally CD34⁻, CD38⁻ or CD45⁻. In another specific embodiment, said isolated OCT-4⁺ placental cells are additionally CD34⁻, CD38⁻ and CD45⁻. In another specific embodiment, said isolated OCT-4⁺ placental cells are additionally CD73⁺ and CD105⁺. In another specific embodiment, said isolated OCT-4⁺ placental cells are additionally CD200⁺. In another specific embodiment, said isolated OCT-4⁺ placental cells are additionally CD73⁺, CD105⁺, CD200⁺, CD34⁻, CD38⁻, and CD45⁻. In another specific embodiment, said cell population is isolated away from placental cells that are not said cells. In another specific embodiment, said cell population is isolated away from placental cells that do not display these markers.

In another embodiment, the isolated placental cells useful in the methods and compositions described herein are isolated HLA-A,B,C⁺, CD45⁻, CD133⁻ and CD34⁻ placental cells. In another embodiment, a cell population useful in the methods and compositions described herein is a population of cells comprising isolated placental cells, wherein at least about 70%, at least about 80%, at least about 90%, at least about 95% or at least about 99% of cells in said isolated population of cells are isolated HLA-A,B,C⁺, CD45⁻, CD133⁻ and CD34⁻ placental cells. In a specific embodiment, said isolated placental cell or population of isolated placental cells is isolated away from placental cells that are not HLA-A,B,C+, CD45⁻, CD133⁻ and CD34⁻ placental cells. In another specific embodiment of any of the placental cells described herein, said isolated placental cells are non-maternal in origin. In another specific embodiment, said isolated population of placental cells are substantially free of maternal components; *e.g.,* at least about 40%, 45%, 5-0%, 55%, 60%, 65%, 70%, 75%, 90%, 85%, 90%, 95%, 98% or 99% of said cells in said isolated population of placental cells are non-maternal in origin.

In another embodiment, the isolated placental cells useful in the methods and compositions described herein are isolated CD10⁺, CD13⁺, CD33⁺, CD45⁻, CD117⁻ and CD133⁻ placental cells. In another embodiment, a cell population useful in the methods and compositions described herein is a population of cells comprising isolated placental cells, wherein at least about 70%, at least about 80%, at least about 90%, at least about 95% or at least about 99% of cells in said population of cells are isolated CD10⁺, CD13⁺, CD33⁺, CD45⁻, CD117⁻ and CD133⁻ placental cells. In a specific embodiment, said isolated placental cells or population of isolated placental cells is isolated away from placental cells that are not said isolated placental cells. In another specific embodiment, said isolated CD10⁺, CD13⁺, CD33⁺, CD45⁻, CD117⁻ and CD133⁻ placental cells are non-maternal in origin, *i.e.,* have the fetal genotype. In another specific embodiment, at least about 40%, 45%, 50%, 55%, 60%, 65%, 70%, 75%, 90%, 85%, 90%, 95%, 98% or 99% of said cells in said isolated population of placental cells, are non-maternal in origin. In another specific embodiment, said isolated placental cells or population of isolated placental cells are isolated away from placental cells that do not display these characteristics.

In another embodiment, the isolated placental cells useful in the methods and compositions described herein are isolated CD10⁻, CD33⁻, CD44⁺, CD45⁻, and CD117⁻ placental cells. In another embodiment, a cell population useful for the in the methods and compositions described herein is a population of cells comprising, *e.g.,* enriched for, isolated placental cells, wherein at least about 70%, at least about 80%, at least about 90%, at least about 95% or at least about 99% of cells in said population of cells are isolated CD10⁻, CD33⁻, CD44⁺, CD45⁻, and CD117⁻ placental cells. In a specific embodiment, said isolated placental cell or population of isolated placental cells is isolated away from placental cells that are not said cells. In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental cell or population of isolated placental cells is isolated away from placental cells that do not display these markers.

In another embodiment, the isolated placental cells useful in the methods and compositions described herein are isolated CD10⁻, CD13⁻, CD33⁻, CD45⁻, and CD117⁻ placental cells. In another embodiment, a cell population useful for in the methods and compositions described herein is a population of cells comprising, *e.g.,* enriched for, isolated CD10⁻, CD13⁻, CD33⁻, CD45⁻, and CD117⁻ placental cells, wherein at least about 70%, at least about 80%, at least about 90%, at least about 95% or at least about 99% of cells in said population are CD10⁻, CD13⁻, CD33⁻, CD45⁻, and CD117⁻ placental cells. In a specific embodiment, said isolated placental cells or population of isolated placental cells are isolated away from placental cells that are not said cells. In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental cells or population of isolated placental cells is isolated away from placental cells that do not display these characteristics.

In another embodiment, the isolated placental cells useful in the methods and compositions described herein are HLA A,B,C+, CD45⁻, CD34⁻, and CD133⁻, and are additionally CD10⁺, CD13⁺, CD38⁺, CD44⁺, CD90⁺, CD105⁺, CD200⁺ and/or HLA-G⁻, and/or negative for CD117. In another embodiment, a cell population useful in the methods described herein is a population of cells comprising isolated placental cells, wherein at least about 20%, 25%, 30%, 35%, 40%, 45%, 50%, 55%, 60%, 65%, 70%, 75%, 80%, 85%, 90%, 95%, 98% or about 99% of the cells in said population are isolated placental cells that are HLA A,B,C⁻, CD45⁻, CD34⁻, CD133⁻, and that are additionally positive for CD10, CD13, CD38, CD44, CD90, CD105, CD200, and/or negative for CD117 and/or HLA-G. In a specific embodiment, said isolated placental cells or population of isolated placental cells are isolated away from placental cells that are not said cells. In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental cells or population of isolated placental cells are isolated away from placental cells that do not display these markers.

In another embodiment, the isolated placental cells useful in the methods and compositions described herein are isolated placental cells that are CD200⁺ and CD10⁺, as determined by antibody binding, and CD117⁻, as determined by both antibody binding and RT-PCR. In another embodiment, the isolated placental cells useful in the methods and compositions described herein are isolated placental cells, *e.g.,* placental stem cells or placental multipotent cells, that are CD10⁺, CD29⁻, CD54⁺, CD200⁺, HLA-G⁻, MHC class I⁺ and β-2-microglobulin⁺. In another embodiment, isolated placental cells useful in the methods and compositions described herein are placental cells wherein the expression of at least one cellular marker is at least two-fold higher than for a mesenchymal stem cell (*e.g.,* a bone marrow-derived mesenchymal stem cell). In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin.

In another embodiment, the isolated placental cells useful in the methods and compositions described herein are isolated placental cells, *e.g.,* placental stem cells or placental multipotent cells, that are one or more of CD10⁺, CD29⁺, CD44⁺, CD45⁻, CD54/ICAM⁺, CD62E⁻, CD62L⁻, CD62P⁻, CD80⁻, CD86⁻, CD103⁻, CD104⁻, CD105⁺, CD106/VCAM⁺, CD144/VE-cadherin^{low}, CD184/CXCR4⁻, β2-microglobulin^{low}, MHC-I^{low}, MHC-II⁻, HLA-G^{low}, and/or PDL1^{low}. In a specific embodiment, the isolated placental cells are at least CD29⁺ and CD54⁺. In another specific embodiment, the isolated placental cells are at least CD44⁺ and CD106⁺. In another specific embodiment, the isolated placental cells are at least CD29⁺.

In another embodiment, a cell population useful in the methods and compositions described herein comprises isolated placental cells, and at least 50%, 60%, 70%, 80%, 90%, 95%, 98% or 99% of the cells in said cell population are isolated placental cells that are one or more of CD10⁺, CD29⁺, CD44⁺, CD45⁻, CD54/ICAM⁺, CD62-E⁻, CD62-L⁻, CD62-P⁻, CD80⁻, CD86⁻, CD103⁻, CD104⁻, CD105⁺, CD106/VCAM⁺, CD144/VE-cadherin^{dim}, CD184/CXCR4⁻, β2-microglobulin^{dim}, HLA-I^{dim}, HLA-II⁻, HLA-G^{dim}, and/or PDL1^{dim}. In another specific embodiment, at least 50%, 60%, 70%, 80%, 90%, 95%, 98% or 99% of cells in said cell population are CD10⁺, CD29⁺, CD44⁺, CD45⁻, CD54/ICAM⁺, CD62-E⁻, CD62-L⁻, CD62-P⁻, CD80⁻, CD86⁻, CD103⁻, CD104⁻, CD105⁺, CD106/VCAM⁺, CD144/VE-cadherin^{dim}, CD184/CXCR4⁻, β2-microglobulin^{dim}, MHC-I^{dim}, MHC-II⁻, HLA-G^{dim}, and PDL1^{dim}.

In another embodiment, the isolated placental cells useful in the methods and compositions described herein are isolated placental cells that are one or more, or all, of CD10⁺, CD29⁺, CD34⁻, CD38⁻, CD44⁺, CD45⁻, CD54⁺, CD90⁺, SH2⁺, SH3⁺, SH4⁺, SSEA3⁻, SSEA4⁻, OCT-4⁺, and ABC-p⁺, where ABC-p is a placenta-specific ABC transporter protein (also known as breast cancer resistance protein (BCRP) and as mitoxantrone resistance protein (MXR)), wherein said isolated placental cells are obtained by perfusion of a mammalian, *e.g.,* human, placenta that has been drained of cord blood and perfused to remove residual blood.

In another specific embodiment of any of the embodiments of placental cells described herein, the placental cells are negative for telomerase gene expression, negative for telomerase activity, or both. Telomerase gene expression can be detected using, *e.g.,* detection of telomerase RNA using, *e.g.,* dot blots or slot blots; or a telomere repeat amplification protocol (TRAP) assay (*e.g*., TRAPEZE® ELISA, fluorometric or gel-based assay kits from Millipore).

In another specific embodiment of any of the embodiments of placental cells described herein, the placental cells are positive for vimentin, *e.g.,* at least 50%, 55%, 60%, 65%, 70%, 75%, 80%, 85%, 90%,. 95%, or 98% of said placental cells express vimentin. Vimentin can be detected, *e.g.,* by flow cytometry using one or more antibodies to vimentin, *e.g.,* that are available from Abcam; by *in situ* fluorescent staining, or the like.

In another specific embodiment of any of the embodiments of placental cells described herein, the placental cells do not secrete detectable amounts of human chorionic gonadotropin (hCG). Human chorionic gonadotropin can be detected, *e.g.,* by ELISA or immunofluorescence using, for example, hCG monoclonal antibody HCG1 (Abcam), or polyclonal anti-hCG antibodies (Abcam, Novus Biologicals).

In another embodiment of any of the isolated placental cells described herein, a population of the isolated placental cells comprises CD56⁺ tissue culture plastic-adherent placental cells that are not natural killer cells. In a specific embodiment, the population comprises about 1% to about 27% of said CD56⁺ placental cells in said population of isolated placental cells, as determined by flow cytometry using CD56-FITC (fluorescein isothiocyanate). In another specific embodiment, the population comprises about 16% to about 62% of said CD56⁺ placental cells in said population of isolated placental cells, as determined by flow cytometry using CD56-APC (allophycocyanin).

In another specific embodiment of any of the above characteristics, expression of the cellular marker (*e.g*., cluster of differentiation or immunogenic marker) is determined by flow cytometry; in another specific embodiment, expression of the marker is determined by RT-PCR.

In any of the embodiments of the adherent placental cells, *e.g.,* placental stem cells, described herein, in a specific embodiment, the cells have additionally been identified as detectably suppressing cancer cell proliferation or tumor growth. In any of the embodiments of the adherent placental cells, *e.g.,* placental stem cells, described herein, in a specific embodiment, the cells detectably suppress cancer cell proliferation or tumor growth, *e.g., in vitro.*

Each of the above-referenced isolated adherent placental cells or populations thereof can comprise cells obtained and isolated directly from a mammalian placenta, or cells that have been cultured and passaged at least 1, 2, 3, 4, 5, 6, 7, 8, 9, 10, 12, 14, 16, 18, 20, 25, 30 or more times, or a combination thereof. Tumor cell suppressive pluralities of the isolated adherent placental cells described above can comprise about, at least, or no more than, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more isolated adherent placental cells.

### 5.7.3. Compositions Comprising Adherent Placental Cell Conditioned Media

Also provided herein is the use of a composition comprising natural killer cells comprising CD56+ CD16- CD3- PINK cells, placental perfusate and/or placental perfusate, and additionally conditioned medium, wherein said composition is tumor suppressive, or is effective in the treatment of cancer or viral infection. Adherent placental cells as described in Section 5.6.2, above, placental perfusate cells and/or natural killer cells, for example, placental intermediate natural killer cells can be used to produce conditioned medium that is tumor cell suppressive, anti-cancer or anti-viral that is, medium comprising one or more biomolecules secreted or excreted by the cells that have a detectable tumor cell suppressive effect, anti-cancer effect or antiviral effect. In various embodiments, the conditioned medium comprises medium in which cells (*e.g*., isolated adherent placental cells, placental perfusate cells, and/or natural killer cells, *e.g.,* PINK cells) have grown (that is, have been cultured) for at least 1, 2, 3, 4, 5, 6, 7, 8, 9, 10, 11, 12, 13, 14 or more days. In other embodiments, the conditioned medium comprises medium in which such cells have grown to at least 30%, 40%, 50%, 60%, 70%, 80%, 90% confluence, or up to 100% confluence. Such conditioned medium can be used to support the culture of a separate population of cells, *e.g.,* placental cells, or cells of another kind. In another, the conditioned medium provided herein comprises medium in which isolated adherent placental cells, *e.g.,* isolated adherent placental stem cells or isolated adherent placental multipotent cells, and cells other than isolated adherent placental cells, *e.g.,* non-placental stem cells or multipotent cells, have been cultured.

Such conditioned medium can be combined with any of, or any combination of, placental perfusate, placental perfusate cells, and/or natural killer cells, *e.g.,* placental intermediate natural killer cells, to form a composition that is tumor cell suppressive, anticancer or antiviral. In certain embodiments, the composition comprises less than half conditioned medium by volume, *e.g.,* about, or less than about, 50%, 45%, 40%, 35%, 30%, 25%, 20%, 15%, 10%, 5%, 4%, 3%, 2%, or 1% by volume.

Thus, in one embodiment, provided herein is a composition comprising culture medium from a culture of isolated adherent placental cells, wherein said isolated adherent placental cells (a) adhere to a substrate; and (b) are CD34⁻, CD10⁺ and CD105⁺; wherein said composition detectably suppresses the growth or proliferation of tumor cells, or is anti-cancer or antiviral. In a specific embodiment, the isolated adherent placental cells are CD34⁻, CD10⁺ and CD105⁺ as detected by flow cytometry. In a more specific embodiment, the isolated CD34⁻, CD10⁺, CD105⁺ adherent placental cells are placental stem cells. In another more specific embodiment, the isolated CD34⁻, CD10⁺, CD105⁺ placental cells are multipotent adherent placental cells. In another specific embodiment, the isolated CD34⁻, CD10⁺, CD105⁺ placental cells have the potential to differentiate into cells having a characteristic of a neural cell, cells having a characteristic of an osteogenic cell, and/or cells having a characteristic of a chondrogenic cell. In a more specific embodiment, the isolated CD34⁻, CD10⁺, CD105⁺ adherent placental cells are additionally CD200⁺. In another more specific embodiment, the isolated CD34⁻, CD10⁺, CD105⁺ adherent placental cells are additionally CD90⁺ or CD45⁻, as detected by flow cytometry. In another more specific embodiment, the isolated CD34⁻, CD10⁺, CD105⁺ adherent placental cells are additionally CD90⁺ and CD45⁻, as detected by flow cytometry. In a more specific embodiment, the CD34⁻, CD10⁺, CD105⁺, CD200⁺ adherent placental cells are additionally CD90⁺ or CD45⁻, as detected by flow cytometry. In another more specific embodiment, the CD34⁻, CD10⁺, CD105⁺, CD200⁺ adherent placental cells are additionally CD90⁺ and CD45⁻, as detected by flow cytometry. In another more specific embodiment, the CD34⁻, CD10⁺, CD105⁺, CD200⁺, CD90⁺, CD45⁻ adherent placental cells are additionally CD80⁻ and CD86⁻, as detected by flow cytometry.

In another embodiment, provided herein is a composition comprising culture medium from a culture of isolated adherent placental cells, wherein said isolated adherent placental cells (a) adhere to a substrate; and (b) express CD200 and do not express HLA-G, or express CD73, CD105, and CD200, or express CD200 and OCT-4, or express CD73 and CD105, and do not express HLA-G, or express CD73 and CD105 and facilitate the formation of one or more embryoid-like bodies in a population of placental cells that comprise the placental stem cells when said population is cultured under conditions that allow formation of embryoid-like bodies, or express OCT-4 and facilitate the formation of one or more embryoid-like bodies in a population of placental cells that comprise the placental stem cells when said population is cultured under conditions that allow formation of embryoid-like bodies; wherein said composition detectably suppresses the growth or proliferation of tumor cells, or is anti-cancer or antiviral. In a specific embodiment, the composition further comprises a plurality of said isolated placental adherent cells. In another specific embodiment, the composition comprises a plurality of non-placental cells. In a more specific embodiment, said non-placental cells comprise CD34⁺ cells, *e.g.,* hematopoietic progenitor cells, such as peripheral blood hematopoietic progenitor cells, cord blood hematopoietic progenitor cells, or placental blood hematopoietic progenitor cells. The non-placental cells can also comprise stem cells, such as mesenchymal stem cells, *e.g.,* bone marrow-derived mesenchymal stem cells. The non-placental cells can also be one ore more types of adult cells or cell lines. In another specific embodiment, the composition comprises an anti-proliferative agent, *e.g.,* an anti-MIP-1α or anti-MIP-1β antibody.

In a specific embodiment, culture medium conditioned by one of the cells or cell combinations described above is obtained from a plurality of isolated adherent placental cells co-cultured with a plurality of tumor cells at a ratio of about 1:1, about 2:1, about 3:1, about 4:1, or about 5:1 isolated adherent placental cells to tumor cells. For example, the conditioned culture medium or supernatant can be obtained from a culture comprising about 1 x 10⁵ isolated adherent placental cells, about 1 x 10⁶ isolated adherent placental cells, about 1 x 10⁷ isolated adherent placental cells, or about 1 x 10⁸ isolated adherent placental cells, or more. In another specific embodiment, the conditioned culture medium or supernatant is obtained from a co-culture comprising about 1 x 10⁵ to about 5 x 10⁵ isolated adherent placental cells and about 1 x 10⁵ tumor cells; about 1 x 10⁶ to about 5 x 10⁶ isolated adherent placental cells and about 1 x 10⁶ tumor cells; about 1 x 10⁷ to about 5 x 10⁷ isolated adherent placental cells and about 1 x 10⁷ tumor cells; or about 1 x 10⁸ to about 5 x 10⁸ isolated adherent placental cells and about 1 x 10⁸ tumor cells.

In a specific embodiment, the conditioned medium suitable for administration to a 70 kg individual comprises supernatant conditioned by about 70 million placental stem cells in about 200 mL culture medium.

Conditioned medium can be condensed to prepare an administrable pharmaceutical-grade product. For example, conditioned medium can be condensed to about 90%, 80%, 70%, 60%, 50%, 40%, 30%, 20%, 10% or more by removal of water, *e.g.,* by evaporation, lyophilization, or the like. In a specific embodiment, for example, 200 mL conditioned medium from about 70 million placental stem cells can be condensed to a volume of about 180 mL, 160 mL, 140 mL, 120 mL, 100 mL, 80 mL, 60 mL, 40 mL, 20 mL or less. The conditioned medium can also be substantially dried, *e.g.,* to a powder, *e.g.,* by evaporation, lyophilization or the like.

### 5.8. Preservation of Perfusate, Placental Perfusate Cells, and Natural Killer Cells

Placental perfusate, *e.g.,* perfusate comprising placental cells, or placental cells, *e.g.,* placental perfusate cells, combined natural killer cells, or natural killer cells, *e.g.,* PINK cells, can be preserved, that is, placed under conditions that allow for long-term storage, or under conditions that inhibit cell death by, *e.g.,* apoptosis or necrosis.

Placental perfusate can be produced by passage of a cell collection composition through at least a part of the placenta, *e.g.,* through the placental vasculature. The cell collection composition comprises one or more compounds that act to preserve cells contained within the perfusate. Such a placental cell collection composition can comprise an apoptosis inhibitor, necrosis inhibitor and/or an oxygen-carrying perfluorocarbon, as described in related U.S. Application Publication No. 20070190042 (U.S. Patent No. 9,598,669).

In one embodiment, perfusate or a population of placental cells are collected from a mammalian, *e.g.,* human, post-partum placenta by contacting the perfusate or population of cells with a cell collection composition comprising an inhibitor of apoptosis and an oxygen-carrying perfluorocarbon, wherein said inhibitor of apoptosis is present in an amount and for a time sufficient to reduce or prevent apoptosis in the population of placental cells, *e.g.,* adherent placental cells, for example, placental stem cells or placental multipotent cells, as compared to a population of cells not contacted with the inhibitor of apoptosis. For example, the placenta can be perfused with the cell collection composition, and placental cells, *e.g.,* total nucleated placental cells, are isolated therefrom. In a specific embodiment, the inhibitor of apoptosis is a caspase inhibitor. In another specific embodiment, said inhibitor of apoptosis is a JNK inhibitor. In a more specific embodiment, said JNK inhibitor does not modulate differentiation or proliferation of adherent placental cells, *e.g.,* adherent placental stem cells or adherent placental multipotent cells. In another embodiment, the cell collection composition comprises said inhibitor of apoptosis and said oxygen-carrying perfluorocarbon in separate phases. In another embodiment, the cell collection composition comprises said inhibitor of apoptosis and said oxygen-carrying perfluorocarbon in an emulsion. In another embodiment, the cell collection composition additionally comprises an emulsifier, *e.g.,* lecithin. In another embodiment, said apoptosis inhibitor and said perfluorocarbon are between about 0°C and about 25°C at the time of contacting the placental cells. In another more specific embodiment, said apoptosis inhibitor and said perfluorocarbon are between about 2°C and 10°C, or between about 2°C and about 5°C, at the time of contacting the placental cells. In another more specific embodiment, said contacting is performed during transport of said population of cells. In another more specific embodiment, said contacting is performed during freezing and thawing of said population of cells.

In another embodiment, placental perfusate and/or placental cells can be collected and preserved by contacting the perfusate and/or cells with an inhibitor of apoptosis and an organ-preserving compound, wherein said inhibitor of apoptosis is present in an amount and for a time sufficient to reduce or prevent apoptosis of the cells, as compared to perfusate or placental cells not contacted with the inhibitor of apoptosis. In a specific embodiment, the organ-preserving compound is UW solution (described in U.S. Patent No. 4,798,824; also known as VIASPAN™; *see also* Southard et al., Transplantation 49(2):251-257 (1990) or a solution described in Stern et al., U.S. Patent No. 5,552,267. In another embodiment, said organ-preserving composition is hydroxyethyl starch, lactobionic acid, raffinose, or a combination thereof. In another embodiment, the placental cell collection composition additionally comprises an oxygen-carrying perfluorocarbon, either in two phases or as an emulsion.

In another embodiment of the method, placental cells are contacted with a cell collection composition comprising an apoptosis inhibitor and oxygen-carrying perfluorocarbon, organ-preserving compound, or combination thereof, during perfusion. In another embodiment, placental cells are contacted with said cell collection compound after collection by perfusion.

Typically, during placental cell collection, enrichment and isolation, it is preferable to minimize or eliminate cell stress due to hypoxia and mechanical stress. In another embodiment of the method, therefore, placental perfusate or a population of placental cells is exposed to a hypoxic condition during collection, enrichment or isolation for less than six hours during said preservation, wherein a hypoxic condition is a concentration of oxygen that is less than normal blood oxygen concentration. In a more specific embodiment, said perfusate or population of placental cells is exposed to said hypoxic condition for less than two hours during said preservation. In another more specific embodiment, said population of placental cells is exposed to said hypoxic condition for less than one hour, or less than thirty minutes, or is not exposed to a hypoxic condition, during collection, enrichment or isolation. In another specific embodiment, said population of placental cells is not exposed to shear stress during collection, enrichment or isolation.

The isolated adherent placental cells, or placental perfusate cells, provided herein can be cryopreserved, *e.g.,* in cryopreservation medium in small containers, *e.g.,* ampoules. Suitable cryopreservation medium includes, but is not limited to, culture medium including, *e.g.,* growth medium, or cell freezing medium, for example commercially available cell freezing medium, *e.g.,* C2695, C2639 or C6039 (Sigma). Cryopreservation medium preferably comprises DMSO (dimethylsulfoxide), at a concentration of, *e.g.,* about 10% (v/v). Cryopreservation medium may comprise additional agents, for example, methylcellulose and/or glycerol. Placental cells are preferably cooled at about 1°C/min during cryopreservation. A preferred cryopreservation temperature is about -80°C to about -180°C, preferably about -125°C to about -140°C. Cryopreserved placental cells can be transferred to liquid nitrogen prior to thawing for use. In some embodiments, for example, once the ampoules have reached about -90°C, they are transferred to a liquid nitrogen storage area. Cryopreserved cells preferably are thawed at a temperature of about 25°C to about 40°C, preferably to a temperature of about 37°C.

### 5.9. Immunomodulatory Compounds

In certain embodiments, the methods of tumor suppression, treatment of individuals having cancer (*e.g.,* a blood cancer or solid tumor), and treatment of individuals having a viral infection, provided herein, comprise contacting the tumor cells, or administering to said individual, an immunomodulatory compound, or pretreating placental natural killer cells comprising CD56+ CD16- CD3- PINK cells, with an immunomodulatory compound.

The immunomodulatory compounds provided herein encompass compounds known as IMiDs® (Celgene Corporation). As used herein and unless otherwise indicated, the terms "immunomodulatory compounds" may encompass certain small organic molecules that inhibit LPS induced monocyte TNF-α, IL-1β, IL-12, IL-6, MIP-1α, MCP-1, GM-CSF, G-CSF, and COX-2 production.

Exemplary immunomodulatory compounds include, but are not limited to, N-{[2-(2,6-dioxo(3-piperidyl)-1,3-dioxoisoindolin-4-yl]methyl}cyclopropyl-carboxamide; 3-[2-(2,6-dioxo-piperidin-3-yl)-1,3-dioxo-2,3-dihydro-1H-isoindol-4-ylmethyl]-1,1-dimethyl-urea; (-)-3-(3,4-Dimethoxy-phenyl)-3-(1-oxo-1,3-dihydro-isoindol-2-yl)-propionamide; (+)-3-(3,4-Dimethoxy-phenyl)-3-(1-oxo-1,3 -dihydro-isoindol-2-yl)-propionamide; (-)-{2-[1-(3-ethoxy-4-methoxyphenyl)-2-methylsulfonylethyl]-4-acetylaminoisoindoline-1,3-dione}; (+)-{2-[1-(3-ethoxy-4-methoxyphenyl)-2-methylsulfonylethyl]-4-acetylaminoisoindoline-1,3-dione}; difluoro-methoxy SelCIDs; 1-phthalimido-1-(3,4-diethoxyphenyl)ethane; 3-(3,4-dimethoxyphenyl)-3-(3,5-dimethoxyphenyl)acrylo nitrile; 1-oxo-2-(2,6-dioxopiperidin-3-yl)-4-aminoisoindoline; 1,3-dioxo-2-(2,6-dioxopiperidin-3-yl)-4-aminoisoindoline; 4-amino-2-(3-methyl-2,6-dioxo-piperidine-3-yl)-isoindole-1,3-dione; 3-(3-acetoamidophthalimido)-3-(3-ethoxy-4-methoxyphenyl)-N-hydroxypropionamide; 1-oxo-2-(2,6-dioxopiperidin-3-yl)-4-methylisoindoline; Cyclopropyl-N-{2-[(1S)-1-(3-ethoxy-4-methoxyphenyl)-2-(methylsulfonyl)ethyl]-3-oxoisoindoline-4-yl}carboxamide; Substituted 2-(3-hydroxy-2,6-dioxopiperidin-5-yl) isoindoline; N-[2-(2,6-Dioxo-piperidin-3-yl)-1,3-dioxo-2,3-dihydro-1H-isoindol-5-ylmethyl]-4-trifluoromethoxybenzamide; (S)-4-chloro-N-((2-(3-methyl-2,6-dioxopiperidin-3-yl)-1,3-dioxoisoindolin-5-yl)methyl) benzamide; Pyridine-2-carboxylic acid [2-[(3S)-3-methyl-2,6-dioxo-piperidin-3-yl]-1,3-dioxo-2,3-dihydro-1H-isoindol-5-ylmethyl]-amide; (S)-N-((2-(3-methyl-2,6-dioxopiperidin-3-yl)-1,3-dioxoisoindolin-5-yl)methyl)-4-(trifluoromethyl)benzamide; 3-(2,5-dimethyl-4-oxo-4H-quinazolin-3-yl)-piperidine-2,6-dione, and the like.

Specific examples of immunomodulatory compounds include cyano and carboxy derivatives of substituted styrenes such as those disclosed in U.S. Patent No. 5,929,117; 1-oxo-2-(2,6-dioxo-3-fluoropiperidin-3yl) isoindolines and 1,3-dioxo-2-(2,6-dioxo-3-fluoropiperidine-3-yl) isoindolines such as those described in U.S. Patent Nos. 5,874,448 and 5,955,476; the tetra substituted 2-(2,6-dioxopiperdin-3-yl)-1-oxoisoindolines described in U.S. Patent No. 5,798,368; 1-oxo and 1,3-dioxo-2-(2,6-dioxopiperidin-3-yl) isoindolines (*e.g.,* 4-methyl derivatives of thalidomide), substituted 2-(2,6-dioxopiperidin-3-yl) phthalimides and substituted 2-(2,6-dioxopiperidin-3-yl)-1-oxoisoindoles including, but not limited to, those disclosed in U.S. Patent Nos. 5,635,517, 6,281,230, 6,316,471, 6,403,613, 6,476,052 and 6,555,554; 1-oxo and 1,3-dioxoisoindolines substituted in the 4- or 5-position of the indoline ring (*e.g.,* 4-(4-amino-1,3-dioxoisoindoline-2-yl)-4-carbamoylbutanoic acid) described in U.S. Patent No. 6,380,239; isoindoline-1-one and isoindoline-1,3-dione substituted in the 2-position with 2,6-dioxo-3-hydroxypiperidin-5-yl (*e.g.,* 2-(2,6-dioxo-3-hydroxy-5-fluoropiperidin-5-yl)-4-aminoisoindolin-1-one) described in U.S. Patent No. 6,458,810; a class of non-polypeptide cyclic amides disclosed in U.S. Patent Nos. 5,698,579 and 5,877,200; and isoindole-imide compounds such as those described in U.S. Patent Application Publication No. 20030045552, U.S. Patent No. 7,091,353, and International Application No. PCT/US01/50401 (International Publication No. WO 02/059106). U.S. Patent Publication No. 20060205787 describes 4-amino-2-(3-methyl-2,6-dioxopiperidin-3-yl)-isoindole-1,3-dione compositions. U.S. Patent Publication No. 20070049618 describes isoindole-imide compounds.

Various immunomodulatory compounds disclosed herein contain one or more chiral centers, and can exist as racemic mixtures of enantiomers or mixtures of diastereomers. This invention encompasses the use of stereomerically pure forms of such compounds, as well as the use of mixtures of those forms. For example, mixtures comprising equal or unequal amounts of the enantiomers of a particular immunomodulatory compounds may be used. These isomers may be asymmetrically synthesized or resolved using standard techniques such as chiral columns or chiral resolving agents. (*See, e.g.,* Jacques, J., et al., Enantiomers, Racemates and Resolutions (Wiley-Interscience, New York, 1981); Wilen, S. H., et al., Tetrahedron 33:2725 (1977); Eliel, E. L., Stereochemistry of Carbon Compounds (McGraw-Hill, NY, 1962); and Wilen, S. H., Tables of Resolving Agents and Optical Resolutions p. 268 (E.L. Eliel, Ed., Univ. of Notre Dame Press, Notre Dame, IN, 1972)).

Immunomodulatory compounds provided herein include, but are not limited to, 1-oxo-and 1,3 dioxo-2-(2,6-dioxopiperidin-3-yl) isoindolines substituted with amino in the benzo ring as described in U.S. Patent No. 5,635,517.

These compounds have the structure I: 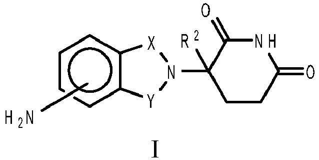 in which one of X and Y is C=O, the other of X and Y is C=O or CH₂, and R² is hydrogen or lower alkyl, in particular methyl. Specific immunomodulatory compounds include, but are not limited to: 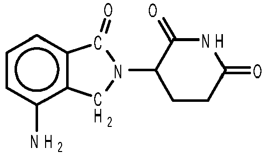 1-oxo-2-(2,6-dioxopiperidin-3-yl)-4-aminoisoindoline; 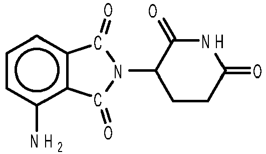 1,3-dioxo-2-(2,6-dioxopiperidin-3-yl)-4-aminoisoindoline; and 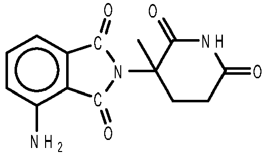 1,3-dioxo-2-(3-methyl-2,6-dioxopiperidin-3-yl)-4-aminoisoindole, and optically pure isomers thereof.

The compounds can be obtained via standard, synthetic methods (*see e.g.,* United States Patent No. 5,635,517). The compounds are also available from Celgene Corporation, Warren, NJ.

Other specific immunomodulatory compounds belong to a class of substituted 2-(2,6-dioxopiperidin-3-yl) phthalimides and substituted 2-(2,6-dioxopiperidin-3-yl)-1-oxoisoindoles, such as those described in U.S. Patent Nos. 6,281,230; 6,316,471; 6,335,349; and 6,476,052, and International Patent Application No. PCT/US97/13375 (International Publication No. WO 98/03502). Representative compounds are of formula: 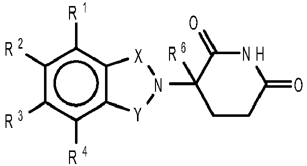 in which:
one of X and Y is C=O and the other of X and Y is C=O or CH₂;
(i) each of R¹, R², R³, and R⁴, independently of the others, is halo, alkyl of 1 to 4 carbon atoms, or alkoxy of 1 to 4 carbon atoms or (ii) one of R¹, R², R³, and R⁴ is -NHR⁵ and the remaining of R¹, R², R³, and R⁴ are hydrogen;
R⁵ is hydrogen or alkyl of 1 to 8 carbon atoms;
R⁶ is hydrogen, alkyl of 1 to 8 carbon atoms, benzyl, or halo;
provided that R⁶ is other than hydrogen if X and Y are C=O and (i) each of R¹, R², R³, and R⁴ is fluoro or (ii) one of R¹, R², R³, or R⁴ is amino.

Compounds representative of this class are of the formulas: 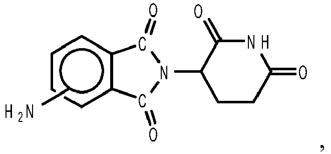 and 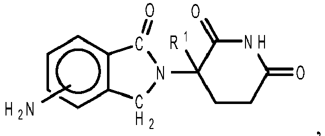 wherein R¹ is hydrogen or methyl. In a separate embodiment, the invention encompasses the use of enantiomerically pure forms (e.g. optically pure (R) or (S) enantiomers) of these compounds.

Still other specific immunomodulatory compounds disclosed herein belong to a class of isoindole-imides disclosed in U.S. Patent No. 7,091,353, U.S. Patent Publication No. 20030045552, and in International Application No. PCT/US01/50401 (International Application Publication No. WO 02/059106). Representative compounds are of formula II: 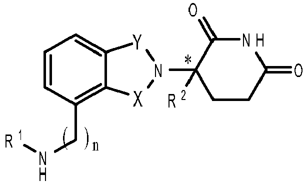 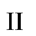 and pharmaceutically acceptable salts, hydrates, solvates, clathrates, enantiomers, diastereomers, racemates, and mixtures of stereoisomers thereof, wherein:
one of X and Y is C=O and the other is CH₂ or C=O;
R¹ is H, (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(S)R³, C(O)OR⁴, (C₁-C₈)alkyl-N(R⁶)2, (C₁-C₈)alkyl-OR⁵, (C₁-C₈)alkyl-C(O)OR⁵, C(O)NHR³, C(S)NHR³, C(O)NR³R^{3'}, C(S)NR³R^{3'} or (C₁-C₈)alkyl-O(CO)R⁵;
R² is H, F, benzyl, (C₁-C₈)alkyl, (C₂-C₈)alkenyl, or (C₂-C₈)alkynyl;
R³ and R^{3'} are independently (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, (C₀-C₈)alkyl-N(R⁶)2, (C₁-C₈)alkyl-OR⁵, (C₁-C₈)alkyl-C(O)OR⁵, (C₁-C₈)alkyl-O(CO)R⁵, or C(O)OR⁵;
R⁴ is (C₁-C₈)alkyl, (C₂-C₈)alkenyl, (C₂-C₈)alkynyl, (C₁-C₄)alkyl-OR⁵, benzyl, aryl, (C₀-C₄)alkyl-(C₁-C₆)heterocycloalkyl, or (C₀-C₄)alkyl-(C₂-C₅)heteroaryl;
R⁵ is (C₁-C₈)alkyl, (C₂-C₈)alkenyl, (C₂-C₈)alkynyl, benzyl, aryl, or (C₂-C₅)heteroaryl;
each occurrence of R⁶ is independently H, (C₁-C₈)alkyl, (C₂-C₈)alkenyl, (C₂-C₈)alkynyl, benzyl, aryl, (C₂-C₅)heteroaryl, or (C₀-C₈)alkyl-C(O)O-R⁵ or the R⁶ groups can join to form a heterocycloalkyl group;
n is 0 or 1; and
* represents a chiral-carbon center.

In specific compounds of formula II, when n is 0 then R¹ is (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(O)OR⁴, (C₁-C₈)alkyl-N(R⁶)₂, (C₁-C₈)alkyl-OR⁵, (C₁-C₈)alkyl-C(O)OR⁵, C(S)NHR³, or (C₁-C₈)alkyl-O(CO)R⁵;
R² is H or (C₁-C₈)alkyl; and
R³ is (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, (C₅-C₈)alkyl-N(R⁶)2 ; (C₀-C₈)alkyl-NH-C(O)O-R⁵; (C₁-C₈)alkyl-OR⁵, (C₁-C₈)alkyl-C(O)OR⁵, (C₁-C₈)alkyl-O(CO)R⁵, or C(O)OR⁵; and the other variables have the same definitions.

In other specific compounds of formula II, R² is H or (C₁-C₄)alkyl.

In other specific compounds of formula II, R¹ is (C₁-C₈)alkyl or benzyl.

In other specific compounds of formula II, R¹ is H, (C₁-C₈)alkyl, benzyl, CH₂OCH₃, CH₂CH₂OCH₃, or

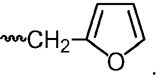

In another embodiment of the compounds of formula II, R¹ is 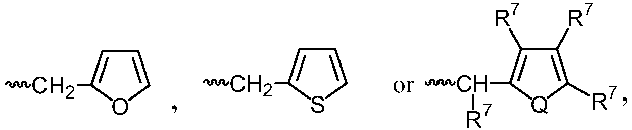 wherein Q is O or S, and each occurrence of R⁷ is independently H,(C₁-C₈)alkyl, (C₃-C₇)cycloalkyl, (C₂-C₈)alkenyl, (C₂-C₈)alkynyl, benzyl, aryl, halogen, (C₀-C₄)alkyl-(C₁-C₆)heterocycloalkyl, (C₀-C₄)alkyl-(C₂-C₅)heteroaryl, (C₀-C₈)alkyl-N(R⁶)2, (C₁-C₈)alkyl-OR⁵, (C₁-C₈)alkyl-C(O)OR⁵, (C₁-C₈)alkyl-O(CO)R⁵, or C(O)OR⁵, or adjacent occurrences of R⁷ can be taken together to form a bicyclic alkyl or aryl ring.

In other specific compounds of formula II, R¹ is C(O)R³.

In other specific compounds of formula II, R³ is (C₀-C₄)alkyl-(C₂-C₅)heteroaryl, (C₁-C₈)alkyl, aryl, or (C₀-C₄)alkyl-OR⁵.

In other specific compounds of formula II, heteroaryl is pyridyl, furyl, or thienyl.

In other specific compounds of formula II, R¹ is C(O)OR⁴.

In other specific compounds of formula II, the H of C(O)NHC(O) can be replaced with (C₁-C₄)alkyl, aryl, or benzyl.

Further examples of the compounds in this class include, but are not limited to: [2-(2,6-dioxo-piperidin-3-yl)-1,3-dioxo-2,3-dihydro-1H-isoindol-4-ylmethyl]-amide; (2-(2,6-dioxo-piperidin-3-yl)-1,3-dioxo-2,3-dihydro-1*H*-isoindol-4-ylmethyl)-carbamic acid *tert-*butyl ester; 4-(aminomethyl)-2-(2,6-dioxo(3-piperidyl))-isoindoline-1,3-dione; *N*-(2-(2,6-dioxo-piperidin-3-yl)-1,3-dioxo-2,3-dihydro-1*H*-isoindol-4-ylmethyl)-acetamide; *N*-{(2-(2,6-dioxo(3-piperidyl)-1,3-dioxoisoindolin-4-yl)methyl}cyclopropyl-carboxamide; 2-chloro-*N-*{(2-(2,6-dioxo(3-piperidyl))-1,3-dioxoisoindolin-4-yl)methyl}acetamide; *N*-(2-(2,6-dioxo(3-piperidyl))-1 ,3-dioxoisoindolin-4-yl)-3-pyridylcarboxamide; 3-{1-oxo-4-(benzylamino)isoindolin-2-yl}piperidine-2,6-dione; 2-(2,6-dioxo(3-piperidyl))-4-(benzylamino)isoindoline-1,3-dione; *N*-{(2-(2,6-dioxo(3-piperidyl))-1,3-dioxoisoindolin-4-yl)methyl}propanamide; *N*-{(2-(2,6-dioxo(3-piperidyl))-1,3-dioxoisoindolin-4-yl)methyl}-3-pyridylcarboxamide; *N*-{(2-(2,6-dioxo(3-piperidyl))-1,3-dioxoisoindolin-4-yl)methyl}heptanamide; *N*-{(2-(2,6-dioxo(3-piperidyl))-1,3-dioxoisoindolin-4-yl)methyl}-2-furylcarboxamide; {N-(2-(2,6-dioxo(3-piperidyl))-1,3-dioxoisoindolin-4-yl)carbamoyl}methyl acetate; *N*-(2-(2,6-dioxo(3-piperidyl))-1,3-dioxoisoindolin-4-yl)pentanamide; *N*-(2-(2,6-dioxo(3-piperidyl))-1,3-dioxoisoindolin-4-yl)-2-thienylcarboxamide; N-{[2-(2,6-dioxo(3-piperidyl))-1,3-dioxoisoindolin-4-yl] methyl}(butylamino)carboxamide; N-{[2-(2,6-dioxo(3-piperidyl))-1,3-dioxoisoindolin-4-yl] methyl}(octylamino)carboxamide; and N-{[2-(2,6-dioxo(3-piperidyl))-1,3-dioxoisoindolin-4-yl]methyl}(benzylamino)carboxamide.

Still other specific immunomodulatory compounds disclosed herein belong to a class of isoindole-imides disclosed in U.S. Patent No. 6,555,554, International Publication No. WO 98/54170, and U.S. Patent No. 6,395,754. Representative compounds are of formula III: 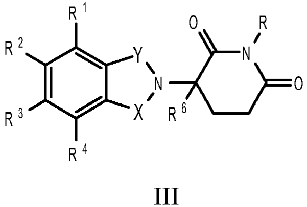 and pharmaceutically acceptable salts, hydrates, solvates, clathrates, enantiomers, diastereomers, racemates, and mixtures of stereoisomers thereof, wherein:
one of X and Y is C=O and the other is CH₂ or C=O;
R is H or CH₂OCOR';
(i) each of R¹, R², R³, or R⁴, independently of the others, is halo, alkyl of 1 to 4 carbon atoms, or alkoxy of 1 to 4 carbon atoms or (ii) one of R¹, R², R³, or R⁴ is nitro or -NHR⁵ and the remaining of R¹, R², R³, or R⁴ are hydrogen;
R⁵ is hydrogen or alkyl of 1 to 8 carbons
R⁶ hydrogen, alkyl of 1 to 8 carbon atoms, benzo, chloro, or fluoro;
R' is R⁷-CHR¹⁰-N(R⁸R⁹);
R⁷ is m-phenylene or p-phenylene or -(CnH2n)- in which n has a value of 0 to 4;
each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂X¹CH₂CH₂- in which X¹ is -O-, -S-, or -NH-;
R¹⁰ is hydrogen, alkyl of to 8 carbon atoms, or phenyl; and
* represents a chiral-carbon center.

Other representative compounds are of formula: 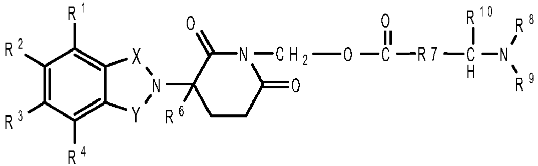 wherein:
one of X and Y is C=O and the other of X and Y is C=O or CH₂;
(i) each of R¹, R², R³, or R⁴, independently of the others, is halo, alkyl of 1 to 4 carbon atoms, or alkoxy of 1 to 4 carbon atoms or (ii) one of R¹, R², R³, and R⁴ is -NHR⁵ and the remaining of R¹, R², R³, and R⁴ are hydrogen;
R⁵ is hydrogen or alkyl of 1 to 8 carbon atoms;
R⁶ is hydrogen, alkyl of 1 to 8 carbon atoms, benzo, chloro, or fluoro;
R⁷ is m-phenylene or p-phenylene or -(CnH2n)- in which n has a value of 0 to 4;
each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂X¹CH₂CH₂- in which X¹ is -O-, -S-, or -NH-; and
R¹⁰ is hydrogen, alkyl of to 8 carbon atoms, or phenyl.

Other representative compounds are of formula: 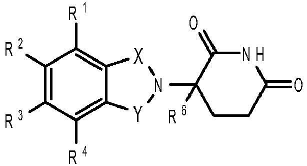 in which
one of X and Y is C=O and the other of X and Y is C=O or CH₂;
each of R¹, R², R³, and R⁴, independently of the others, is halo, alkyl of 1 to 4 carbon atoms, or alkoxy of 1 to 4 carbon atoms or (ii) one of R¹, R², R³, and R⁴ is nitro or protected amino and the remaining of R¹, R², R³, and R⁴ are hydrogen; and
R⁶ is hydrogen, alkyl of 1 to 8 carbon atoms, benzo, chloro, or fluoro.

Other representative compounds are of formula: 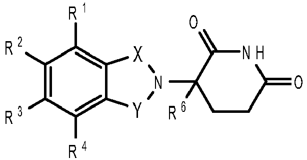 in which:
one of X and Y is C=O and the other of X and Y is C=O or CH₂;
(i) each of R¹, R², R³, and R⁴, independently of the others, is halo, alkyl of 1 to 4 carbon atoms, or alkoxy of 1 to 4 carbon atoms or (ii) one of R¹, R², R³, and R⁴ is -NHR⁵ and the remaining of R¹, R², R³, and R⁴ are hydrogen;
R⁵ is hydrogen, alkyl of 1 to 8 carbon atoms, or CO-R⁷-CH(R¹⁰)NR⁸R⁹ in which each of R⁷, R⁸, R⁹, and R¹⁰ is as herein defined; and
R⁶ is alkyl of 1 to 8 carbon atoms, benzo, chloro, or fluoro.

Specific examples of the compounds are of formula: 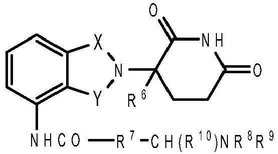 in which:
one of X and Y is C=O and the other of X and Y is C=O or CH₂;
R⁶ is hydrogen, alkyl of 1 to 8 carbon atoms, benzyl, chloro, or fluoro;
R⁷ is m-phenylene, p-phenylene or -(CnH2n)- in which n has a value of 0 to 4; each of R⁸ and R⁹ taken independently of the other is hydrogen or alkyl of 1 to 8 carbon atoms, or R⁸ and R⁹ taken together are tetramethylene, pentamethylene, hexamethylene, or-CH₂CH₂X¹CH₂CH₂- in which X¹ is -O-, -S- or -NH-; and
R¹⁰ is hydrogen, alkyl of 1 to 8 carbon atoms, or phenyl.

Other specific immunomodulatory compounds are 1-oxo-2-(2,6-dioxo-3-fluoropiperidin-3yl) isoindolines and 1,3-dioxo-2-(2,6-dioxo-3-fluoropiperidine-3-yl) isoindolines such as those described in U.S. Patent Nos. 5,874,448 and 5,955,476. Representative compounds are of formula: 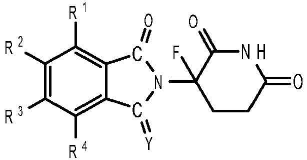 wherein:
Y is oxygen or H₂ and
each of R¹, R², R³, and R⁴, independently of the others, is hydrogen, halo, alkyl of 1 to 4 carbon atoms, alkoxy of 1 to 4 carbon atoms, or amino.

Other specific immunomodulatory compounds are the tetra substituted 2-(2,6-dioxopiperdin-3-yl)-1-oxoisoindolines described in U.S. Patent No. 5,798,368. Representative compounds are of formula: 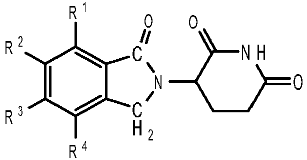 wherein each of R¹, R², R³, and R⁴, independently of the others, is halo, alkyl of 1 to 4 carbon atoms, or alkoxy of 1 to 4 carbon atoms.

Other specific immunomodulatory compounds are 1-oxo and 1,3-dioxo-2-(2,6-dioxopiperidin-3-yl) isoindolines disclosed in U.S. Patent No. 6,403,613. Representative compounds are of formula: 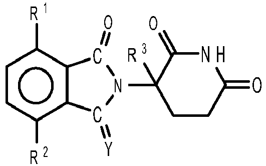 in which
Y is oxygen or H₂,
a first of R¹ and R² is halo, alkyl, alkoxy, alkylamino, dialkylamino, cyano, or carbamoyl, the second of R¹ and R², independently of the first, is hydrogen, halo, alkyl, alkoxy, alkylamino, dialkylamino, cyano, or carbamoyl, and
R³ is hydrogen, alkyl, or benzyl.

Specific examples of the compounds are of formula: 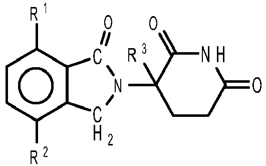 wherein
a first of R¹ and R² is halo, alkyl of from 1 to 4 carbon atoms, alkoxy of from 1 to 4 carbon atoms, dialkylamino in which each alkyl is of from 1 to 4 carbon atoms, cyano, or carbamoyl;
the second of R¹ and R², independently of the first, is hydrogen, halo, alkyl of from 1 to 4 carbon atoms, alkoxy of from 1 to 4 carbon atoms, alkylamino in which alkyl is of from 1 to 4 carbon atoms, dialkylamino in which each alkyl is of from 1 to 4 carbon atoms, cyano, or carbamoyl; and
R³ is hydrogen, alkyl of from 1 to 4 carbon atoms, or benzyl. Specific examples include, but are not limited to, 1-oxo-2-(2,6-dioxopiperidin-3-yl)-4-methylisoindoline.

Other representative compounds are of formula: 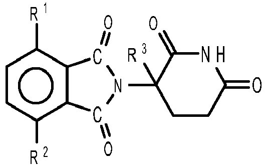 wherein:
a first of R¹ and R² is halo, alkyl of from 1 to 4 carbon atoms, alkoxy of from 1 to 4 carbon atoms, dialkylamino in which each alkyl is of from 1 to 4 carbon atoms, cyano, or carbamoyl;
the second of R¹ and R², independently of the first, is hydrogen, halo, alkyl of from 1 to 4 carbon atoms, alkoxy of from 1 to 4 carbon atoms, alkylamino in which alkyl is of from 1 to 4 carbon atoms, dialkylamino in which each alkyl is of from 1 to 4 carbon atoms, cyano, or carbamoyl; and
R³ is hydrogen, alkyl of from 1 to 4 carbon atoms, or benzyl.

Other specific immunomodulatory compounds disclosed herein are 1-oxo and 1,3-dioxoisoindolines substituted in the 4- or 5-position of the indoline ring described in U.S. Patent No. 6,380,239 and U.S. Patent No. 7,244,759. Representative compounds are of formula: 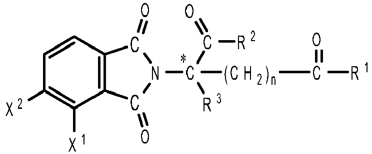 in which the carbon atom designated C* constitutes a center of chirality (when n is not zero and R¹ is not the same as R²); one of X¹ and X² is amino, nitro, alkyl of one to six carbons, or NH-Z, and the other of X¹ or X² is hydrogen; each of R¹ and R² independent of the other, is hydroxy or NH-Z; R³ is hydrogen, alkyl of one to six carbons, halo, or haloalkyl; Z is hydrogen, aryl, alkyl of one to six carbons, formyl, or acyl of one to six carbons; and n has a value of 0, 1, or 2; provided that if X¹ is amino, and n is 1 or 2, then R¹ and R² are not both hydroxy; and the salts thereof.

Further representative compounds are of formula: 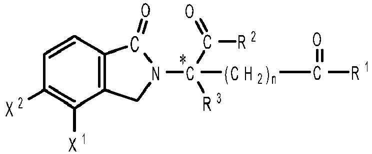 in which the carbon atom designated C* constitutes a center of chirality when n is not zero and R¹ is not R²; one of X¹ and X² is amino, nitro, alkyl of one to six carbons, or NH-Z, and the other of X¹ or X² is hydrogen; each of R¹ and R² independent of the other, is hydroxy or NH-Z; R³ is alkyl of one to six carbons, halo, or hydrogen; Z is hydrogen, aryl or an alkyl or acyl of one to six carbons; and n has a value of 0, 1, or 2.

Specific examples include, but are not limited to, 2-(4-ammo-1-oxo-1,3-dihydro-isoindol-2-yl)-4-carbamoyl-butyric acid and 4-(4-amino-1-oxo-1,3-dihydro-isoindol-2-yl)-4-cabamoyl-butyric acid, which have the following structures, respectively, and pharmaceutically acceptable salts, solvates, prodrugs, and stereoisomers thereof: 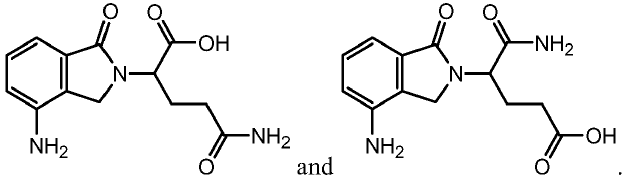 Other representative compounds are of formula: 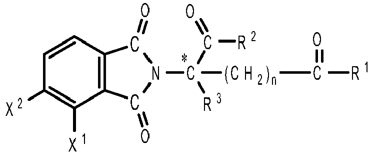 in which the carbon atom designated C* constitutes a center of chirality when n is not zero and R¹ is not R²; one of X¹ and X² is amino, nitro, alkyl of one to six carbons, or NH-Z, and the other of X¹ or X² is hydrogen; each of R¹ and R² independent of the other, is hydroxy or NH-Z; R³ is alkyl of one to six carbons, halo, or hydrogen; Z is hydrogen, aryl, or an alkyl or acyl of one to six carbons; and n has a value of 0, 1, or 2; and the salts thereof.

Specific examples include, but are not limited to, 4-carbamoyl-4-{4-[(furan-2-yl-methyl)-amino]-1,3-dioxo-1,3-dihydro-isoindol-2-yl}-butyric acid, 4-carbamoyl-2- {4-[(furan-2-yl-methyl)-amino]-1,3-dioxo-1,3-dihydro-isoindol-2-yl}-butyric acid, 2- {4-[(furan-2-yl-methyl)-amino]-1,3-dioxo-1,3-dihydro-isoindol-2-yl}-4-phenylcarbamoyl-butyric acid, and 2-{4-[(furan-2-yl-methyl)-amino]-1,3-dioxo-1,3-dihydro-isoindol-2-yl}-pentanedioic acid, which have the following structures, respectively, and pharmaceutically acceptable salts, solvate, prodrugs, and stereoisomers thereof:

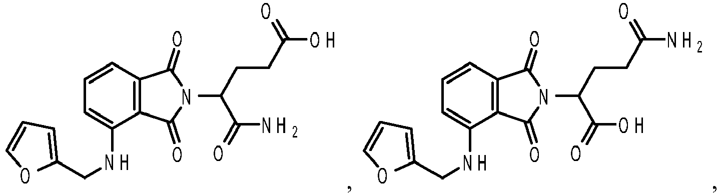

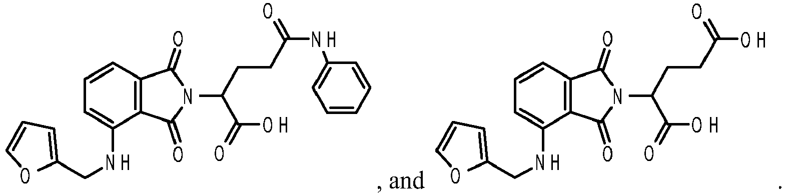

Other specific examples of the compounds are of formula: 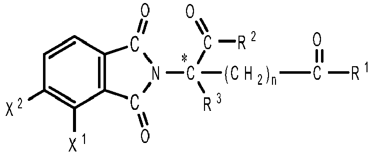 wherein:
one of X¹ and X² is nitro, or NH-Z, and the other of X¹ or X² is hydrogen;
each of R¹ and R², independent of the other, is hydroxy or NH-Z;
R³ is alkyl of one to six carbons, halo, or hydrogen;
Z is hydrogen, phenyl, an acyl of one to six carbons, or an alkyl of one to six carbons; and
n has a value of 0, 1, or 2; and
if -COR² and -(CH₂)*ₙ*COR¹ are different, the carbon atom designated C* constitutes a center of chirality.

Other representative compounds are of formula: 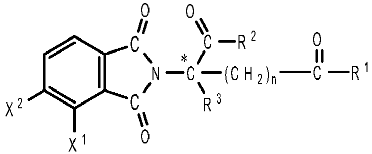 wherein:
one of X¹ and X² is alkyl of one to six carbons;
each of R¹ and R², independent of the other, is hydroxy or NH-Z;
R³ is alkyl of one to six carbons, halo, or hydrogen;
Z is hydrogen, phenyl, an acyl of one to six carbons, or an alkyl of one to six carbons; and
n has a value of 0, 1, or 2; and
if -COR² and -(CH₂)*ₙ*COR¹ are different, the carbon atom designated C* constitutes a center of chirality.

Still other specific immunomodulatory compounds are isoindoline-1-one and isoindoline-1,3-dione substituted in the 2-position with 2,6-dioxo-3-hydroxypiperidin-5-yl described in U.S. Patent No. 6,458,810. Representative compounds are of formula: 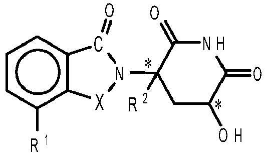 wherein:
the carbon atoms designated * constitute centers of chirality;
X is -C(O)- or -CH₂-;
R¹ is alkyl of 1 to 8 carbon atoms or -NHR³;
R² is hydrogen, alkyl of 1 to 8 carbon atoms, or halogen; and
R³ is hydrogen,
alkyl of 1 to 8 carbon atoms, unsubstituted or substituted with alkoxy of 1 to 8 carbon atoms, halo, amino, or alkylamino of 1 to 4 carbon atoms,
cycloalkyl of 3 to 18 carbon atoms,
phenyl, unsubstituted or substituted with alkyl of 1 to 8 carbon atoms, alkoxy of 1 to 8 carbon atoms, halo, amino, or alkylamino of 1 to 4 carbon atoms,
benzyl, unsubstituted or substituted with alkyl of 1 to 8 carbon atoms, alkoxy of 1 to 8 carbon atoms, halo, amino, or alkylamino of 1 to 4 carbon atoms, or -COR⁴ in which
R⁴ is hydrogen,
alkyl of 1 to 8 carbon atoms, unsubstituted or substituted with alkoxy of 1 to 8 carbon atoms, halo, amino, or alkylamino of 1 to 4 carbon atoms,
cycloalkyl of 3 to 18 carbon atoms,
phenyl, unsubstituted or substituted with alkyl of 1 to 8 carbon atoms, alkoxy of 1 to 8 carbon atoms, halo, amino, or alkylamino of 1 to 4 carbon atoms, or
benzyl, unsubstituted or substituted with alkyl of 1 to 8 carbon atoms, alkoxy of 1 to 8 carbon atoms, halo, amino, or alkylamino of 1 to 4 carbon atoms.

All of the compounds described can either be commercially purchased or prepared according to the methods described in the patents or patent publications disclosed herein. Further, optically pure compounds can be asymmetrically synthesized or resolved using known resolving agents or chiral columns as well as other standard synthetic organic chemistry techniques. Additional information on immunomodulatory compounds, their preparation, and use can be found, for example, in U.S. Patent Application Publication Nos. 20060188475 U.S Patent No. 8,394,832), 20060205787, and 20070049618.

The compounds may be small organic molecules having a molecular weight less than about 1,000 g/mol, and are not proteins, peptides, oligonucleotides, oligosaccharides or other macromolecules.

It should be noted that if there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

Immunomodulatory compounds can either be commercially purchased or prepared according to the methods described in the patents or patent publications referred to herein. Further, optically pure compositions can be asymmetrically synthesized or resolved using known resolving agents or chiral columns as well as other standard synthetic organic chemistry techniques. Immunomodulatory compounds may be racemic, stereomerically enriched or stereomerically pure, and may encompass pharmaceutically acceptable salts, solvates, and prodrugs thereof.

### 5.10. Administration of PINK Cells, Human Placental Perfusate, or Combined Natural Killer Cells and an Immunomodulatory Compound

The methods of tumor suppression, treatment of individuals having cancer (*e.g.,* a blood cancer or solid tumor), and treatment of individuals having a viral infection, provided herein, comprise contacting the tumor cells, or administering to said individual, natural killer cells comprising CD56+ CD16- CD3- PINK cells, combined natural killer cells, or combinations thereof. In certain embodiments, the methods of tumor suppression and treatment additionally comprise contacting the tumor cells, or administering to the individual an immunomodulatory compound or thalidomide.

### 5.10.1. Pretreatment of Natural Killer Cells with Immunomodulatory Compounds or Thalidomide

In one embodiment, natural killer cells comprising CD56+ CD16- CD3- PINK cells, are contacted with an immunomodulatory compound or thalidomide prior to administration of cells in connection with the methods presented herein. For example, such cells can be contacted with an immunomodulatory compound or thalidomide during expansion of the natural killer cells. In certain embodiments, proliferation and/or cytotoxicity of the contacted natural killer cells is detectably enhanced as compared to natural killer cells not contacted with the immunomodulatory compound or thalidomide. Natural killer cells can be contacted with the immunomodulatory compound or thalidomide at a concentration of about 0.1 µM to about 100 µM.

Isolated natural killer cells comprising CD56+ CD16- CD3- PINK cells or combined natural killer cells, as described elsewhere herein, can be treated with an immunomodulatory compound or thalidomide, *e.g.,* contacted with an immunomodulatory compound or thalidomide, to enhance the anticancer or antitumor activity of the cell, or to enhance the activity of the cell against viral infection, *e.g.,* against cells infected with virus. Thus, provided herein is a method of increasing the cytotoxicity of a natural killer cell to a tumor cell comprising contacting the natural killer cell with an immunomodulatory compound or thalidomide for a time and in a concentration sufficient for the natural killer cell to demonstrate increased cytotoxicity towards a tumor cell compared to a natural killer cell not contacted with the immunomodulatory compound or thalidomide. In another embodiment, provided herein is a method of increasing the expression of granzyme B or perforin in a natural killer cell comprising contacting the natural killer cell with an immunomodulatory compound or thalidomide for a time and in a concentration sufficient for the natural killer cell to demonstrate increased expression of granzyme B or perforin compared to a natural killer cell not contacted with the immunomodulatory compound or thalidomide. The immunomodulatory compound can be any compound described in Section 5.9, above, *e.g.,* lenalidomide or pomalidomide.

In specific embodiments of the above, the natural killer cells comprise CD56⁺, CD16⁻, CD3-placental intermediate natural killer cells (PINK cells). In another specific embodiment of the above embodiments, the natural killer cells are combined natural killer cells, *i.e.,* natural killer cells from matched placental perfusate and umbilical cord blood.

In another specific embodiment, said plurality of natural killer cells, *e.g.,* PINK cells or combined natural killer cells, contacted with said immunomodulatory compound or thalidomide express one or more of BAX, CCL5, CCR5, CSF2, FAS, GUSB, IL2RA, or TNFRSF18 at a higher level than an equivalent number of said natural killer cells not contacted with said immunomodulatory compound or thalidomide. In another specific embodiment, said plurality of natural killer cells, *e.g.,* PINK cells, contacted with said immunomodulatory compound or thalidomide express one or more of ACTB, BAX, CCL2, CCL3, CCL5, CCR5, CSF1, CSF2, ECE1, FAS, GNLY, GUSB, GZMB, ILIA, IL2RA, IL8, IL10, LTA, PRF1, PTGS2, SKI, and TBX21 at a higher level than an equivalent number of said natural killer cells not contacted with said immunomodulatory compound or thalidomide.

Also provided herein is a method of increasing the cytotoxicity of a population of human placental perfusate cells, *e.g.,* total nucleated cells from placental perfusate, towards a plurality of tumor cells, comprising contacting the placental perfusate cells with an immunomodulatory compound or thalidomide for a time and in a concentration sufficient for the placental perfusate cells to demonstrate detectably increased cytotoxicity towards said plurality of tumor cells compared to an equivalent number of placental perfusate cells not contacted with the immunomodulatory compound or thalidomide. In another embodiment, provided herein is a method of increasing the expression of granzyme B in a population of placental perfusate cells comprising contacting the population of placental perfusate cells with an immunomodulatory compound or thalidomide for a time and in a concentration sufficient for the population of placental perfusate cells to express a detectably increased amount of granzyme B compared to an equivalent number of placental perfusate cells not contacted with the immunomodulatory compound or thalidomide.

### 5.10.2. Administration of Cells and Immunomodulatory Compounds or Thalidomide

Cells and immunomodulatory compounds, as described above, or thalidomide, can be administered to an individual having cancer, *e.g.,* a person having tumor cells, for example, a person with a blood cancer or a solid tumor; or an individual having a viral infection, for the treatment of said blood cancer, solid tumor, or viral infection. In certain embodiments, the cells have not been contacted with an immunomodulatory compound prior to use, *e.g.,* prior to administration to said individual in combination with an immunomodulatory compound or thalidomide. In certain other embodiments, the cells have been contacted with an immunomodulatory compound prior to use, *e.g.,* prior to administration to said individual in combination with an immunomodulatory compound or thalidomide.

In one embodiment, natural killer cells comprising CD56+ CD16- CD3- PINK cells, and an immunomodulatory compound or thalidomide are combined, *e.g.,* after natural killer cell expansion, or during formulation of the natural killer cells for administration to an individual having cancer or a viral infection. In another embodiment, natural killer cells comprising said PINK cells, and an immunomodulatory compound or thalidomide are combined immediately prior to administration to an individual having cancer or a viral infection, for example, at a point-of-care facility at which the individual receives treatment. In another embodiment, natural killer cells comprising said PINK cells, and an immunomodulatory compound or thalidomide are administered separately to an individual having cancer or a viral infection, *e.g.,* in different formulations. In a specific embodiment, the natural killer cells can be administered to the individual prior to administration of the immunomodulatory compound or thalidomide. In another specific embodiment, the immunomodulatory compound or thalidomide is administered to the individual after administration of the natural killer cells to the individual. In another specific embodiment, the immunomodulatory compound or thalidomide and natural killer cells are administered at the same time or at approximately the same time.

In a specific embodiment, the methods of treatment of an individual having tumor cells, a blood cancer or a solid tumor, *e.g.,* an individual having cancer, or an individual having a viral infection, comprising administration of said natural killer cells, and optionally an immunomodulatory compound or thalidomide, further comprises administration to the individual of an immunosuppressive compound, *e.g.,* cyclosporine; FK506; 2-acetyl-4(5)-(1,2,3,4-tetrahydroxybutyl)imidazole (THI); ciamexone; or the like. In certain embodiments, the immunosuppressive compound is or comprises adherent placental cells (for example, the adherent placental stem or multipotent cells disclosed in U.S. Patent No. 7,468,276 and U.S. Patent Application Publication No. 2007/0275362, (U.S. Patent No. 8,057,788) or mesenchymal stem cells, *e.g.,* bone marrow-derived mesenchymal stem cells.

The cells, *e.g.,* natural killer cells, for example, PINK cells; human placental perfusate cells; combined natural killer cells; populations of cells comprising such cells; or combinations thereof, and optionally immunomodulatory compound or thalidomide, may be administered to an individual prophylactically. For example, the cells, and optionally immunomodulatory compound or thalidomide, as described above, can be administered to an individual at risk of developing metastatic cancer, for example, an individual having breast cancer, prostate cancer, multiple myeloma, or other type of cancer that tends to metastasize, *e.g.,* to bone or brain tissue. Evidence of metastasis, or lack thereof, is not a necessary prerequisite for prophylactic use of immunomodulatory compounds or thalidomide and the cells.

The cells, *e.g.,* natural killer cells, for example, PINK cells; human placental perfusate cells; combined natural killer cells; populations of cells comprising such cells; or combinations thereof, and optionally immunomodulatory compound or thalidomide, may be administered to an individual, *e.g.,* an individual having tumor cells or an individual having a blood cancer or a solid tumor, *e.g.,* a cancer patient, or an individual having a viral infection, by any medically-acceptable route known in the art suitable to the administration of live cells. In various embodiments, the isolated natural killer cells comprising CD56+ CD16- CD3- PINK cells may be surgically implanted, injected, infused, *e.g.,* by way of a catheter or syringe, or otherwise administered directly or indirectly to the site in need of repair or augmentation. In one embodiment, the said natural killer cells are administered to said individual; that is, intratumorally. In another embodiment, the said natural killer cells, and optionally immunomodulatory compound or thalidomide, are administered to the individual at the site of a tumor, *e.g.,* any solid tumor, for example, by intra-tumor injection. In specific embodiments, the solid tumor is a bladder cancer tumor or a liver cancer tumor. In a specific embodiment in which the individual has a tumor at more than one site, the cells, and optionally immunomodulatory compound or thalidomide, are administered to at least two, or all, tumor sites. In certain other embodiments, the isolated natural killer cells comprising said PINK cells are administered orally, nasally, intraarterially, parenterally, ophthalmically, intramuscularly, subcutaneously, intraperitoneally, intracerebrally, intraventricularly, intracerebroventricularly, intrathecally, intracisternally, intraspinally and/or perispinally. In certain specific embodiments, the said natural killer cells are delivered via intracranial or intravertebral needles and/or catheters with or without pump devices.

The natural killer cells, *e.g.,* PINK cells, human placental perfusate cells, combined natural killer cells, or combinations thereof, or cell populations comprising such cells, and optionally immunomodulatory compound or thalidomide, can be administered to an individual in a composition, *e.g.,* a matrix, hydrogel, scaffold, or the like that comprise the cells.

In one embodiment, the isolated natural killer cells comprising CD56+ CD16- CD3- PINK cells are seeded onto a natural matrix, *e.g.,* a placental biomaterial such as an amniotic membrane material. Such an amniotic membrane material can be, *e.g.,* amniotic membrane dissected directly from a mammalian placenta; fixed or heat-treated amniotic membrane, substantially dry (*i.e.,* <20% H₂O) amniotic membrane, chorionic membrane, substantially dry chorionic membrane, substantially dry amniotic and chorionic membrane, and the like. Preferred placental biomaterials on which placental stem cells can be seeded are described in Hariri, U.S. Application Publication No. 2004/0048796.

In another embodiment, the natural killer cells comprising CD56+ CD16- CD3- PINK cells, human placental perfusate cells, combined natural killer cells, or combinations thereof, or cell populations comprising such cells, are suspended in a hydrogel solution suitable for, *e.g.,* injection. Suitable hydrogels for such compositions include self-assembling peptides, such as RAD16. In one embodiment, a hydrogel solution comprising the cells can be allowed to harden, for instance in a mold, to form a matrix having cells dispersed therein for implantation. The cells in such a matrix can also be cultured so that the cells are mitotically expanded prior to implantation. The hydrogel can be, for example, an organic polymer (natural or synthetic) that is cross-linked via covalent, ionic, or hydrogen bonds to create a three-dimensional open-lattice structure that entraps water molecules to form a gel. Hydrogel-forming materials include polysaccharides such as alginate and salts thereof, peptides, polyphosphazines, and polyacrylates, which are crosslinked ionically, or block polymers such as polyethylene oxide-polypropylene glycol block copolymers which are crosslinked by temperature or pH, respectively. In some embodiments, the hydrogel or matrix disclosed herein is biodegradable.

In some aspects of the disclosure, the formulation comprises an *in situ* polymerizable gel (*see., e.g.,* U.S. Patent Application Publication 2002/0022676; Anseth et al., J. Control Release, 78(1-3):199-209 (2002); Wang et al., Biomaterials, 24(22):3969-80 (2003).

In some embodiments, the polymers are at least partially soluble in aqueous solutions, such as water, buffered salt solutions, or aqueous alcohol solutions, that have charged side groups, or a monovalent ionic salt thereof. Examples of polymers having acidic side groups that can be reacted with cations are poly(phosphazenes), poly(acrylic acids), poly(methacrylic acids), copolymers of acrylic acid and methacrylic acid, poly(vinyl acetate), and sulfonated polymers, such as sulfonated polystyrene. Copolymers having acidic side groups formed by reaction of acrylic or methacrylic acid and vinyl ether monomers or polymers can also be used. Examples of acidic groups are carboxylic acid groups, sulfonic acid groups, halogenated (preferably fluorinated) alcohol groups, phenolic OH groups, and acidic OH groups.

The placental stem cells or co-cultures thereof can be seeded onto a three-dimensional framework or scaffold and implanted *in vivo.* Such a framework can be implanted in combination with any one or more growth factors, cells, drugs or other components that stimulate tissue formation or otherwise enhance or improve the practice of the invention.

Examples of scaffolds that can be used in the present invention include nonwoven mats, porous foams, or self assembling peptides. Nonwoven mats can be formed using fibers comprised of a synthetic absorbable copolymer of glycolic and lactic acids (*e.g.,* PGA/PLA) (VICRYL, Ethicon, Inc., Somerville, N.J.). Foams, composed of, *e.g.,* poly(ε-caprolactone)/poly(glycolic acid) (PCL/PGA) copolymer, formed by processes such as freeze-drying, or lyophilization (*see, e.g.,* U.S. Pat. No. 6,355,699), can also be used as scaffolds.

Placental stem cells can also be seeded onto, or contacted with, a physiologically-acceptable ceramic material including, but not limited to, mono-, di-, tri-, alpha-tri-, beta-tri-, and tetra-calcium phosphate, hydroxyapatite, fluoroapatites, calcium sulfates, calcium fluorides, calcium oxides, calcium carbonates, magnesium calcium phosphates, biologically active glasses such as BIOGLASS®, and mixtures thereof. Porous biocompatible ceramic materials currently commercially available include SURGIBONE® (CanMedica Corp., Canada), ENDOBON® (Merck Biomaterial France, France), CEROS® (Mathys, AG, Bettlach, Switzerland), and mineralized collagen bone grafting products such as HEALOS™ (DePuy, Inc., Raynham, MA) and VITOSS®, RHAKOSS™, and CORTOSS® (Orthovita, Malvern, Pa.). The framework can be a mixture, blend or composite of natural and/or synthetic materials.

In another aspect, placental stem cells can be seeded onto, or contacted with, a felt, which can be, *e.g.,* composed of a multifilament yarn made from a bioabsorbable material such as PGA, PLA, PCL copolymers or blends, or hyaluronic acid.

The placental stem cells can, in another embodiment, be seeded onto foam scaffolds that may be composite structures. Such foam scaffolds can be molded into a useful shape, such as that of a portion of a specific structure in the body to be repaired, replaced or augmented. In some embodiments, the framework is treated, *e.g.,* with 0.1M acetic acid followed by incubation in polylysine, PBS, and/or collagen, prior to inoculation of the cells of the disclosure in order to enhance cell attachment. External surfaces of a matrix may be modified to improve the attachment or growth of cells and differentiation of tissue, such as by plasma-coating the matrix, or addition of one or more proteins (*e.g.,* collagens, elastic fibers, reticular fibers), glycoproteins, glycosaminoglycans (*e.g.,* heparin sulfate, chondroitin-4-sulfate, chondroitin-6-sulfate, dermatan sulfate, keratin sulfate, *etc*.), a cellular matrix, and/or other materials such as, but not limited to, gelatin, alginates, agar, agarose, and plant gums, and the like.

In some embodiments, the scaffold comprises, or is treated with, materials that render it non-thrombogenic. These treatments and materials may also promote and sustain endothelial growth, migration, and extracellular matrix deposition. Examples of these materials and treatments include but are not limited to natural materials such as basement membrane proteins such as laminin and Type IV collagen, synthetic materials such as EPTFE, and segmented polyurethaneurea silicones, such as PURSPAN™ (The Polymer Technology Group, Inc., Berkeley, Calif.). The scaffold can also comprise anti-thrombotic agents such as heparin; the scaffolds can also be treated to alter the surface charge (*e.g.,* coating with plasma) prior to seeding with placental stem cells.

### 5.10.3. Administration of Immunomodulatory Compounds or Thalidomide

In certain embodiments in which immunomodulatory compounds or thalidomide are administered to an individual having tumor cells, *e.g.,* cancer, for example, a blood cancer or a solid tumor; or an individual having a viral infection, the immunomodulatory compound or thalidomide can be, as noted above, administered separately, and thus formulated separately, from the cells.

Any route of administration may be used. For example, an immunomodulatory compound or thalidomide can be administered by oral, parenteral, intravenous, transdermal, intramuscular, rectal, sublingual, mucosal, nasal, or other means. In addition, an immunomodulatory compound or thalidomide can be administered in a form of pharmaceutical composition and/or unit dosage form. Suitable dosage forms include, but are not limited to, capsules, tablets (including rapid dissolving and delayed release tablets), powder, syrups, oral suspensions and solutions for parenteral administration. Suitable administration methods for immunomodulatory compounds or thalidomide, as well as suitable dosage forms and pharmaceutical compositions, can be found in U.S. Patent Application Publication Nos. 20060188475 (U.S. Patent No. 8,394,832), 20060205787, and 20070049618.

Typical dosage forms comprise an immunomodulatory compound or thalidomide, or a pharmaceutically acceptable salt, solvate, stereoisomer, or prodrug thereof, in an amount of from about 0.001 to about 150 mg. In particular, dosage forms comprise an immunomodulatory compound or thalidomide, or a pharmaceutically acceptable salt, solvate, stereoisomer, or prodrug thereof in an amount of about 0.001, 0.01, 0.1, 1, 2, 5, 7.5, 10, 12.5, 15, 17.5, 20, 25, 50, 100, 150 or 200 mg. In a particular embodiment, a dosage form comprises 4-(amino)-2-(2,6-dioxo(3-piperidyl))-isoindoline-1,3-dione in an amount of about 0.001, 0.01, 0.1, 1, 2, 5, 10, 25 or 50 mg.

Pharmaceutical compositions comprising an immunomodulatory compound or thalidomide can also contain one of more pharmaceutically acceptable excipients. *See, e.g.,* Rowe et al., Handbook of Pharmaceutical Excipients, 4th Ed. (2003).

In a specific embodiment, a dosage form comprises 3-(4-amino-1-oxo-1,3-dihydro-isoindol-2-yl)-piperidine-2,6-dione in an amount of about 0.001, 0.01, 0.1, 1, 5, 10, 25 or 50 mg. Typical dosage forms comprise the second active ingredient in an amount of 1 µg to about 1000 mg, from about 0.01 to about 500 mg, from about 0.1 to about 350 mg, or from about 1 to about 200 mg. This invention also encompasses the use of racemic mixture, (S)-isomer, and (R)-isomer of 3-(4-amino-1-oxo-1,3-dihydro-isoindol-2-yl)-piperidine-2,6-dione. Typically, racemic 3-(4-amino-1-oxo-1,3-dihydro-isoindol-2-yl)-piperidine-2,6-dione can be administered at an amount of 1, 5, 10, 15, 25, or 50 mg per day. Optical isomers also can be administered at an amount comparable to racemic mixture. Doses can be adjusted depending on the type of disease or disorder being treated, prevented or managed, and the amount of an immunomodulatory compound or thalidomide and any optional additional agents concurrently administered to the patient, which are all within the skill of the art.

### 6. EXAMPLES

### 6.1. Example 1: Characterization of Placenta-Derived Intermediate Natural Killer Cells from Placental Perfusate and Umbilical Cord Blood

The present example demonstrates the isolation and culture of natural killer cells from human placental perfusate.

*Isolation of placental natural killer cells.* Natural killer cells were isolated from 8 units of human placental perfusate (HPP), and from 4 units of umbilical cord blood (UCB), using CD56-conjugated microbeads. Isolation of PINK cells was conducted by magnetic bead selection (Miltenyi Biotec). The post partum placenta was exsanguinated and perfused with about 200 to about 750 mL of perfusion solution (0.9% NaCl injection solution USP Grade (Cat No. 68200-804, VWR). The unprocessed perfusate was collected and processed to remove erythrocytes. Mononuclear cells from HPP or UCB were washed one time with fluorescence-activated cell sorting (FACS) buffer (RPMI 1640, without phenol red, plus 5% FBS), then centrifuged at 1500 rpm for 6 minutes. The cell number was counted, and the cell pellet was resuspended in 80 µL of buffer per 10⁷ total cells with 20 µL of CD3 Microbeads (Catalog No. 130-050-101, Miltenyi). The system was mixed well and incubated for 15 minutes at 4-8°C. 1-2 mL of buffer per 10⁷ total cells was added, and the mixture was then centrifuged at 300g for 10 minutes. The supernatant was pipetted off completely. The cell pellet was resuspended up to 10⁸ cells in 500 µL of buffer and prepared for magnetic separation. An LS column (Miltenyi Biotec) was placed in the magnetic field of a MIDIMACS™ cell separator (Miltenyi Biotec), 3 mL buffer was applied to rinse the column, and the cell/microbead suspension was applied to the column. Unlabeled CD3⁻ cells, which passed through the column and which would include natural killer cells, were collected, together with 2 x 3 mL washing buffer. The CD3⁻ cells were counted, washed one time, then were stained with CD56 MicroBeads (Cat#: 130-050-401, Miltenyi), and separated/isolated using the same protocol as for the CD3 microbead separation described above. A CD56+CD3- population was thus collected and ready for further analysis. The percentage range of natural killer cells was 3.52 to 11.6 (median 6.04, average 5.22) in HPP, and 1.06 to 8.44 in UCB (median: 3.42, average: 4.2). CD56 microbead selection of natural killer cells from HPP produced a population that was approximately 80% pure. *See* FIG. 1. Among the whole CD56⁺, CD3⁻ natural killer cell population, the percentage range of CD56⁺, CD16⁻ natural killer cells (that is, PINK cells) was 56.6 to 87.2 (median 74.2, average 65.5) from HPP, and 53.7 to 96.6 (median 72.8) from UCB. The percentage range of CD56⁺, CD16⁺ natural killer cells was 12.8 to 43.3 (median 25.8, average 34.5) from HPP, and 3.4 to 46.3 (median 27.3, average 33.4) for UCB.

In other experiments, natural killer cells were isolated using a magnetic negative selection kit that targets cell surface antigens on human blood cells (CD3, CD4, CD14, CD19, CD20, CD36, CD66b, CD123, HLA-DR, glycophorin A). HPP and UCB cryopreserved units were thawed and diluted at 1:1 with Thaw media (RPMI Media 1640 (Catalog #22400, Gibco) plus 20% Fetal Bovine Serum-Heat Inactivated (Catalog #SH30070.03, Hyclone)) and centrifuged at 1500 rpm for 8 minutes. The supernatant was removed and ammonium chloride treatment was applied to further deplete erythrocytes; each unit was resuspended in approximately 30 mL of ice cold FACS buffer (RPMI 1640, without phenol red, plus 5% FBS), and then 60 mL ice cold ammonium chloride (Catalog #07850, Stem Cell) was added, the solution was vortexed and then incubated on ice for 5 minutes. The mononuclear cells were then washed with FACS buffer 3 times and then centrifuged at 1500 rpm for 8 minutes. The cell number was counted and the cell pellet was resuspended in 5x10⁷ live cells/ml in RoboSep Buffer (Catalog #20104, Stem Cell) plus 0.1 mg/mL DNAase I solution (Catalog #07900, Stem Cell) was added to the cell suspension, mixed gently by pipette and incubated 15 minutes at room temperature prior to isolation. Clumps were removed from the cell suspension by filtering with 40µm mesh nylon strainer (Catalog #352340, BD Falcon) before proceeding to isolation. Isolation is automated by the device RoboSep (Catalog #20000, Stem Cell) and the program "Human NK Negative Selection 19055 and high recovery" (50µl/ml cocktail addition, 100 µL/mL Microparticle addition, 10 and 5 minute incubations, 1x 2.5 minute separations) with Human NK Cell Enrichment Kit (Catalog #19055, Stem Cell) including EasySep Negative Selection Human NK Cell Enrichment Cocktail and EasySep Magnetic Microparticles. A CD56⁺CD3⁻ population was thus collected and ready for further analysis.

*Expansion of Natural Killer Cells.* In general, natural killer cells were expanded as follows. Start Medium for natural killer cell culture was prepared based on a modification of a protocol described in Yssel et al., J. Immunol. Methods 72(1):219-227 (1984) and Litwin et al., J. Exp. Med. 178(4):1321-1326 (1993). Briefly, Start Medium includes IMDM (Invitrogen) with 10% FCS (Hyclone), containing the following reagents with final concentration of 35 µg/mL transferrin (Sigma-Aldrich), 5 µg/mL insulin (Sigma-Aldrich), 2 x 10⁻⁵M ethanolamine (Sigma-Aldrich), 1 µg/mL oleic acid (Sigma-Aldrich), 1 µg/mL linoleic acid (Sigma-Aldrich), 0.2 µg/mL palmitic acid (Sigma-Aldrich), 2.5 µg/mL BSA (Sigma-Aldrich) and 0.1 µg/mL Phytohemagglutinin (PHA-P, Sigma-Aldrich). CD56⁺CD3⁻NK cells were resuspended at 2.5x10⁵ live cells/mL Start Medium plus 200 iu/mL IL-2 (R&D Systems) in cell culture treated 24-well plate or T flask. Mitomycin C-treated allogeneic PBMC and K562 cells (chronic myelogenous leukemia cell line) were both added to the Start Medium as feeder cells, to a final concentration of 1 x 10⁶ per mL. NK cells were cultured for 5-6 days at 37°C in 5% CO₂. After 5-6 days and then every 3-4 days an equal volume of Maintenance Medium (IMDM with 10% FCS, 2% Human AB serum, antibiotics, L-glutamine and 400 units of IL-2 per mL) was added to the culture. NK cells were harvested at day 21.

*Characterization of Placenta-Derived Intermediate Natural Killer Cells.* Donor matched HPP and CB was thawed, and the cells were washed with FACS buffer (RPMI-1640 with 5% FBS). Natural killer cells were then enriched with CD56 microbeads using the ROBOSEP® magnetic separation system (StemCell Technologies) as instructed by the manufacturer. The CD56 enriched natural killer cell population was stained with the following antibodies (BD Bioscience if not otherwise indicated) for immunophenotypic characterization: anti-CD56 conjugated to PE-Cy-7, anti-CD3 APC Cy7, anti-CD 16 FITC, anti-NKG2D APC, anti-NKp46 APC, anti-CD94 PE(R&D), anti-NKBl PE, and anti-KIR-NKAT2 PE. CD94, NKG2D and NKp46 are markers absent, or showing reduced expression, in NK cell progenitors but present on fully-differentiated NK cells. *See* Freud et al., "Evidence for Discrete States of Human Natural Killer Cell Differentiation In Vivo," J. Exp. Med. 203(4):1033-1043 (2006); Eagle & Trowsdale, "Promiscuity and the Single Receptor: NKG2D," Nature Reviews Immunology Published online August 3, 2007; Walzer et al., "Natural Killer Cells: From CD3-NKp46+ to Post-Genomics Meta-Analyses," Curr. Opinion Immunol. 19:365-372 (2007). As shown in Table 1, expression of KIR3DL1, KIR2DL2/L3, NKG2D, NKp46 and CD94 was not significantly different between an enriched CD56⁺ cell population from HPP and an HLA-matched CD56⁺ cell population from umbilical cord blood (CB).

**Table 1. Percentage of NK cells bearing certain marker combinations. Mean of 3 samples.**

| | **Mean (%)** | | |
|---|---|---|---|
| | **CB** | **HPP** | **p value** |
| CD3-CD56+ | 0.6 | 0.7 | 0.799 |
| CD3-CD56+CD16- | 53.9 | 58.7 | 0.544 |
| CD3-CD56+CD16+ | 46.1 | 41.3 | 0.544 |
| CD3-CD56+KIR3DL1+ | 5.8 | 7.3 | 0.762 |
| CD3-CD56+KIR2DL2/L3+ | 10.7 | 9.9 | 0.89 |
| CD3-CD56+NKG2D+ | 60.3 | 58.5 | 0.865 |
| CD3-CD56+CD94+ | 74.6 | 76.8 | 0.839 |

### 6.2. Example 2: Characterization of Placenta-Derived Intermediate Natural Killer Cells From Combined Placental Perfusate and Umbilical Cord Blood

Donor matched mononucleated cells of umbilical cord blood and placental perfusate (combo) were mixed and washed with FACS buffer (RPMI-1640 with 5% FBS) once and immunophenotypically characterized using the antibodies listed in Table 2 on a BD FACSCanto (BD Biosciences). The data were analyzed by FlowJo software (Tree Star).

**Table 2: List of antibodies used in immunophenotypic characterization.**

| **Item** | **vendor** | **Cat No.** |
|---|---|---|
| FITC anti-hu CD3 | BD Bioscience | 555332 |
| FITC anti-hu CD3 | Miltenyi | 130-080-401 |
| APC-Cy7 anti-hu CD3 | BD Bioscience | 557832 |
| FITC anti-hu CD16 | BD Bioscience | 555406 |
| PE-Cy5 anti-hu CD16 | BD Bioscience | 555408 |
| PE anti-hu CD56 | BD Bioscience | 555516 |
| PE anti-hu CD56 | Miltenyi | 130-090-755 |
| PE-CY5 anti-hu CD56 | BD Bioscience | 555517 |
| PE-Cy7 anti-hu CD56 | BD Bioscience | 557747 |
| PE anti-hu CD94 | R&D | FAB-1058P |
| PE anti-hu KIR-NKAT2 (2DL2/L3) | BD Bioscience | 556071 |
| PE anit-hu NKB1 (3DL1) | BD Bioscience | 555967 |
| APC anit-hu NKG2D | BD Bioscience | 558071 |
| APC anit-hu NKp46 | BD Bioscience | 558051 |
| PE anti-hu CD226 | BD Bioscience | 559789 |
| PE anit-hu NKp44 | BD Bioscience | 558563 |
| PE anti-hu NKp30 | BD Bioscience | 558407 |
| PE anti-hu 2B4 | BD Bioscience | 550816 |
| Isotype FITC mouse IgG1 | BD Bioscience | 340755 |
| Isotype FITC mouse IgG2b | BD Bioscience | 556577 |
| Isotype PE mouse IgG1 | BD Bioscience | 340761 |
| Isotype PE mouse IgG2b | BD Bioscience | 555743 |
| Isotype PerCP mouse IgG1 | BD Bioscience | 340762 |
| Isotype PE-Cy5 mouse IgG2b | BD Bioscience | 555744 |
| Isotype APC mouse IgG1 | BD Bioscience | 340754 |
| Isotype APC mouse IgG2a | BD Bioscience | 555576 |
| Isotype APC-Cy7 mouse IgG1 | BD Bioscience | 348802 |
| Isotype PE-Cy7 mouse IgG1 | BD Bioscience | 348798 |

*Immunophenotypic Characterization of Placental NK Cells And Peripheral Blood (PB) NK Cells.* NK cells can be divided into two main groups: CD56⁺CD16⁺ NK cells, and CD56⁺CD16⁻ cells. CD56⁺CD16⁺ NK cells have abundant cytolytic granules and high expression of CD16, and are therefore capable of eliciting antibody-dependent cell-mediated cytotoxicity (ADCC). CD56⁺CD16⁻ NK cells, conversely, have very few cytolytic granules, low or no expression of CD16, but are capable of producing cytokines and chemokines upon activation. Individual NK cells display a diverse repertoire of activating and inhibitory receptors, including the killer immunoglobulin-like receptors (KIRs, *e.g.,* KIR3DL1, and KIR2DL2/3), natural cytotoxicity receptors NCRs (*e.g.,* NKp30, NKp44, and NKp46), killer cell lectin-like receptors (KLRs; *e.g.,* CD94, NKG2D), 2B4 and CD226.

FACS analysis was performed on placental NK and peripheral blood NK cells using fluorescence-conjugated mAbs against specific NK receptors. Among 11 NK subsets characterized, the numbers of cells in seven out of 11 NK subsets (CD3⁻CD56⁺CD16⁻, CD3⁻ CD56⁺CD16⁺, CD3⁻CD56⁺KIR2DL2/3⁺, CD3⁻CD56⁺NKp46⁺, CD3⁻CD56⁺NKp30⁺, CD3⁻ CD56⁺2B4⁺ and CD3⁻CD56⁺CD94⁺) showed significant difference (p < 0.05) between placental NK and peripheral blood NK cells (accounted for 64% difference) (Table 3A; *see also* Tables 3B and 3C).

**Table 3A. Phenotypic characterization of CD3⁻CD56⁺ NK cells in 16 units of combined donor-matched umbilical cord blood and human placental perfusate (combo) and 13 units of peripheral blood (PB). Mean % for rows comprising CD3, CD56, and a third marker represent the percentage of CD3⁻CD56⁺ cells also expressing the third marker. The two-sample t-test is used to determine if population means are equal in placental and peripheral blood units.**

| | **Combo (16 units)** | **PB (13 units)** | |
|---|---|---|---|
| **Surface markers** | **Mean %** | **Mean %** | **P value** |
| CD3⁻CD56⁺ | 2.2 | 2.4 | 0.728 |
| CD3⁻CD56⁺CD16⁻ | 60.9 | 21.4 | 0.000 |
| CD3⁻CD56⁺CD16⁺ | 30.1 | 78.6 | 0.000 |
| CD3⁻CD56⁺KIR3DL1⁺ | 12.3 | 7.1 | 0.099 |
| CD3⁻CD56⁺KIR2DL2/L3+ | 21.9 | 9.5 | 0.004 |
| CD3⁻CD56⁺NKG2D⁺ | 42.1 | 29.9 | 0.126 |
| CD3⁻CD56⁺NKp46⁺ | 7.0 | 18.9 | 0.011 |
| CD3⁻CD56⁺CD226⁺ | 16.0 | 26.7 | 0.135 |
| CD3⁻CD56⁺NKp44⁺ | 9.5 | 4.9 | 0.073 |
| CD3⁻CD56⁺NKp30⁺ | 39.1 | 19.0 | 0.006 |
| CD3⁻CD56⁺2B4⁺ | 11.1 | 4.5 | 0.019 |
| CD3⁻CD56⁺CD94⁺ | 71.3 | 26.2 | 0.000 |

Tables 3B and 3C show the phenotypic characterization of CD3⁻CD56⁺CD16⁻ and CD3⁻ CD56⁺CD16⁺NK cells in 16 units of combined donor-matched umbilical cord blood and human placental perfusate (combo) and 13 units of peripheral blood (PB) in a separate experiment. Mean % for rows comprising CD3, CD56, CD16 and a fourth marker represent the percentage of CD3⁻CD56⁺CD16⁺ or CD3⁻CD56⁺CD16⁻ cells also expressing the fourth marker.

**Table 3B.**

| | **Combo** | **PB** | |
|---|---|---|---|
| **Surface markers** | **Mean%** | **Mean%** | **P Value** |
| CD3⁻CD56⁺CD16⁻ | 62.3 | 14.1 | 0.000 |
| CD3⁻CD56⁺CD16⁻KIR3DL1⁺ | 7.8 | 1.5 | 0.004 |
| CD3⁻CD56⁺CD16-NKG2D⁺ | 43.5 | 42.7 | 0.941 |
| CD3⁻CD56⁺CD16⁻ KIR2DL2/L3⁺ | 13.6 | 2.4 | 0.000 |
| CD3⁻CD56⁺CD16⁻NKp46⁺ | 6.7 | 43.6 | 0.001 |
| CD3⁻CD56⁺CD16⁻CD94⁺ | 69.8 | 48.5 | 0.057 |
| CD3⁻CD56⁺CD16⁻CD226⁺ | 7.6 | 4.9 | 0.068 |
| CD3⁻CD56⁺CD16⁻NKp44⁺ | 3.4 | 0.6 | 0.076 |
| CD3⁻CD56⁺CD16⁻NKp30⁺ | 46.7 | 22.0 | 0.000 |
| CD3⁻CD56⁺CD16⁻2B4⁺ | 3.7 | 0.5 | 0.078 |

**Table 3C.**

| | **Combo** | **PB** | |
|---|---|---|---|
| **Surface markers** | **Mean%** | **Mean%** | **P Value** |
| CD3⁻CD56⁺CD16⁺ | 37.7 | 85.9 | 0.000 |
| CD3-CD56⁺CD16⁺KIR3DL1⁺ | 21.5 | 8.9 | 0.014 |
| CD3⁻CD56⁺CD16⁺NKG2D⁺ | 42.1 | 28.5 | 0.066 |
| CD3⁻CD56⁺CD16⁺KIR2DL2/L3⁺ | 34.5 | 12.1 | 0.000 |
| CD3⁻CD56⁺CD16⁺NKp46⁺ | 10.4 | 14.5 | 0.242 |
| CD3⁻CD56⁺CD16⁺CD94⁺ | 72.9 | 23.8 | 0.000 |
| CD3⁻CD56⁺CD16⁺CD226⁺ | 35.5 | 32.6 | 0.347 |
| CD3⁻CD56⁺CD16⁺NKp44⁺ | 22.6 | 6.4 | 0.016 |
| CD3⁻CD56⁺CD16⁺NKp30⁺ | 45.7 | 19.7 | 0.000 |
| CD3⁻CD56⁺CD16⁺2B4⁺ | 31.2 | 6.1 | 0.008 |

60.9% of placental NK cells are CD56⁺CD16⁻ (placenta-derived intermediate natural killer (PINK) cells) while only 21.4% of peripheral blood NK cells are CD56⁺CD16⁻. After cultivation for 21 days, the percentage of four out 11 NK subsets (CD3⁻CD56⁺KIR2DL2/3⁺, CD3⁻CD56⁺NKp46⁺, CD3⁻CD56⁺NKp44⁺ and CD3⁻CD56⁺NKp30⁺) showed significant difference (p < 0.05) between placental and peripheral blood NK cells (Table 4).

**Table 4. Phenotypic characterization of day 21-cultured NK cells derived from 12 units of combined donor-matched umbilical cord blood and human placental perfusate (Combo), and 9 units of peripheral blood (PB). The two-sample t-test is used to determine if population means are equal in combo and peripheral blood units.**

| | **Combo (16 units)** | **PB (13 units)** | |
|---|---|---|---|
| **Surface markers** | **Mean%** | **Mean%** | **P Value** |
| **CD3-CD56+** | **22** | **2.4** | **0.728** |
| **CD3-CD56+CD16-** | **60.9** | **21.4** | **0.000** |
| **CD3-CD56+CD16+** | **39.1** | **78.6** | **0.000** |
| **CD3CD56+KIRDL1** | **12.3** | **7.1** | **0.099** |
| **CD3-CD56+KIR2DL2/L3** | **21.9** | **9.5** | **0.004** |
| **CD3-CD56+NKG72D** | **42.1** | **29.9** | **0.126** |
| **CD3-CD56+NIKp46** | **7.0** | **18.9** | **0.011** |
| **CD3-CD56+CD226** | **16.0** | **26.7** | **0.135** |
| **CD3-CD56+NIKp44** | **9.5** | **4.9** | **0.073** |
| **CD3-CD56+NIKp30** | **39.1** | **19.0** | **0.006** |
| **CD3-CD56+284** | **11.1** | **4.5** | **0.019** |
| **CD3-CD56+CD94** | **71.3** | **26.2** | **0.000** |

In addition, in a separate experiment, it was determined that, after cultivation for 21 days, placental and peripheral blood NK cells demonstrated unique cytokine profiles, particularly for IL-8, as determined by Luminex assay (Table 5).

**Table 5**

| **Cytokine** | **PB (pg/mL)** | **Combo (pg/mL)** |
|---|---|---|
| **IL-13** | 1.26 | 1.89 |
| **IL-8** | 6.61 | 15.77 |
| **IL-10** | 1.26 | 2.23 |
| **TNFa** | 0.28 | 0.34 |
| **MCP-1** | 10.49 | 11.32 |

*MicroRNA Profiling of Placental NK Cells And Peripheral Blood NK Cells.* Isolated or expanded NK cells were subjected to microRNA (miRNA) preparation using a MIRVANA™ miRNA Isolation Kit (Ambion, Cat# 1560). NK cells (0.5 to 1.5 x 10⁶ cells) were disrupted in a denaturing lysis buffer. Next, samples were subjected to acid-phenol+chloroform extraction to isolate RNA highly enriched for small RNA species. 100% ethanol was added to bring the samples to 25% ethanol. When this lysate/ethanol mixture was passed through a glass fiber filter, large RNAs were immobilized, and the small RNA species were collected in the filtrate. The ethanol concentration of the filtrate was then increased to 55%, and the mixture was passed through a second glass fiber filter where the small RNAs became immobilized. This RNA was washed a few times, and eluted in a low ionic strength solution. The concentration and purity of the recovered small RNA was determined by measuring its absorbance at 260 and 280 nm.

miRNAs found to be unique for PINK cells are shown in Table 6. One miRNA, designated hsa-miR-199b, was found to be unique for peripheral blood NK cells.

**Table 6. miRNA profiling for PINK cells and PB NK cells via qRT-PCR.**

| **miRNA ID** | **Sanger Accession No.** | **Sequence** | **SEQ ID NO** |
|---|---|---|---|
| hsa-miR-100 | MIMAT0000098 | aacccguagauccgaacuugug | 1 |
| hsa-miR-127 | MIMAT0000446 | ucggauccgucugagcuuggcu | 2 |
| hsa-miR-211 | MIMAT0000268 | uucccuuugucauccuucgccu | 3 |
| hsa-miR-302c | MIMAT0000717 | uaagugcuuccauguuucagugg | 4 |
| hsa-miR-326 | MIMAT0000756 | ccucugggcccuuccuccag | 5 |
| hsa-miR-337 | MIMAT0000754 | uccagcuccuauaugaugccuuu | 6 |
| hsa-miR-497 | MIMAT0002820 | cagcagcacacugugguuugu | 7 |
| hsa-miR-512-3p | MIMA T0002823 | aagugcugucauagcugagguc | 8 |
| hsa-miR-515-5p | MIMAT0002826 | uucuccaaaagaaagcacuuucug | 9 |
| hsa-miR-517b | MIMAT0002857 | ucgugcaucccuuuagaguguu | 10 |
| hsa-miR-517c | MIMAT0002866 | aucgugcauccuuuuagagugu | 11 |
| hsa-miR-518a | MIMAT0002863 | aaagcgcuucccuuugcugga | 12 |
| hsa-miR-518e | MIMAT0002861 | aaagcgcuucccuucagagug | 13 |
| hsa-miR-519d | MIMAT0002853 | caaagugccucccuuuagagug | 14 |
| hsa-miR-520g | MIMAT0002858 | acaaagugcuucccuuuagagugu | 15 |
| hsa-miR-520h | MIMAT0002867 | acaaagugcuucccuuuagagu | 16 |
| hsa-miR-564 | MIMAT0003228 | aggcacggugucagcaggc | 17 |
| hsa-miR-566 | MIMAT0003230 | gggcgccugugaucccaac | 18 |
| hsa-miR-618 | MIMAT0003287 | aaacucuacuuguccuucugagu | 19 |
| hsa-miR-99a | MIMAT0000097 | aacccguagauccgaucuugug | 20 |

*Immunophenotypic Characterization of Cultured Placental NK Cells and Uncultured NK Cells.* The overall properties of cultured PINK cells were evaluated by extensive immunophenotypic studies and cytotoxicity assays. To determine the phenotype of expanded NK cells, expression of NK receptors (NKRs) such as KIRs, NKG2D, NKp46, NKp44 and 2B4 were analyzed. Cytotoxicity assays were performed by labeling tumor cells (K562 cells) with PKH26 then co-culturing with PINK cells for 4 hours. From day 0 to day 21 the expression of NKG2D was increased from 60.9% ± 4.8% to 86% ± 17.4% (p value of 0.024); NKp46 was increased from 10.5% ± 5.4% to 82.8% ± 9.0% (p value of 0.00002); NKp44 was increased from 9.6% ± 6.5% to 51.6% ± 27.5% (p value of 0.022); and 2B4 was decreased from 13.0% ± 7.1% to 0.65% ± 0.5% (p value of 0.009%)(Table 7). Under these culture conditions the inhibitory KIRs including KIR3DL1 (killer cell immunoglobulin-like receptor, three domains, long cytoplasmic tail 1, an inhibitory receptor) and KIR2DL2/L3 (killer cell immunoglobulin-like receptor, two domains, long cytoplasmic tail 2 and long cytoplasmic tail 3; inhibitory receptors) remained not affected during 21-day expansion. The changes in the expression NKRs were further correlated with a marked increase in cytolytic activity at day 21 versus day 14 against K562 cells (63% ± 15% versus 45% ± 4%, p value of 0.0004). These findings have led to identification of the putative markers of NK cells which correlate well with the NK cell cytotoxicity activity.

**Table 7. Phenotypic characterization of PINK cells before and after 21-day cultivation.**

| Standard deviation (Stdev) was calculated for population means for 5 donors. | | | | |
|---|---|---|---|---|
| | Day 0 | | Day 21 | |
| | Mean% | Stdev | Mean% | Stdev |
| CD3-CD56+ | 2.9 | 1.1 | 85.5 | 8.6 |
| CD3-CD56+CD16- | 62.6 | 20.2 | 27.8 | 8.3 |
| CD3-CD56+CD16+ | 37.4 | 20.2 | 72.2 | 8.3 |
| CD3-CD56+KIR3DL1+ | 22.7 | 4.2 | 20.0 | 16.7 |
| CD3-CD56+KIR2DL2/L3+ | 28.4 | 4.2 | 29.6 | 6.4 |
| CD3-CD56+NKG2D+ | 60.9 | 4.8 | 86.0 | 17.4 |
| CD3-CD56+NKp46+ | 10.5 | 5.4 | 82.8 | 8.9 |
| CD3-CD56+CD226+ | 19.5 | 7.4 | 14.1 | 13.3 |
| CD3-CD56+NKp44+ | 9.6 | 6.5 | 51.6 | 27.5 |
| CD3-CD56+NKp30+ | 58.9 | 7.0 | 76.5 | 19.4 |
| CD3-CD56+2B4+ | 13.0 | 7.1 | 0.6 | 0.5 |
| CD3-CD56+CD94+ | 79.7 | 4.9 | 63.9 | 19.4 |

*Membrane Proteomic Profiling of Cultured Placental NK Cells and Cultured Peripheral Blood NK Cells via Lipid-Based Protein Immobilization Technology and Linear Ion Trap LC*/*MS.* Membrane Protein Purification: Placental natural killer cells from combined placental perfusate and cord blood cells, and PB NK cells, cultured for 21 days, were incubated for 15 min with a protease inhibitor cocktail solution (P8340, Sigma Aldrich, St. Louis, MO; contains 4-(2-aminoethyl)benzenesulfonyl fluoride (AEBSF), pepstatin A, E-64, bestatin, leupeptin, and aprotinin, without metal chelators) prior to cell lysis. The cells were then lysed by the addition of a 10 mM HCl solution, without detergents, and centrifuged for 10 min at 400g to pellet and remove the nuclei. The post-nuclear supernatant was transferred to an ultracentriguation tube and centrifuged on a WX80 ultracentrifuge with T-1270 rotor (Thermo Fisher Scientific, Asheville, NC) at 100,000g for 150 minutes generating a membrane protein pellet.

Generation, Immobilization and Digestion of Proteoliposomes: The membrane protein pellet was washed several times using NANOXIS® buffer (10 mM Tris, 300 mM NaCl, pH 8). The membrane protein pellet was suspended in 1.5 mL of NANOXIS® buffer and then tip-sonicated using a VIBRA-CELL™ VC505 ultrasonic processor (Sonics & Materials, Inc., Newtown, CT) for 20 minutes on ice. The size of the proteoliposomes was determined by staining with FM1-43 dye (Invitrogen, Carlsbad, CA) and visualization with fluorescence microscopy. The protein concentration of the proteoliposome suspension was determined by a BCA assay (Thermo Scientific). The proteoliposomes were then injected onto an LPI™Flow Cell (Nanoxis AB, Gothenburg, Sweden) using a standard pipette tip and allowed to immobilize for 1 hour. After immobilization, a series of washing steps were carried out and trypsin at 5 µg/mL (Princeton Separations, Adelphi, NJ) was injected directly onto the LPI™ Flow Cell. The chip was incubated overnight at 37°C. Tryptic peptides were then eluted from the chip and then desalted using a Sep-Pak cartridge (Waters Corporation, Milford, MA).

Strong Cation-Exchange (SCX) Fractionation: Tryptic peptides were reconstituted in a 0.1% formic acid/water solution and loaded onto a strong-cation exchange (SCX) TOP-TIP™ column (PolyLC, Columbia, MD), a pipette tip packed with 30 µm polysufoETHYL aspartamide SCX packing material. Peptides were eluted from the SCX TOP-TIP™ using a step-gradient of ammonium formate buffer, pH 2.8 (10 mM-500 mM). Each SCX fraction was dried using a speed-vac system and reconstituted with 5% acetonitrile, 0.1% Formic Acid in preparation for downstream LC/MS analysis.

LTQ Linear Ion Trap LC/MS/MS Analysis: Each SCX fraction was separated on a 0.2 mm x 150 mm 3µm 200 Å MAGIC C18 column (Michrom Bioresources, Inc., Auburn, CA) that was interfaced directly to an axial desolvation vacuum-assisted nanocapillary electrospray ionization (ADVANCE) source (Michrom Bioresources, Inc.) using a 180 min gradient (Buffer A: Water, 0.1% Formic Acid; Buffer B: Acetonitrile, 0.1% Formic Acid). The ADVANCE source achieves a sensitivity that is comparable to traditional nanoESI while operating at a considerably higher flow rate of 3 µL/min. Eluted peptides were analyzed on an LTQ linear ion trap mass spectrometer (Thermo Fisher Scientific, San Jose, CA) that employed ten data-dependent MS/MS scans following each full scan mass spectrum.

Bioinformatics: Six RAW files corresponding to the 6 salt fractions that were collected for each tumor cell line (AML, CML) were searched as a single search against the IPI Human Database using an implementation of the SEQUEST algorithm on a SORCERER™ SOLO™ workstation (Sage-N Research, San Jose, CA). A peptide mass tolerance of 1.2 amu was specified, oxidation of methionine was specified as a differential modification, and carbamidomethylation was specified as a static modification. A Scaffold software implementation of the Trans-Proteomic Pipeline (TPP) was used to sort and parse the membrane proteomic data. Proteins were considered for analysis if they were identified with a peptide probability of 95%, protein probability of 95% and 1 unique peptide. Comparisons between membrane proteomic datasets were made using custom Perl scripts that were developed in-house.

The analysis revealed the identification of 8 membrane proteins from cultured placental NK cells that were unique with respect to membrane proteins identified from peripheral blood NK cells. *See* Table 8. Further, 8 membrane proteins were identified from peripheral blood NK cells that were unique with respect to cultured placental NK cells. *See* Table 8. Proteins specific for PINK cells and peripheral blood NK cells.

**Table 8.**

| **PROTEINS SPECIFIC FOR PLACENTAL NK CELLS** | **PROTEINS SPECIFIC FOR PB NK CELLS** |
|---|---|
| Aminopeptidase N | Fibroblast growth factor receptor 4 precursor |
| Apolipoprotein E | Immunity-associated nucleotide 4-like 1 protein |
| Atrophin-1 interacting protein 1 | Integrin alpha-L precursor |
| Innexin inx-3 | Integrin beta-2 precursor |
| Integrin alpha-2 precursor | Integrin beta-4 precursor |
| Integrin beta-5 precursor | Membrane-bound lytic murein transglycosylase D precursor |
| Mast cell surface glycoprotein GP49B precursor | Oxysterol binding protein-related protein 8 |
| Ryanodine receptor 1 | Perforin 1 precursor |

### 6.3. Example 3: Natural Killer Cell Cytotoxicity Towards Tumor Cells

This example demonstrates that placental intermediate natural killer cells are cytotoxic towards tumor cells. PINK cells from HPP are cytotoxic to acute myelogenous leukemia cells, as demonstrated in a cytotoxicity assay and by Luminex analysis of NK cell cytokine secretion.

In the cytokine secretion assay, CD56 microbead-enriched NK cells from HPP were mixed with KG-1a acute myelogenous leukemia cells at a 1:1 ratio. After incubation for 24 hours, supernatant was collected and subjected to Luminex analysis of IFN-γ and GM-CSF secretion. Increased levels of IFN-γ and GM-CSF was observed after 24h incubation of CD56-enriched HPP cells with KG-la cells as shown in FIG. 2.

### Cytotoxicity of PINK Cells

In a cytotoxicity assay utilizing PINK cells, target tumor cells were labeled with carboxyfluoroscein succinimidyl ester (CFSE). CFSE is a vital stain that is non-toxic to cells, and is partitioned between daughter cells during cell division. The cells were then placed in 96-well U-bottomed tissue culture plates and incubated with freshly isolated CD56⁺CD16⁻ PINK cells at effector-target (E:T) ratios of 20:1, 10:1, 5:1 and 1:1 in RPMI 1640 supplemented with 10% FBS. After a 4 hour incubation time, cells were harvested and examined by flow cytometry for the presence of CFSE. The number of target cells recovered from culture without NK cells was used as a reference. Cytotoxicity is defined as: (1-CFSEₛₐₘₚₗₑ/CFSE_{control})*100%. Significant tumor cell cytotoxicity was observed at the 20:1 ratio. *See* FIG. 3.

### Tumor Cell Susceptibility to Cultured PINK Cells

*Lactate Dehydrogenase (LDH)-Release Assay.* The LDH-release assay was performed using the CYTOTOX 96® colorimetric cytotoxicity assay kit (Promega, Cat# G1780). In this assay, cultured NK cells, comprising a combination of CD56⁺CD16⁻ cells and CD56⁺CD16⁺ cells derived from matched HPP/UCB, were effector cells, and tumor cells were target cells. Effector cells and target cells were placed in 96-well U-bottom tissue culture plates and incubated at various effector-target (E:T) ratios in 100 µl RPMI 1640 without phenol red (Invitrogen, Cat# 11835-030) supplemented with 2% human AB serum (Gemini, Cat# 100-512). Cultures were incubated for 4h at 37°C in 5% CO₂. After incubation, 50 µl supernatant was transferred to enzymatic assay plate, LDH activity was detected as provided by the manufacturer, and absorption was measured at 490 nm in an ELISA reader (Synergy HT, Biotek). The degree of cytotoxicity was calculated according to the following equation: %Cytotoxicity = (Sample - Effector Spontaneous - Target Spontaneous)/(Target maximum - Target Spontaneous)*100.

Certain tumor types may be more responsive to NK cells than others. To analyze susceptibility of tumor cells to cultured PINK cells, twelve different tumor cell lines, cocultured with PINK cells, were analyzed in an LDH release assay. The 12 tumor cell lines included human chronic myelogenous leukemia (CML), lymphoma, retinoblastoma (RB), and multiple myeloma (MM) (Table 9). The NK cell cytotoxicity was measured by the LDH release assay after 4-hour co-culture.

**Table 9. ATCC Tumor cell lines**

| **Name** | **Description** |
|---|---|
| CCRF-CEM | Human leukemia |
| KG-1 | Human acute myeloid leukemia |
| KG-1A | Human acute myeloid leukemia |
| K562 | Human chronic myeloid leukemia |
| KU812 | Human chronic myeloid leukemia |
| U-937 | Human histiocytic lymphoma |
| WERI-RB-1 | Human retinoblastoma |
| HCC2218 | Human breast cancer |
| RPMI 8226 | Human multiple myeloma |
| HCT116 | Human colorectal carcinoma |
| HT29 | Human colorectal adenocarcinama |
| U266 | Human multiple myeloma |

At effector to target (E:T) ratio of 10:1 significant cytotoxicity of cultured PINK cells was seen towards K562 cells (CML) at 88.6% ± 5.6%, U937 cells (lymphoma) at 89.2% ± 9.8%, WERI-RB-1 cells (RB) at 73.3% ± 11.8%, RPMI8226 cells (MM) at 61.3% ± 1.3%, and U266 cells (MM) at 57.4% ± 4.7% (Table 10).

**Table 10. Differential susceptibility of tumor cells to cultured PINK cells. Standard error of the mean (S. E. M.) was calculated for average cytotoxicity from 3 donors.**

| **Cell Line** | **%Cytotoxicity** | **S.E.M** |
|---|---|---|
| CCRF-CEM | 7.6 | 1.2 |
| KG-1 | 20.5 | 1.5 |
| KG-1a | 6.0 | 3.2 |
| K562 | 88.6 | 5.6 |
| KU812 | 40.3 | 8.2 |
| U937 | 89.2 | 9.8 |
| WERI-RB-1 | 73.3 | 11.8 |
| RPMI8226 | 61.3 | 1.3 |
| U266 | 57.4 | 4.7 |
| HCT-116 | 61.0 | 5.1 |
| HCC2218 | 14.8 | 3.7 |
| HT-29 | 45.6 | 6.0 |

### Enhancement of PINK Cell Cytotoxicity By Treatment with Lenalidomide and Pomalidomide

*RNA isolation and purification.* Isolated or expanded NK cells were subjected to RNA preparation using RNAQUEOUS®-4PCR Kit (Ambion, Cat #AM1914). In brief, NK cells (0.5 to 1.5 x 10⁶ cells) were lysed in the guanidinium lysis solution. The sample lysate was then mixed with an ethanol solution, and applied to a silica-based filter which selectively and quantitatively binds mRNA and the larger ribosomal RNAs; very small RNAs such as tRNA and 5S ribosomal RNA were not quantitatively bound. The filter was then washed to remove residual DNA, protein, and other contaminants, and the RNA was eluted in nuclease-free water containing a trace amount of EDTA to chelate heavy metals. The silica filter was housed in a small cartridge which fits into the RNase-free microfuge tubes supplied with the kit. The sample lysate, wash solutions, and elution solution were moved through the filter by centrifugation or vacuum pressure. After elution from the filter the RNA was treated with the ultra-pure DNase 1 provided with the kit to remove trace amounts of DNA. Finally, the DNase and divalent cations were removed by a reagent also provided with the kit. The concentration and purity of the recovered RNA was determined by measuring its absorbance at 260 and 280 nm.

*Quantitative real-time (qRT-PCR) analysis.* Isolated RNA can then be used for cDNA synthesis using TAQMAN® Reverse Transcription Reagents (Applied Biosystems, Cat #N8080234) followed by real-time PCR analysis by the 7900HT Fast Real-Time PCR System using Human Immune Array (Applied Biosystems, Cat# 4370573) and Human MicroRNA Array (Applied Biosystems, Cat# 4384792).

Lenalidomide and pomalidomide are chemical analogs of thalidomide with enhanced anti-cancer and anti-inflammatory activities. To study if lenalidomide and pomalidomide could enhance PINK cell cytotoxicity, *ex vivo* cultured (day 19) PINK cells were pre-treated with lenalidomide or pomalidomide for 24 hours followed by co-culturing with target colorectal carcinoma cell line HCT-116. Lenalidomide-treated NK cells demonstrated 42.1% cytotoxicity and pomalidomide-treated NK cells showed 47.4% cytotoxicity, while control untreated PINK cells showed only 24.3% cytotoxicity.

Quantitative real-time PCR (qRT-PCR) and flow cytometry analyses showed that the pomalidomide-elicited enhancement of NK cell cytotoxicity was correlated with increased granzyme B (GZMB) gene expression (60% ± 1.7% increase) (Table 11) and increased percentage of GZMB-positive NK cells (25% increase). In addition, expression of GM-CSF was increased in lenalidomide (232% ± 1.6% increase) and pomalidomide (396% ± 0.3% increase)-treated PINK cells (Table 11A, 11B).

Table 11A, 11B. qRT-PCT analysis of lenalidomide- and pomalidomide-treated cultured PINK cells compared to untreated cells. 11A: Fold change of gene expression between lenalidomide-treated and lenalidomide-untreated samples for genes listed. The paired t-test is used to determine if fold changes are equal in lenalidomide-treated and -untreated samples. 11B: Fold change of gene expression between pomalidomide-treated and pomalidomide-untreated samples for 25 genes listed. The paired t-test is used to determine if fold changes are equal in treated and untreated samples.

**Table 11A**

| | **Veh** | **Len.** | **Veh-stdev** | **Len.-stdev** | **P Value** |
|---|---|---|---|---|---|
| BAX | 1 | 1.39 | 0.06 | 0.02 | 0.05 |
| CCL5 | 1 | 1.24 | 0.11 | 0.07 | 0.04 |
| CCR5 | 1 | 0.9 | 0.07 | 0.08 | 0.02 |
| CD68 | 1 | 4.04 | 0.05 | 0.13 | 0.01 |
| CD8A | 1 | 1.3 | 0.01 | 0.02 | 0.02 |
| CSF2 | 1 | 2.32 | 0.14 | 0.02 | 0.02 |
| FAS | 1 | 1.11 | 0.02 | 0.04 | 0.04 |
| GUSB | 1 | 1.13 | 0.04 | 0.07 | 0.05 |
| IL2RA | 1 | 1.26 | 0.03 | 0.01 | 0.03 |
| TNFRSF18 | 1 | 0.7 | 0.1 | 0.16 | 0.04 |

| | | | | | |
|---|---|---|---|---|---|
| BAX - BCL2-associated X protein CCL5 - chemokine (C-C motif) ligand 5 CCR5 - chemokine (C-C motif) receptor 5 CSF2 - colony stimulating factor 2 (granulocyte-macrophage) FAS - TNF receptor superfamily, member 6 GUSB - β glucuronidaseβ IL2RA - α interleukin 2 receptor TNFRSF18 - tumor necrosis factor receptor superfamily, member 18 | | | | | |

**Table 11B**

| | **Veh** | **Pom.** | **Veh-stdev** | **Pom.-stdev** | **P Value** |
|---|---|---|---|---|---|
| ACTB | 1 | 0.77 | 0.01 | 0 | 0.01 |
| BAX | 1 | 2.23 | 0.06 | 0 | 0.01 |
| CCL2 | 1 | 5.46 | 0.01 | 0.37 | 0.02 |
| CCL3 | 1 | 2.2 | 0.04 | 0.16 | 0.02 |
| CCL5 | 1 | 1.78 | 0.11 | 0.04 | 0.02 |
| CCR5 | 1 | 0.68 | 0.07 | 0 | 0.05 |
| CD68 | 1 | 8.74 | 0.05 | 0.19 | 0 |
| CD80 | 1 | 1.59 | 0.13 | 0.19 | 0.02 |
| CD8A | 1 | 2.39 | 0.01 | 0.08 | 0.01 |
| CSF1 | 1 | 1.41 | 0.07 | 0.05 | 0.01 |
| CSF2 | 1 | 3.96 | 0.14 | 0 | 0.01 |
| ECE1 | 1 | 1.56 | 0.06 | 0.12 | 0.02 |
| FAS | 1 | 1.34 | 0.02 | 0.03 | 0.01 |
| GNLY | 1.01 | 1.96 | 0.18 | 0.02 | 0.05 |
| GUSB | 1 | 1.76 | 0.04 | 0.01 | 0.01 |
| GZMB | 1 | 1.59 | 0.06 | 0.02 | 0.03 |
| IL10 | 1.02 | 1.52 | 0.31 | 0.22 | 0.04 |
| IL1A | 1.01 | 2.61 | 0.19 | 0.12 | 0.01 |
| IL2RA | 1 | 1.58 | 0.03 | 0.06 | 0.01 |
| IL8 | 1 | 1.62 | 0.04 | 0.06 | 0.04 |
| LTA | 1 | 2.88 | 0.02 | 0.21 | 0.02 |
| PRF1 | 1 | 1.17 | 0.07 | 0.1 | 0.05 |
| PTGS2 | 1 | 1.68 | 0.01 | 0.05 | 0.02 |
| SKI | 1 | 1.96 | 0.04 | 0.02 | 0.01 |
| TBX21 | 1.01 | 2.05 | 0.14 | 0.2 | 0.01 |

| | | | | | |
|---|---|---|---|---|---|
| ACTB - β-actin BAX - BCL2-associated X protein CCL2 - chemokine (C-C motif) ligand 2 CCL3 - chemokine (C-C motif) ligand 3 CCL5 - chemokine (C-C motif) ligand 5 CCR5 - chemokine (C-C motif) receptor 5 CSF1 - colony stimulating factor 1 (macrophage) CSF2 - colony stimulating factor 2 (granulocyte-macrophage) ECE1 - endothelin converting enzyme 1 FAS - TNF receptor superfamily, member 6 GNLY - granulysin GUSB - glucuronidase-β GZMB - granzyme B (granzyme 2, cytotoxic T-lymphocyte-associated serine esterase 1) ILIA - α interleukin 1 IL2RA - interleukin 2 receptor-α IL8 - interleukin 8 IL10 - interleukin 10 LTA - lymphotoxin α (TNF superfamily, member 1) PRF1 - perforin 1 (pore forming protein) PTGS2 - prostaglandin-endoperoxide synthase 2 (prostaglandin G/H synthase and cyclooxygenase) SKI - v-ski sarcoma viral oncogene homolog (avian) TBX21 - T-box 21 | | | | | |

In a separate experiment, secretion of perforin and granzyme B from pomalidomide- or lenalidomide-treated day 21 cultivated placental NK cells was evaluated by ELISA. Secretion of both enzymes from IMiD-treated day 21 cultivated NK cells was significantly enhanced as compared to vehicle-treated cells, suggesting that IMiDs significantly augment the cytolytic activity of cultured NK cell by increasing the production of perforin and granzyme B. Specifically, pomalidomide-treated NK cells showed a 121% increase in perforin secretion and a 69% increase in granzyme B secretion, and lenalidomide-treated NK cells showed an 86% increase in perforin secretion and a 113% increase in granzyme B secretion as compared to controls.

In another separate experiment, the effect of pomalidomide and lenalidomide on cytokine expression by placental NK cells was evaluated. Secretion of GM-CSF and TNF-α was evaluated by Luminex utilizing a Five-Plex Antibody Kit. Secretion of GM-CSF and TNF-α from treated cultured NK cells was significantly enhanced as compared to vehicle-treated cells, indicating that pomalidomide and lenalidomide significantly augment the cytolytic activity of cultured NK cells by increasing GM-CSF and TNF-α production. Specifically, pomalidomide-treated NK cells showed a 357% increase in GM-CSF secretion and a 147% increase in TNF-α secretion; and lenalidomide-treated NK cells showed a 166% increase in GM-CSF secretion and a 76% increase in TNF-α secretion as compared to controls.

### Cytotoxicity of Combined Natural Killer Cells

In a separate cytotoxicity assay, cultured NK cells derived from donor matched umbilical cord blood and placental perfusate were effector cells, while tumor cells were target cells. Tumor cells were labeled with PKH26 (Sigma-Aldrich Catalog # PKH26-GL) (see, *e.g.,* Lee-MacAry et al., J. Immunol. Meth. 252(1-2):83-92 (2001)), which inserts into cell plasma membrane due to its lipophilic aliphatic residue, then placed in 96-well U-bottom tissue culture plates and incubated with cultured NK cells at various effector-target (E:T) ratios in 200 µl RPMI 1640 supplemented with 10% FBS. Cultures were incubated for 4h at 37°C in 5% CO₂. After incubation, cells were harvested and TO-PRO-3 (Invitrogen Catalog # T3605), a membrane-impermeable DNA stain, was added to cultures to 1 µM final concentration followed by FACS analysis using BD FACSCanto. Cytotoxicity was expressed as percentage of dead cell (PKH26⁺TO-PRO-3⁺) within the total PKH26⁺ target tumor cells.

In this cytotoxicity assay, human chronic myeloid lymphoma (CML) K562 cells were labeled with PKH26, which inserts into cell plasma membrane, and placed in 96-well U-bottomed tissue culture plates. Placental (combo) or peripheral blood NK cells cultured for 21 days were mixed with K562 cells at effector to target (E:T) ratios of 10:1, 5:1, 2.5:1 and 1.25:1 in RPMI 1640 supplemented with 10% v/v FBS. After a 4 hour incubation time, cells were harvested and TO-PRO-3 was added to cell cultures followed by flow cytometry for the presence of PKH26 and TO-PRO-3. Cytotoxicity was expressed as percentage of PKH26⁺TO-PRO-3⁺ dead cells within the total PKH26⁺ target tumor cells. Both placental NK cells and peripheral blood NK cells showed substantial toxicity towards K562 cells at all E:T ratios tested (FIG. 4). Significantly higher toxicity of placental NK cells than peripheral blood NK cells towards K562 cells was observed at two E:T ratios, 10:1 and 5:1 (FIG. 4).

### 6.4. Example 4: Cytotoxicity of Human Placental Perfusate Towards Tumor Cells

This Example demonstrates that human placental perfusate cells are cytotoxic to tumor cells, and that the cytotoxicity of total nucleated cells from HPP (TNC-HPP) on KG-la was higher than that of TNC from matched UCB. Total nucleated cells from HPP or umbilical cord blood (UCB) were mixed with KG-la cells at ratios of 1:1, 5:1, 10:1, 20:1 or 100:1. After 24h or 48h incubation, cells were harvested and examined for the presence of CFSE by FACS analysis (BD FACSCanto, BD Bioscience). Tumor cells cultured alone were used as controls. Cytotoxicity was defined as: (1-CFSEₛₐₘₚₗₑ/CFSE_{Control})*100%. Significant cytotoxicity was shown at the 100:1 ratio. *See* FIG. 5.

In a separate experiment, the cytotoxicity of total nucleated cells from HPP was compared to that of total nucleated cells from umbilical cord blood. Matched TNC-HPP or UCB was mixed with KG-la cells at ratios of 0.78:1, 1.56:1, 3.12:1, 6.25:1, 12.5:1, 25:1, 50:1 or 100:1. TNC-HPP showed consistently higher cytotoxicity at all ratios compared to that of UCB. *See* FIG. 6.

In another experiment, 24 hours prior to incubation with KG-la cells, TNC-HPP was stimulated with 100U/mL, or 1000U/mL of IL-2, while HPP cultured with RPMI medium was used as control. At a ratio of 6.25 NK cells per KG-1a cells and above, IL-2 appears to increase the cytotoxicity of the TNC-HPP. *See* FIG. 7.

The experiments were repeated using a broader array of tumor cell types, as shown in Table 12, using 5 x 10⁵ HPP cells and 1 x 10⁴ tumor cells.

**Table 12: Tumor Cell Types Tested for Cytotoxic Effect of Placental Perfusate**

| | |
|---|---|
| HCC2218 | Human primary ductal carcinoma |
| CCRF-CEM | Human leukemia |
| J.RT3-T3.5 | Human acute T cell leukemia |
| K562 | Human chronic myeloid lymphoma (CML) |
| KG-1 | Human acute myelogenous leukemia |
| KG-1a | Human acute myelogenous leukemia (AML) |
| KU812 | Human leukemia (CML) |
| NCI-H1417 | Human lung carcinoma |
| SNU-CI | Human colon adenocarcinoma |
| U-937 | Human histiocytic lymphoma |
| WERI-RB-1 | Human retinoblastoma |
| HCT-116 | Human colorectal carcinoma |
| HT-29 | Human colorectal adenocarcinoma |
| U266 | Human myeloma |

When HPP cells and tumor cells were co-cultured for 24 hours or 48 hours at a 50:1 ratio, the HPP cells showed substantial toxicity towards the tumor cells. For both times, co-culture resulted in the death of over 50% of the tumor cells. *See* FIGS. 8A and 8B.

### 6.5. Example 5: Cytokine Production by Human Placental Perfusate Cells During Exposure to Tumor Cells

To determine the primary mechanism of action responsible for mediating the potent anti-leukemic effects of HPP cells, the cytokine release profile of HPP cells cocultured with tumor cell lines was analyzed, and compared to that of UCB cells, at different time points by multiplexed Luminex assay.

Supernatants collected post-incubation were subjected to Luminex assay to determine the concentrations of IFN-γ, TNF-α, and GM-CSF (Cat# HCYTO-60K-03, Millipore). These three cytokines are related with NK cytotoxicity. (*See, e.g.,* Imai et al., Blood 2005. 106(1):376-83.). Quantitative RT-PCR was also performed to examine the expression of IFN-γ, TNF-α, and GM-CSF using Applied Biosystems FAST 7900HT instrument and primers. Culture conditions were the same as for the co-culture cytotoxicity assays described above. Concentrations of cytokines were determined using a Luminex assay.

Secretion of IFN-γ, TNF-α, and GM-CSF from HPP cells cocultured with tumor cells was determined to be significantly higher than that of UCB cells. In one experiment, HPP cells were mixed with KG-la cells at ratios of 0.78:1, 1.56:1, 3.12:1, 6.25:1, 12.5:1, 25:1, 50:1 or 100:1, in the presence or absence of 100 U IL-2. TNC-HPP showed consistently increased production of IFN-γ in the presence of IL-2 compared to the absence of IL-2. IFN-γ levels at 24h were determined to increase about 5-26 fold (median: 16 fold); at 48h around 3∼65 fold (median: 27 fold), which was consistent with the results from cytotoxicity study. *See* FIG. 9.

In another experiment, 24 hours prior to incubation with KG-la cells, TNC-HPP was stimulated with 100U/mL, or 1000U/mL of IL-2, while HPP cultured with RPMI media was used as control. HPP or matched UCB cells were incubated 24h with or without IL-2, before cocultured with KG-la cells. The secretion of IFN-γ was most increased in HPP cells cocultured with K562 and KG-la at 48h. When HPP cells were treated with 100 U/mL IL-2, the cytotoxicity of HPP cells on KG-la at 24h and 48h was increased. The secretion level of IFN-γ in HPP cells was higher than that of the matched UCB cells upon IL-2 treatment. Higher expression of IFN-γ was confirmed by RT-PCR analysis of cells from matched HPP and UCB. These results show that HPP cells exhibit higher anti-leukemic activity as compared to UCB cells and this higher activity is associated with a significant increase in IFN-γ production.

IFN-γ production in HPP cells, and in umbilical cord blood cells, during co-culture with a panel of tumor cell lines was analyzed using the tumor cells lines listed in Table 1, above. HPP cells and tumor cells were co-cultured for 24 hours or 48 hours at a ratio of 50:1, using 10⁴ tumor cells and 5 x 10⁵ HPP cells. For CCRF-CEM, J.RT3-T3.5, K562, KG1, KG-1a, KU812, NC1-H1417, U-937 and WER1-RB-1 cell lines, the increase in IFN-γ production in HPP cells at 24 hours co-culture exceeded that of umbilical cord blood cells co-cultured with these cell lines for the same time. *See* FIG. 10A. At 48 hours co-culture, the increase in IFN-γ production in HPP cells exceeded that of umbilical cord blood for all tumor cell lines. *See* FIG. 10B. Of the tumor cell lines, K562 cells induced the greatest increase in IFN-γ production in HPP cells at both 24 hours and 48 hours. Similar results were observed for TNF-α and GM-CSF.

Cell cycle analysis demonstrated that the percentage of KG-la in S phase decreased 30% when cocultured with HPP as compared to KG-la cells cultured alone. Further coculture experiments performed using different enriched fractions of HPP demonstrated that the anti-leukemic activity of HPP was largely attributed to the high concentration of unique immature natural killer cells characterized by high expression of CD56⁺, lack of expression of CD16.

### 6.6. Example 6: Suppression of Tumor Cell

### Proliferation In Vivo by Human Placental Perfusate Cells

### 6.6.1. Materials & Methods

This Example demonstrates the effectiveness of human placental perfusate *in vivo* against tumor cells using a NOD/SCID mouse xenograft tumor model.

*Culturing of KG-1 Cells.* KG-1 cells were maintained in Iscove's modified Dulbecco's medium supplemented with 20% of fetal bovine serum (growth medium) at 37°C in 95% air/5% CO₂ and 100% humidity. Medium in the culture was changed every other day and cells were passaged weekly. KG-1 cells grow as suspensions. Therefore, for changing medium or passaging cells, the cell suspensions were collected in centrifuge tubes and centrifuged at 2,000 rpm for 10 min in a SORVALL® HERAEUS® rotor (part no. 75006434). The supernatant was discarded and an appropriate amount of the cell pellet was resuspended in the growth medium for continuation of culturing.

*KG-1 Cell Preparation for Implantation.* For cell implantation to mice, cells were harvested by centrifugation as described above. Cell pellets were collected and re-suspended in phosphate buffered saline. To determine the number of cells to be implanted to the mice, an aliquot of the cell suspension was counted using a hemacytometer. Trypan Blue dye was used to exclude the non-viable cells in the suspension.

*HPP Cell Preparation for Implantation.* For HPP storage and thawing, samples were received frozen in a dry shipper container in good condition. The units were stored in the dry shipping container until thawing on February 7, 2007. On the day of thawing, HPP units were removed from the cryofreezer (one at a time) and placed into ziptop plastic bags. The bags were then placed into a 37°C water bath with gentle agitation until mostly thawed (a small frozen piece remaining in the bag). The bags were then removed from the water bath, the units were removed from the zip-top bags, and the units were gently inverted until completely thawed. The units were then placed into a laminar flow hood and the outer surface of the blood bag was sterilized by spraying with 70% ethanol. Blood bags were cut open using sterile scissors, and cells were transferred to sterile 50 ml conical tubes (1 tube for each HPP unit; 2 tubes for each UCB unit) by sterile pipette. Next, 10 mL thawing buffer (2.5% human albumin, 5% dextran 40) was slowly added to each tube with gentle mixing (over a period of 2.2 - 2.9 minutes). Each blood bag was then rinsed with 10 mL thawing buffer, which was then slowly added to the 50 ml conical tube (over a period of 0.7 - 1.3 min).

After thawing, each unit was stored on wet ice prior to centrifugation. All tubes were centrifuged for 10 min (440 x g at 10°C), supernatants were aspirated using sterile pipettes, and pellets were gently disrupted by shaking the tube. A 1-ml aliquot of vehicle (PBS + 1% fetal calf serum) was added to one of the tubes, and the tube was mixed by gentle swirling. Using a 2-ml pipette, the contents were transferred to a second tube and then to a third tube and then a fourth tube. The emptied tubes were washed with 0.2 ml dilution buffer.

For cell counting, a 25 µl aliquot of was transferred to a 15 ml conical tube containing 975 µl vehicle on ice. Erythrocytes were then lysed by adding 4 ml cold ammonium chloride lysis reagent and incubating on ice for 10 min. After incubation, 5 ml cold PBS was added to each tube and the tubes were centrifuged (10 min, 400 x g, 10°C). After RBC lysis, cells were counted by hemacytometer, using trypan blue to assess viability. Counting results were corrected for dilution and then divided by a lysis factor (0.46) to estimate the number of cells present before RBC lysis.

For HPP dose preparation, after counting, HPP cells were diluted to 1 x 10⁸ cells/ml by adding vehicle. HPP cells were then stored on ice until syringes were loaded. The elapsed time between thawing the first unit and completion of dose preparation was less than 3 hours.

Before filling syringes, a 50 µl aliquot of the dosing material was set aside for post-dose verification by counting as described above. After dosing, remaining dose material was assessed for dose verification.

*Study Design.* On Day 1, twenty-four NOD/SCID male mice (Jackson Laboratories) were implanted with 5 million viable KG-1 cells S/C at the flank region. The mice were separated such that four to five mice were housed in a micro-isolation cage system with wood chip bedding. Sterilized rodent feed and water was provided *ad libitum.* The mice were closely monitored twice a week for tumor growth. The first measurable tumor was observed on Day 25. Body weights were then recorded once a week and tumor measurements recorded twice a week with a caliper. On Day 52 post-implantation, the animals were randomized into three separate groups, with tumor volumes averaging about 300-350 mm³. *See* Table 13, below. The first group consisted of four control mice with an average tumor volume of 312 mm³. Two of these mice were implanted intravenously (IV), and two intra-tumorally (IT), with 200µl and 50µl of a vehicle solution, respectively. The second group with an average tumor volume of 345 mm³ consisted of four mice implanted intravenously with 200µl of HPP cells per mouse (2 x 10⁷ cells). The last group, implanted IT with 50µl of HPP cells per mouse also consisted of four mice with an average tumor volume of 332 mm³.

**Table 13: Experimental groups for in vivo tumor suppression experiment.**

| **Animal** # | **HPP Treatment Group** | **Tumor Volume On Day of HPP Implantation** |
|---|---|---|
| **Group 1 (Control)** | | |
| 1 | IV 1 | 457 |
| 2 | IT 2 | 429 |
| 3 | IT 3 | 214 |
| 4 | IV 4 | 147 |
| | **Mean:** | **312** |

| **Group 2 (IV Cell Implantation)** | | |
|---|---|---|
| 5 | 1 | 466 |
| 6 | 2 | 209 |
| 7 | 3 | 217 |
| 8 | 4 | 487 |
| | **Mean:** | **345** |

| **Group 3 (IT Cell Implantation)** | | |
|---|---|---|
| 9 | 1 | 491 |
| 10 | 2 | 256 |
| 11 | 3 | 296 |
| 12 | 4 | 285 |
| | **Mean:** | **332** |
| IV - 200 µL implantation; IT - 50 µL implantation. | | |

On Day 66, 14 days after the implantation of HPP cells, the study was terminated due to high tumor volumes.

### 6.6.2. Results

The tumor volumes (TV) were measured until Day 66 (day 14 post HPP-cell implantation) when the TV of the control group reached an average of 2921 mm³. The IV treatment group at the end of study had an average TV of 2076 mm³, and the IT group had a TV of 2705 mm³. With respect to % increase in the TV post-treatment, the IT group showed a modest 20% inhibition whereas the IV group showed more than 35% inhibition of tumor growth compared to the control group. Inhibition in the IT group was demonstrable. *See* FIG. 11.

### 6.7. Example 7: Suppression of Tumor Cell

### Proliferation In Vivo by Cultured Placental NK Cells

### 6.7.1. Materials & Methods

This Example demonstrates the effectiveness of day 21 cultured placental NK (PINK) cells *in vivo* against tumor cells using a NOD/SCID mouse xenograft tumor model.

*Culturing of KG-1 Cells.* KG-1 cells (a human myeloid cell line) were maintained in Iscove's modified Dulbecco's medium supplemented with 20% of fetal bovine serum (growth medium) at 37°C in 95% air/5% CO₂ and 100% humidity. Medium in the culture was changed every other day and cells were passaged weekly. KG-1 cells grow as suspensions. Therefore, for changing medium or passaging cells, the cell suspensions were collected in centrifuge tubes and centrifuged at 2,000 rpm for 10 min in a SORVALL® HERAEUS® rotor (part no. 75006434). The supernatant was discarded and an appropriate amount of the cell pellet was resuspended in the growth medium for continuation of culturing.

*KG-1 Cell Preparation for Implantation.* For cell implantation to mice, cells were harvested by centrifugation as described above. Cell pellets were collected and re-suspended in phosphate buffered saline. To determine the number of cells to be implanted to the mice, an aliquot of the cell suspension was counted using a hemocytometer. Trypan Blue dye was used to determine viability.

*NK Cell Preparation for Implantation.* On the day of thawing, cryopreserved NK cells in cryo-vials were removed from the cryofreezer and immediately placed into a ziploc bag. The ziploc bag containing cryo-vials was then immersed into preset 37°C water bath with agitation until frozen cells mostly thawed. The ziploc bag was then removed from the water bath, cleaned with 70% ethanol, and transferred to a laminar flow hood. NK cells were transferred from cryo-vials to a 50 mL conical tube with pipette. Next, 10 volume of Dilution Buffer (Plasmalyte A + 2.5% HSA) was added to NK cells. Tubes were centrifuged at 1500 rpm (475g) for 8 minutes and supernatant was removed. Cell pellets were then re-suspended in Dilution Buffer (Plasmalyte A + 2.5% HSA) (In general, the volume of Dilution Buffer = 1 mL × the number of vials thawed; For example, if cells are pooled from 4 vials, then add 4 mL dilution buffer). Cell counting was performed using a hemocytometer and trypan blue was used to assess cell viability.

After counting, NK cells were diluted to the desired concentrations (Table 14) by Dilution Buffer. NK cells were then stored on ice until syringes were loaded. The elapsed time between thawing the first unit and completion of dose preparation was less than 3 hours. Before filling syringes, a 50 µl aliquot of the dosing material was set aside for post-dose verification by counting as described above. After dosing, remaining dose material was assessed for dose verification.
*Study Design.* On Day 1, 70 NOD/SCID male mice (Jackson Laboratories) were implanted with 5 million viable KG-1 cells subcutaneously at the flank region. The mice were separated such that four to five mice were housed in a micro-isolation cage system with wood chip bedding. Sterilized rodent feed and water was provided *ad libitum.* The mice were closely monitored twice a week for tumor growth. After first measurable tumor was observed, body weights were then recorded once a week and tumor measurements recorded twice a week with a caliper. When tumor volumes reached 80-100 mm³, mice were randomized into 8 groups using only mice having tumor volumes closest to the mean value. Mice with tumor volumes too big or too small were excluded from the study. Treatment with NK cells was initiated on the day after randomization. The dosing routes were intraveneous (i.v.) or intra-tumoral (IT) injections as described in Table 1. 21 days after NK cell administration, the study was terminated.

**Table 14: Study Design**

| Group | # of Mice | Treatment | Dose and Dosing Route | Dosing Volume |
|---|---|---|---|---|
| 1 | 8 | Control | Vehicle | |
| 2 | 6 | Un-expanded NK cell | 2.0x 10⁶ | 0.3mL |
| 3 | 8 | NK cell (Donor 1) | 2.0x 10⁶ (i.v.) | 0.3mL |
| 4 | 8 | NK Cell (Donor 1) | 6.0x 10^{b} (i.v.) | 0.3mL |
| 5 | 8 | NK Cell (Donor 1) | 2.0x 10⁶ (IT) | 0.05mL |
| 6 | 8 | NK Cell (Donor 2) | 2.0x 10⁶ (i.v.) | 0.3mL |
| 7 | 8 | NK Cell (Donor 2) | 6.0x 10^{b} (i.v.) | 0.3mL |
| 8 | 8 | NK Cell (Donor 2) | 2.0x 10⁶ (IT) | 0.05mL |

*Results:* Tumor volumes (TV) were measured twice weekly until day 21 post NK cell administration (Table 15). Using NK cells derived from donor 1, the high dose IV group showed significant tumor suppression at day 6 post NK cell administration. The high dose IV group and the IT group showed significant tumor suppression at day 13 and day 21 post NK cell administration. Using NK cells derived from donor 2, the high dose IV group and the IT group showed significant tumor suppression starting at day 6 and day 13 post NK cell administration. All three treatment groups showed significant tumor suppression at day 21 post NK cell administration.

**Table 15: Summary of in vivo tumor suppression by day 21 cultured placental NK cells. Mean: mean of 8 animals; SD: standard deviation for population means of 8 animals; SE: standard error for population means of 8 animals; p value: the two-sample t-test is used to determine if population means are equal in Vehicle-treated and NK-treated groups at various time points.**

| | TV (mm3) | Day1 | Day4 | Day6 | Day9 | Day13 | Day15 | Day18 | Day21 |
|---|---|---|---|---|---|---|---|---|---|
| Vehicle | Mean | 93.28 | 233.94 | 279.24 | 357.13 | 575.57 | 687.20 | 862.02 | 1345.66 |
| | SD | 20.11 | 52.37 | 38.98 | 71.61 | 88.42 | 91.07 | 142.31 | 279.82 |
| | SE | 7.60 | 19.79 | 14.73 | 27.07 | 33.42 | 34.42 | 53.79 | 105.76 |
| NK1-IV-2MM | Mean | 93.14 | 237.90 | 264.53 | 336.65 | 541.97 | 642.34 | 950.84 | 1170.16 |
| | SD | 20.02 | 57.17 | 57.55 | 84.97 | 147.18 | 193.69 | 327.02 | 243.09 |
| | SE | 7.57 | 21.61 | 21.75 | 32.11 | 55.63 | 73.21 | 123.60 | 91.88 |
| | p Value | 0.49 | 0.44 | 0.28 | 0.31 | 0.29 | 0.28 | 0.25 | 0.10 |
| NK1-IV-6MM | Mean | 93.84 | 198.14 | 240.82 | 267.50 | 449.88 | 553.22 | 769.39 | 1030.13 |
| | SD | 19.25 | 50.53 | 45.90 | 55.34 | 128.50 | 144.91 | 185.08 | 263.54 |
| | SE | 7.27 | 19.10 | 17.35 | 20.92 | 48.57 | 54.77 | 69.96 | 99.61 |
| | p Value | 0.48 | 0.09 | 0.05 | 0.01 | 0.02 | 0.02 | 0.14 | 0.02 |
| NK1-IT-2MM | Mean | 93.87 | 215.93 | 266.55 | 328.94 | 450.18 | 574.89 | 730.97 | 832.68 |
| | SD | 19.20 | 74.10 | 58.06 | 72.69 | 127.35 | 155.68 | 202.13 | 326.93 |
| | SE | 7.26 | 28.01 | 21.94 | 27.48 | 48.13 | 58.84 | 76.40 | 123.57 |
| | p Value | 0.48 | 0.29 | 0.31 | 0.22 | 0.02 | 0.05 | 0.08 | 0.00 |
| NK2-IV-2MM | Mean | 94.77 | 192.91 | 268.05 | 335.38 | 529.99 | 634.11 | 846.61 | 1113.85 |
| | SD | 18.57 | 34.56 | 55.02 | 37.38 | 116.48 | 147.00 | 165.72 | 204.24 |
| | SE | 7.58 | 14.11 | 22.46 | 15.26 | 47.55 | 60.01 | 67.65 | 83.38 |
| | p Value | 0.44 | 0.05 | 0.33 | 0.24 | 0.20 | 0.20 | 0.42 | 0.05 |
| NK2-IV-6MM | Mean | 93.06 | 193.64 | 241.05 | 304.40 | 472.32 | 596.11 | 834.62 | 1091.41 |
| | SD | 15.97 | 34.04 | 40.09 | 49.47 | 96.65 | 137.62 | 201.18 | 313.09 |
| | SE | 6.04 | 12.87 | 15.15 | 18.70 | 36.53 | 52.02 | 76.04 | 118.34 |
| | p Value | 0.49 | 0.04 | 0.04 | 0.05 | 0.02 | 0.07 | 0.38 | 0.05 |
| NK2-IT-2MM | Mean | 92.65 | 164.47 | 207.40 | 256.88 | 347.15 | 505.84 | 764.87 | 1029.05 |
| | SD | 14.64 | 54.75 | 55.67 | 69.20 | 100.70 | 144.71 | 259.06 | 387.86 |
| | SE | 5.53 | 20.69 | 21.04 | 26.15 | 38.06 | 54.70 | 97.92 | 146.60 |
| | p Value | 0.47 | 0.01 | 0.00 | 0.01 | 0.00 | 0.00 | 0.18 | 0.04 |

| | | | | | | | | | |
|---|---|---|---|---|---|---|---|---|---|
| TV = tumor volume | | | | | | | | | |

### 6.8. Example 8: Suppression of CML Cell Proliferation In Vivo by Cultured Placental NK Cells

The example demonstrates the effectiveness of day 21 cultured placental NK cells on the growth of K562 human leukemia xenografts in NOD/SCID mice.

*Cell Culture.* K562 cells, a chronic myelogenous leukemia cell line, were maintained in Iscove's modified Dulbecco's medium (IMDM) supplemented with 20% of fetal bovine serum. The cells were cultured at 37°C in 95% air/5% CO₂ and 100% humidity. Medium in the culture was changed every other day and cells were passaged weekly. This cell line grew as suspensions. Therefore, for changing medium or passaging cells, the cell suspensions were collected in centrifuge tubes and centrifuged at 2,000 rpm for 5 min. The supernatant was discarded and appropriate amount of the cell pellets was resuspended in the appropriate medium for continuation of culturing.

*K562 cell preparation for implantation.* For cell implantation to mice, cells were harvested by centrifugation as described above. The cell pellets were collected and resuspended in phosphate buffered saline (PBS). To determine the number of cells to be implanted to the mice, an aliquot of the cell suspension was counted using a hemocytometer, and Trypan Blue dye was used to measure the number of viable cells in the suspension. The harvested cells were washed once with PBS and resuspended in serum-free medium at a density of 5 ×10⁶ cells/100 µl.

*Preparation of the NK Cells for dosage administration.* Two NK cell lines were used, the characteristics of which are described in Table 16.

**Table 16: Description of NK cells**

| NK Cell ID No. | Viability | %CD56⁺CD3⁻ | %CD56⁺CD3⁺ | *In Vitro* Cytotoxicity Against K562 |
|---|---|---|---|---|
| 1 | 82% | 89.3 | 4.7 | 70% |
| 2 | 85% | 38.6 | 39.6 | 46% |

On the day of thawing, cryopreserved NK cells were thawed and counted. After counting, the NK cells were diluted to the desired concentrations (Table 17):

**Table 17: Study design of the K562 xenograft study; cells administered intravenously (i.v.) or intratumorally (IT)**

| Group | # of Mice | Treatment | Dose Level and Dosing Route | Dosing Volume |
|---|---|---|---|---|
| 1 | 16 | Plasmalyte A | i.v. | 0.3 ml |
| 2 | 8 | NK Cell ID No. 1 | 6.0 × 10⁶ (i.v.) | 0.3 ml |
| 3 | 8 | NK Cell ID No. 1 | 2.0 × 10⁶ (IT) | 0.05 ml |
| 4 | 16 | NK Cell ID No. 2 | 2.0 × 10⁶ (i.v.) | 0.3 ml |
| 5 | 8 | NK Cell ID No. 2 | 6.0 × 10⁶ (i.v.) | 0.3 ml |
| 6 | 8 | NK Cell ID No. | 2.0 × 10⁶ (IT) | 0.05 ml |

| | | | | |
|---|---|---|---|---|
| NK cells were then stored on ice until syringes were loaded. | | | | |

*Assay procedure.* To initiate the study, 5 × 10⁶ K562 viable cells, suspended in 100 µl of serum-free medium, were subcutaneously implanted to the nude mice in the right flank region. One hundred mice were implanted with K562 cells. Animals were monitored for tumor growth daily after cell implantation. When tumor volumes reached 80-100 mm³, the mice were randomized into 8 groups using only mice having tumor volumes closest to the mean value. Mice with tumor volumes too big or too small were excluded from the study. Treatment with the NK cells was initiated on the day after randomization, and only one single dose of NK cells was administered to the mice. Tumor volumes were measured using the formula V = L × W × H × π/6, where L and W represented the longer and shorter diameters of the tumor and H represented the height of the tumor. Throughout the entire study, tumor volumes were measured twice weekly and body weights once weekly. Animals were observed for possible toxic effect from the treatment of NK cells. Unscheduled sacrifices were performed when tumor volumes exceeded 1,500 mm³, or loss of the original body weights exceeded 20%, or tumor ulceration occurred, or mice became moribund. At the end of the experiment, statistical analysis was performed on tumor growth rate between the control and each treatment group. Major organ tissues were harvested at the termination of the study.

*Results.* The growth rates of K562 leukemia tumors were distinctly reduced by NK Cell ID No. 1 at an intravenous dose level of 6 million, or at an intratumoral dose level of 2 million inhibited the growth of K562 tumors. Growth inhibitory effect was statistically significant in the intratumorally (P < 0.05 by Students' t test). Tumors in all 3 groups treated with 091008 NK cells were not significantly reduced.

During the course of the study, 3 out of the 10 tumors completely regressed to zero after 13 days of treatment. In addition, throughout the entire study, all the animals stayed healthy and active, and none of the mice in each group lost any body weight, indicating that the NK cell administration, itself, did not result in increased morbidity and was generally safe.

## Claims

1. Isolated natural killer cells comprising isolated CD56⁺, CD16⁻, CD3⁻ placental intermediate natural killer cells for use in a method for treatment of an individual having cancer, wherein the method comprises administering to said individual said isolated natural killer cells comprising isolated CD56⁺, CD16⁻, CD3⁻ placental intermediate natural killer cells,
wherein said placental intermediate natural killer cells are isolated from placental perfusate and express one or more of the microRNAs hsa-miR-100, hsa-miR-127, hsa-miR-211, hsa-miR-302c, hsa-miR-326, hsa-miR-337, hsa-miR-497, hsa-miR-512-3p, hsa-miR-515-5p, hsa-miR-517b, hsa-miR-517c, hsa-miR-518a, hsa-miR-518e, hsa-miR-519d, hsa-miR-520g, hsa-miR-520h, hsa-miR-564, hsa-miR-566, hsa-miR-618, or hsa-miR-99a at a detectably higher level than peripheral blood natural killer cells; and
wherein said cancer is acute promyelocytic leukemia, acute myeloblastic leukemia, acute megakaryoblastic leukemia, precursor B acute lymphoblastic leukemia, precursor T acute lymphoblastic leukemia, Burkitt's leukemia (Burkitt's lymphoma), acute biphenotypic leukemia, chronic monocytic leukemia, chronic lymphocytic leukemia (CLL)/Small lymphocytic lymphoma, B-cell prolymphocytic leukemia; hairy cell lymphoma; T-cell prolymphocytic leukemia, lymphoplasmacytic lymphoma, Waldenström macroglobulinemia, splenic marginal zone lymphoma, plasmacytoma, a monoclonal immunoglobulin deposition disease, or a heavy chain disease, extranodal marginal zone B cell lymphoma (MALT lymphoma), nodal marginal zone B cell lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, T cell large granular lymphocytic leukemia, aggressive NK cell leukemia, extranodal NK/T cell lymphoma, nasal type, enteropathy-type T cell lymphoma, hepatosplenic T cell lymphoma, blastic NK cell lymphoma, mycosis fungoides (Sezary syndrome), a primary cutaneous CD30-positive T cell lymphoproliferative disorder, primary cutaneous anaplastic large cell lymphoma, lymphomatoid papulosis, angioimmunoblastic T cell lymphoma, peripheral T cell lymphoma, unspecified, anaplastic large cell lymphoma, a Hodgkin lymphoma, or a nodular lymphocyte-predominant Hodgkin lymphoma.

2. The isolated natural killer cells for use according to claim 1, wherein the method additionally comprises administering to said individual an effective amount of lenalidomide, pomalidomide, or thalidomide.

3. The isolated natural killer cells for use according to claim 1, wherein said isolated natural killer cells have been contacted with pomalidomide, lenalidomide, or thalidomide prior to said administering.

4. The isolated natural killer cells for use according to claim 1, wherein said natural killer cells comprise natural killer cells not obtained from placental perfusate.

5. Isolated natural killer cells comprising isolated CD56⁺, CD16⁻, CD3⁻ placental intermediate natural killer cells for use in a method for treatment of an individual having a viral infection, wherein the method comprises administering to said individual said isolated natural killer cells comprising isolated CD56⁺, CD16⁻, CD3⁻ placental intermediate natural killer cells,
wherein said placental intermediate natural killer cells are isolated from placental perfusate and express one or more of the microRNAs hsa-miR-100, hsa-miR-127, hsa-miR-211, hsa-miR-302c, hsa-miR-326, hsa-miR-337, hsa-miR-497, hsa-miR-512-3p, hsa-miR-515-5p, hsa-miR-517b, hsa-miR-517c, hsa-miR-518a, hsa-miR-518e, hsa-miR-519d, hsa-miR-520g, hsa-miR-520h, hsa-miR-564, hsa-miR-566, hsa-miR-618, or hsa-miR-99a at a detectably higher level than peripheral blood natural killer cells.

6. The isolated natural killer cells for use according to claim 5, wherein the method additionally comprises administering to said individual an effective amount of lenalidomide, pomalidomide, or thalidomide.

7. The isolated natural killer cells for use according to claim 5, wherein said isolated natural killer cells have been contacted with pomalidomide, lenalidomide, or thalidomide prior to said administering.

8. The isolated natural killer cells for use according to claim 5, wherein said natural killer cells comprise natural killer cells not obtained from placental perfusate.

9. The isolated natural killer cells for use according to claim 1 or 5, wherein said natural killer cells are combined natural killer cells that comprise natural killer cells isolated from placental perfusate and natural killer cells isolated from umbilical cord blood.

10. The isolated natural killer cells for use according to claim 9, wherein said umbilical cord blood is isolated from the placenta from which said placental perfusate is obtained.

11. The isolated natural killer cells for use according to claim 9, wherein said combined natural killer cells comprise:
a detectably higher number of CD3⁻CD56⁺CD16⁻ natural killer cells than an equivalent number of natural killer cells from peripheral blood;
a detectably lower number of CD3⁻CD56⁺CD16⁺ natural killer cells than an equivalent number of natural killer cells from peripheral blood;
a detectably higher number of CD3⁻CD56⁺KIR2DL2/L3⁺ natural killer cells than an equivalent number of natural killer cells from peripheral blood;
a detectably lower number of CD3⁻CD56⁺NKp46⁺ natural killer cells than an equivalent number of natural killer cells from peripheral blood;
a detectably higher number of CD3⁻CD56⁺NKp30⁺ natural killer cells than an equivalent number of natural killer cells from peripheral blood;
a detectably higher number of CD3⁻CD56⁺2B4⁺ natural killer cells than an equivalent number of natural killer cells from peripheral blood; or
a detectably higher number of CD3⁻CD56⁺CD94⁺ natural killer cells than an equivalent number of natural killer cells from peripheral blood.

12. The isolated natural killer cells for use according to claim 3 or 7, wherein said natural killer cells are contacted with said pomalidomide, lenalidomide, or thalidomide in an amount and for a time sufficient for said natural killer cells to express detectably more granzyme B, or mRNA encoding granzyme B, than an equivalent number of natural killer cells not contacted with said pomalidomide, lenalidomide, or thalidomide.

13. The isolated natural killer cells for use according to claim 1 or 5, wherein said natural killer cells have not been cultured prior to said administering.

14. The isolated natural killer cells for use according to claim 1 or 5, wherein said placental intermediate natural killer cells express one or more of aminopeptidase N protein, apolipoprotein E protein, atrophin-1 interacting protein 1, innexin inx-3 protein, integrin alpha-2 precursor protein, integrin beta-5 precursor, mast cell surface glycoprotein GP49B precursor protein, or ryanodine receptor 1 protein; and wherein said placental intermediate natural killer cells do not express one or more of fibroblast growth factor receptor 4 precursor protein, immunity-associated nucleotide 4-like protein, integrin alpha-L precursor protein, integrin beta 2 precursor protein, integrin beta 4 precursor protein, membrane-bound lytic murein transglycosylase D precursor protein, oxysterol binding protein-related protein 8, or perforin 1 precursor 1 protein.

## Patentansprüche

1. Isolierte natürliche Killerzellen, umfassend isolierte CD56⁺, CD16⁻, CD3⁻ plazentare intermediäre natürliche Killerzellen zur Verwendung in einem Verfahren zur Behandlung eines Individuums, das Krebs aufweist, wobei das Verfahren die Verabreichung der isolierten natürlichen Killerzellen, umfassend isolierte CD56⁺, CD16⁻, CD3⁻ plazentare intermediäre natürliche Killerzellen, an dieses Individuum umfasst,
wobei die plazentaren intermediären natürlichen Killerzellen aus plazentarem Perfusat isoliert sind und eine oder mehrere der microRNAs hsa-miR-100, hsa-miR-127, hsa-miR-211, hsa-miR-302c, hsa-miR-326, hsa-miR-337, hsa-miR-497, hsa-miR-512-3p, hsa-miR-515-5p, hsa-miR-517b, hsa-miR-517c, hsa-miR-518a, hsa-miR-518e, hsa-miR-519d, hsa-miR-520g, hsa-miR-520h, hsa-miR-564, hsa-miR-566, hsa-miR-618 oder hsa-miR-99a auf einem nachweisbar höheren Niveau als natürliche Killerzellen des peripheren Blutes exprimieren; und
wobei der Krebs akute promyelozytische Leukämie, akute myeloblastische Leukämie, akute megakaryoblastische Leukämie, Vorläufer von B akuter lymphoblastischer Leukämie, Vorläufer von T akuter lymphoblastischer Leukämie, Burkitt-Leukämie (Burkitt-Lymphom), akute biphänotypische Leukämie, chronische monozytische Leukämie, chronische lymphatische Leukämie (CLL)/kleines lymphatisches Lymphom, B-Zell-Prolymphozyten-Leukämie; Haarzell-Lymphom; T-Zell-Prolymphozyten-Leukämie, lymphoplasmatisches Lymphom, Waldenström-Makroglobulinämie, Milz-Randzonen-Lymphom, Plasmacytom, eine Krankheit aufgrund der Ablagerung von monoklonalem Immunglobulin oder eine schwere Ketten-Krankheit, extranodales Randzone B-Zell-Lymphom (MALT-Lymphom), nodales Randzone B-Zell-Lymphom (NMZL), follikuläres Lymphom, Mantelzell-Lymphom, diffuses großes B-Zell-Lymphom, mediastinales (thymisches) großes B-Zell-Lymphom, intravaskuläres großes B-Zell-Lymphom, primäres Erguss-Lymphom, T-Zellen-LGL-Leukämie, aggressive NK-Zell-Leukämie, extranodales NK/T-Zell-Lymphom, nasaler Typ, T-Zell-Lymphom vom enteropathischen Typ, hepatosplenisches T-Zell-Lymphom, blastisches NK-Zell-Lymphom, Mycosis fungoides (Sezary-Syndrom), eine primäre kutane CD30-positive T-Zell-Lymphoproliferationsstörung, primäres kutanes anaplastisches Großzell-Lymphom, lymphomatoide Papulose, angioimmunoblastisches T-Zell-Lymphom, peripheres T-Zell-Lymphom, unspezifisches, anaplastisches Großzell-Lymphom, ein Hodgkin-Lymphom oder ein noduläres Lymphozyten-prädominantes Hodgkin-Lymphom ist.

2. Isolierte natürliche Killerzellen zur Verwendung nach Anspruch 1, wobei das Verfahren zusätzlich die Verabreichung einer wirksamen Menge von Lenalidomid, Pomalidomid oder Thalidomid an das Individuum umfasst.

3. Isolierte natürliche Killerzellen zur Verwendung nach Anspruch 1, wobei die isolierten natürlichen Killerzellen vor der Verabreichung mit Pomalidomid, Lenalidomid oder Thalidomid in Kontakt gebracht wurden.

4. Isolierte natürliche Killerzellen zur Verwendung nach Anspruch 1, wobei die natürlichen Killerzellen natürliche Killerzellen umfassen, die nicht aus plazentarem Perfusat gewonnen wurden.

5. Isolierte natürliche Killerzellen, umfassend isolierte CD56⁺, CD16⁻, CD3⁻ plazentare intermediäre natürliche Killerzellen zur Verwendung in einem Verfahren zur Behandlung eines Individuums, das eine Virusinfektion hat, wobei das Verfahren die Verabreichung der isolierten natürlichen Killerzellen, umfassend isolierte CD56⁺, CD16⁻, CD3⁻ plazentare intermediäre natürliche Killerzellen, an das Individuum umfasst,
wobei die plazentaren intermediären natürlichen Killerzellen aus plazentarem Perfusat isoliert sind und eine oder mehrere der microRNAs hsa-miR-100, hsa-miR-127, hsa-miR-211, hsa-miR-302c, hsa-miR-326, hsa-miR-337, hsa-miR-497, hsa-miR-512-3p, hsa-miR-515-5p, hsa-miR-517b, hsa-miR-517c, hsa-miR-518a, hsa-miR-518e, hsa-miR-519d, hsa-miR-520g, hsa-miR-520h, hsa-miR-564, hsa-miR-566, hsa-miR-618 oder hsa-miR-99a auf einem nachweisbar höheren Niveau als natürliche Killerzellen im peripheren Blut exprimieren.

6. Isolierte natürliche Killerzellen zur Verwendung nach Anspruch 5, wobei das Verfahren zusätzlich die Verabreichung einer wirksamen Menge von Lenalidomid, Pomalidomid oder Thalidomid an das Individuum umfasst.

7. Isolierte natürliche Killerzellen zur Verwendung nach Anspruch 5, wobei die isolierten natürlichen Killerzellen vor der Verabreichung mit Pomalidomid, Lenalidomid oder Thalidomid in Kontakt gebracht wurden.

8. Isolierte natürliche Killerzellen zur Verwendung nach Anspruch 5, wobei die natürlichen Killerzellen natürliche Killerzellen umfassen, die nicht aus plazentarem Perfusat gewonnen wurden.

9. Isolierte natürliche Killerzellen zur Verwendung nach Anspruch 1 oder 5, wobei die natürlichen Killerzellen kombinierte natürliche Killerzellen sind, die natürliche Killerzellen, die aus plazentarem Perfusat isoliert sind, und natürliche Killerzellen, die aus Nabelschnurblut isoliert sind, umfassen.

10. Isolierte natürliche Killerzellen zur Verwendung nach Anspruch 9, wobei das Nabelschnurblut aus der Plazenta isoliert wird, aus der das plazentare Perfusat gewonnen wird.

11. Isolierte natürliche Killerzellen zur Verwendung nach Anspruch 9, wobei die kombinierten natürlichen Killerzellen umfassen:
eine nachweisbar höhere Anzahl von CD3⁻CD56⁺CD16⁻ natürlichen Killerzellen als eine äquivalente Anzahl natürlicher Killerzellen aus peripherem Blut;
eine nachweisbar geringere Anzahl von CD3⁻CD56⁺CD16⁻ natürlichen Killerzellen als eine äquivalente Anzahl natürlicher Killerzellen aus peripherem Blut;
eine nachweisbar höhere Anzahl von CD3⁻CD56⁺KIR2DL2/L3⁺ natürlichen Killerzellen als eine äquivalente Anzahl natürlicher Killerzellen aus peripherem Blut;
eine nachweisbar geringere Anzahl von CD3⁻CD56⁺NKp46⁺ natürlichen Killerzellen als eine äquivalente Anzahl natürlicher Killerzellen aus peripherem Blut;
eine nachweisbar höhere Anzahl von CD3⁻CD56⁺NKp30⁺ natürlichen Killerzellen als eine äquivalente Anzahl natürlicher Killerzellen aus peripherem Blut;
eine nachweisbar höhere Anzahl von CD3⁻CD56⁺2B4⁺ natürlichen Killerzellen als eine äquivalente Anzahl der natürlichen Killerzellen aus peripherem Blut; oder
eine nachweisbar höhere Anzahl von CD3⁻CD56⁺CD94⁺ natürlichen Killerzellen als eine äquivalente Anzahl natürlicher Killerzellen aus peripherem Blut.

12. Isolierte natürliche Killerzellen zur Verwendung nach Anspruch 3 oder 7, wobei die natürlichen Killerzellen mit dem Pomalidomid, Lenalidomid oder Thalidomid in einer Menge und für eine Zeit in Kontakt gebracht werden, die ausreicht, so dass die natürlichen Killerzellen nachweisbar mehr Granzym B oder mRNA, die für Granzym B kodiert, exprimieren als eine äquivalente Anzahl natürlicher Killerzellen, die nicht mit dem Pomalidomid, Lenalidomid oder Thalidomid in Kontakt gebracht worden ist.

13. Isolierte natürliche Killerzellen zur Verwendung nach Anspruch 1 oder 5, wobei die natürlichen Killerzellen vor der Verabreichung nicht kultiviert worden sind.

14. Isolierte natürliche Killerzellen zur Verwendung nach Anspruch 1 oder 5, wobei die plazentaren intermediären natürlichen Killerzellen ein oder mehrere von Aminopeptidase N-Protein, Apolipoprotein E-Protein, Atrophin-1 interagierendes Protein 1, Innexin inx-3-Protein, Integrin alpha-2-Vorläuferprotein, Integrin beta-5-Vorläufer, Mastzelloberfläche Glycoprotein GP49B-Vorläuferprotein oder Ryanodinrezeptor 1-Protein exprimieren; und wobei die plazentaren intermediären natürlichen Killerzellen ein oder mehrere von Fibroblasten-Wachstumsfaktor-Rezeptor-4-Vorläuferprotein, immunitätsassoziiertem Nukleotid-4-ähnlichem Protein, Integrin-alpha-L-Vorläuferprotein, Integrin-beta-2-Vorläuferprotein, Integrin-beta-4-Vorläuferprotein, membrangebundenem lytischem Murein-Transglycosylase-D-Vorläuferprotein, Oxysterol-bindendem Protein-verwandtem Protein-8 oder Perforin-1-Vorläufer-1-Protein nicht exprimieren.

## Revendications

1. Cellules tueuses naturelles isolées comprenant des cellules tueuses naturelles intermédiaires placentaires CD56⁺, CD16⁻, CD3⁻ isolées pour l'utilisation dans un procédé de traitement d'un individu souffrant d'un cancer, dans lesquelles le procédé comprend l'administration audit individu desdites cellules tueuses naturelles isolées comprenant des cellules tueuses naturelles intermédiaires placentaires CD56⁺, CD16⁻, CD3⁻ isolées,
dans lesquelles lesdites cellules tueuses naturelles intermédiaires placentaires sont isolées à partir d'un perfusat placentaire et expriment un ou plusieurs des micro-ARN hsa-miR-100, hsa-miR-127, hsa-miR-211, hsa-miR-302c, hsa-miR-326, hsa-miR-337, hsa-miR-497, hsa-miR-512-3p, hsa-miR-515-5p, hsa-miR-517b, hsa-miR-517c, hsa-miR-518a, hsa-miR-518e, hsa-miR-519d, hsa-miR-520g, hsa-miR-520h, hsa-miR-564, hsa-miR-566, hsa-miR-618, ou hsa-miR-99a à un niveau détectable plus élevé que les cellules tueuses naturelles du sang périphérique; et
dans lesquelles ledit cancer est une leucémie aiguë promyélocytaire, une leucémie aiguë myéloblastique, une leucémie aiguë mégacaryoblastique, une leucémie aiguë lymphoblastique à précurseur B, une leucémie aiguë lymphoblastique à précurseur T, la leucémie de Burkitt (lymphome de Burkitt), une leucémie aiguë biphénotypique, une leucémie monocytique chronique, une leucémie lymphoïde chronique (LLC)/petite leucémie lymphoïde, une leucémie prolymphocytaire à cellules B; un lymphome à tricholeucocytes; une leucémie prolymphocytaire à cellules T, un lymphome lymphoplasmocytaire, la macroglobulinémie de Waldenström, un lymphome de la zone marginale splénique, un plasmocytome, une maladie associée au dépôt d'immunoglobulines monoclonales, ou une maladie de la chaîne lourde, un lymphome extraganglionnaire à cellules B de la zone marginale (lymphome MALT), un lymphome ganglionnaire à cellules B de la zone marginale (NMZL), un lymphome folliculaire, un lymphome à cellules du manteau, un lymphome diffus à grandes cellules B, un lymphome médiastinal (thymique) à grandes cellules B, un lymphome intravasculaire à grandes cellules B, un lymphome primaire d'épanchement, une leucémie lymphoïde granulaire à grandes cellules T, une leucémie à cellules NK agressives, une leucémie extraganglionnaire à cellules NK/T, un lymphome T de type entéropathie, de type nasal, un lymphome T hépatosplénique, un lymphome blastique à cellules NK, un mycosis fongoïde (syndrome de Sérazy), un trouble lymphoprolifératif cutané primaire à cellules T CD30-positives, un lymphome anaplasique cutané primaire à grandes cellules, une papulose lymphomatoïde, un lymphome angioimmunoblastique à cellules T, un lymphome périphérique à cellules T, non spécifié, un lymphome anaplasique à grandes cellules, un lymphome hodgkinien, ou un lymphome hodgkinien ganglionnaire à lymphocytes prédominants.

2. Cellules tueuses naturelles isolées pour l'utilisation selon la revendication 1, dans lesquelles le procédé comprend en outre l'administration audit individu d'une quantité efficace de lénalidomide, de pomalidomide, ou de thalidomide.

3. Cellules tueuses naturelles isolées pour l'utilisation selon la revendication 1, dans lesquelles lesdites cellules tueuses naturelles isolées ont été mises en contact avec du pomalidomide, du lénalidomide, ou du thalidomide avant ladite administration.

4. Cellules tueuses naturelles isolées pour l'utilisation selon la revendication 1, dans lesquelles lesdites cellules tueuses naturelles comprennent des cellules tueuses naturelles non obtenues à partir d'un perfusat placentaire.

5. Cellules tueuses naturelles isolées comprenant des cellules tueuses naturelles intermédiaires placentaires CD56⁺, CD16⁻, CD3⁻ isolées pour l'utilisation dans un procédé de traitement d'un individu souffrant d'une infection virale, dans lesquelles le procédé comprend l'administration audit individu desdites cellules tueuses naturelles isolées comprenant des cellules tueuses naturelles intermédiaires placentaires CD56⁺, CD16⁻, CD3⁻ isolées,
dans lesquelles lesdites cellules tueuses naturelles intermédiaires placentaires sont isolées à partir d'un perfusat placentaire et expriment un ou plusieurs des micro-ARN hsa-miR-100, hsa-miR-127, hsa-miR-211, hsa-miR-302c, hsa-miR-326, hsa-miR-337, hsa-miR-497, hsa-miR-512-3p, hsa-miR-515-5p, hsa-miR-517b, hsa-miR-517c, hsa-miR-518a, hsa-miR-518e, hsa-miR-519d, hsa-miR-520g, hsa-miR-520h, hsa-miR-564, hsa-miR-566, hsa-miR-618, ou hsa-miR-99a à un niveau détectable plus élevé que les cellules tueuses naturelles du sang périphérique.

6. Cellules tueuses naturelles isolées pour l'utilisation selon la revendication 5, dans lesquelles le procédé comprend en outre l'administration audit individu d'une quantité efficace de lénalidomide, de pomalidomide, ou de thalidomide.

7. Cellules tueuses naturelles isolées pour l'utilisation selon la revendication 5, dans lesquelles lesdites cellules tueuses naturelles isolées ont été mises en contact avec du pomalidomide, du lénalidomide, ou du thalidomide avant ladite administration.

8. Cellules tueuses naturelles isolées pour l'utilisation selon la revendication 5, dans lesquelles lesdites cellules tueuses naturelles comprennent des cellules tueuses naturelles non obtenues à partir d'un perfusat placentaire.

9. Cellules tueuses naturelles isolées pour l'utilisation selon la revendication 1 ou 5, dans lesquelles lesdites cellules tueuses naturelles sont des cellules tueuses naturelles combinées qui comprennent des cellules tueuses naturelles isolées à partir d'un perfusat placentaire et des cellules tueuses naturelles isolées à partir du sang de cordon ombilical.

10. Cellules tueuses naturelles isolées pour l'utilisation selon la revendication 9, dans lesquelles ledit sang de cordon ombilical est isolé à partir du placenta à partir duquel ledit perfusat placentaire est obtenu.

11. Cellules tueuses naturelles isolées pour l'utilisation selon la revendication 9, dans lesquelles lesdites cellules tueuses naturelles combinées comprennent:
un nombre détectable plus élevé de cellules tueuses naturelles CD3⁻CD56⁺CD16⁻ qu'un nombre équivalent de cellules tueuses naturelles provenant du sang périphérique;
un nombre détectable plus bas de cellules tueuses naturelles CD3⁻CD56⁺CD16⁺ qu'un nombre équivalent de cellules tueuses naturelles provenant du sang périphérique;
un nombre détectable plus élevé de cellules tueuses naturelles CD3⁻ CD56⁺KIR2DL2/L3⁺ qu'un nombre équivalent de cellules tueuses naturelles provenant du sang périphérique;
un nombre détectable plus bas de cellules tueuses naturelles CD3⁻CD56⁺NKp46⁺ qu'un nombre équivalent de cellules tueuses naturelles provenant du sang périphérique;
un nombre détectable plus élevé de cellules tueuses naturelles CD3⁻CD56⁺NKp30⁺ qu'un nombre équivalent de cellules tueuses naturelles provenant du sang périphérique;
un nombre détectable plus élevé de cellules tueuses naturelles CD3⁻CD56⁺2B4⁺ qu'un nombre équivalent de cellules tueuses naturelles provenant du sang périphérique; ou
un nombre détectable plus élevé de cellules tueuses naturelles CD3⁻CD56⁺CD94⁺ qu'un nombre équivalent de cellules tueuses naturelles provenant du sang périphérique.

12. Cellules tueuses naturelles isolées pour l'utilisation selon la revendication 3 ou 7, dans lesquelles lesdites cellules tueuses naturelles sont mises en contact avec ledit pomalidomide, lénalidomide, ou thalidomide en une quantité et pendant une durée suffisantes pour que lesdites cellules tueuses naturelles expriment de manière détectable davantage de granzyme B, ou d'ARNm codant pour le granzyme B, qu'un nombre équivalent de cellules tueuses naturelles non mises en contact avec ledit pomalidomide, lénalidomide, ou thalidomide.

13. Cellules tueuses naturelles isolées pour l'utilisation selon la revendication 1 ou 5, dans lesquelles lesdites cellules tueuses naturelles n'ont pas été mises en culture avant ladite administration.

14. Cellules tueuses naturelles isolées pour l'utilisation selon la revendication 1 ou 5, dans lesquelles lesdites cellules tueuses naturelles intermédiaires placentaires expriment un ou plusieurs parmi une protéine d'aminopeptidase N, une protéine d'apolipoprotéine E, une protéine interagissant avec l'atrophine-1, une protéine d'innexine inx-3, une protéine de précurseur d'intégrine alpha-2, un précurseur d'intégrine bêta-5, une protéine de précurseur de glycoprotéine GP49B de la surface des mastocytes, ou une protéine du récepteur 1 de la ryanodine; et dans lesquelles lesdites cellules tueuses naturelles intermédiaires placentaires n'expriment pas une ou plusieurs parmi une protéine de précurseur du récepteur 4 du facteur de croissance des fibroblastes, une protéine de type nucléotide 4 associé à l'immunité, une protéine de précurseur d'intégrine alpha-L, une protéine de précurseur d'intégrine bêta 2, une protéine de précurseur d'intégrine bêta 4, une protéine de précurseur de muréine transglycosylase D lytique liée à la membrane, une protéine 8 associée à la protéine de liaison à l'oxystérol, ou une protéine de précurseur 1 de la perforine 1.
